# EUROPEAN PATENT SPECIFICATION

(11) **EP 4 573 210 B1**
(45) Date of publication and mention of the grant of the patent: **06.05.2026**
(21) Application number: 23825329.8
(22) Date of filing: 15.11.2023
(51) Int. Cl.: C12Q 1/6813, C12Q 1/6841

(54) **METHOD FOR ANALYSING GENE EXPRESSION IN CELLS IN A PELLET**
VERFAHREN ZUR ANALYSE DER GENEXPRIMIERUNG IN ZELLEN IN EINEM PELLET
PROCÉDÉ POUR ANALYSER L'EXPRESSION DES GÈNES DANS UN PELLET DES CÉLLULES

(30) Priority: 16.11.2022 US 202263425991 P
(43) Date of publication of application: 25.06.2025
(60) Divisional application: 26166758.8
(73) Proprietor: 10X Genomics, Inc., Pleasanton, CA 94588-3260 (US)
(72) Inventor: BELHOCINE, Zahra Kamila, Pleasanton, CA 94588-3260 (US); FREEMAN, Melanie, Pleasanton, CA 94588-3260 (US); GONZALEZ MUÑOZ, Veronica Emelina, Pleasanton, CA 94588-3260 (US); KRISHNAN, Sreenath, Pleasanton, CA 94588-3260 (US); MARKS, Patrick J., Pleasanton, CA 94588-3260 (US); SASAKI, Hiroshi, Pleasanton, CA 94588-3260 (US)
(74) Representative: J A Kemp LLP
(86) International application number: PCT/US2023/079887
(87) International publication number: WO 2024/107887

(56) References cited:
- WO-A1-2018/175779
- WO-A1-2019/068880
- CN-A- 110 373 451
- ASENSIO A FERNÁNDEZ ET AL: "Targeting HER2 protein in individual cells using ICP-MS detection and its potential as prognostic and predictive breast cancer biomarker", TALANTA, ELSEVIER, AMSTERDAM, NL, vol. 235, 8 August 2021 (2021-08-08), XP086773513, ISSN: 0039-9140, [retrieved on 20210808], DOI: 10.1016/J.TALANTA.2021.122773
- X.-B. ZHONG ET AL: "Visualization of oligonucleotide probes and point mutations in interphase nuclei and DNA fibers using rolling circle DNA amplification", PROCEEDINGS OF THE NATIONAL ACADEMY OF SCIENCES, vol. 98, no. 7, 27 March 2001 (2001-03-27), pages 3940 - 3945, XP055562866, ISSN: 0027-8424, DOI: 10.1073/pnas.061026198

## Description

### FIELD

The present disclosure relates in some aspects to assays for transcriptomic profiling *in situ.*

### BACKGROUND

Genomic, transcriptomic, and proteomic profiling of cells and tissue samples using microscopic imaging can resolve multiple analytes of interest at the same time, thereby providing valuable information regarding analyte abundance and localization *in situ.* Thus, *in situ* assays are important tools, for example, for understanding the molecular basis of cell identity and developing treatment for diseases. There is a need for improving *in situ* assays. Provided herein are methods and compositions that address such and other needs.

### SUMMARY

The invention is defined by the appended claims. In some aspects, provided herein is a performance test for assessing the performance of *in situ* analyte detection and/or platforms (e.g., methods, assays, workflows, systems and/or instruments for *in situ* analyte detection). In some aspects, results from different *in situ* analyte detection and/or sequencing experiments can be variable, in part because the results depend on the analyzed biological sample (which can vary between experiments) as well as the specific *in situ* analyte detection and/or platform used to analyze the biological sample. This variability can make it difficult to assess the performance of *in situ* analyte detection and/or platforms based on the results, and/or to compare performance of different *in situ* analyte detection and/or platforms. Thus, there is a need for methods to assess and/or compare performance of *in situ* analyte detection and/or platforms.

In some aspects, the performance test provided herein facilitates assessment and/or comparison of the performance of in situ analysis platforms. In some aspects, the assessment is quantitative and/or qualitative. In some aspects, the performance test depends on 1) a standardized test biological sample with known properties, 2) specific target analytes, and 3) specific methods of analysis, to produce results. In some aspects, the performance test produces expected results if performance is satisfactory. In some aspects, the performance test produces unexpected results if performance is not optimal and/or unsatisfactory.

Provided herein is a method, comprising: (a) providing a test biological sample, wherein the test biological sample is a section of a cell pellet comprising a defined mixture of a first cell population and a second cell population; (b) contacting the test sample with a probe mixture comprising: (i) a first panel of probes or probe sets, wherein each probe or probe set of the first panel comprises a recognition sequence complementary to a target sequence of an RNA transcript or product thereof of a first set of genes expressed in the first cell population, and (ii) a second panel of probes or probe sets, wherein each probe or probe set of the second panel comprises a recognition sequence complementary to a target sequence of an RNA transcript or product thereof of a second set of genes expressed in the second cell population, and allowing the probes or probe sets to hybridize to their target sequences in the test biological sample, wherein the first cell population does not detectably express the second set of genes, and the second cell population does not detectably express the first set of genes; (c) detecting the hybridized probes or probe sets or products of the hybridized probes or probe sets at locations in the biological sample, thereby detecting RNA transcripts of the first and second sets of genes in the test biological sample; (d) identifying discrete cells in the test biological sample and comparing the detected RNA transcripts of the first and second sets of genes in the cells to expected expression levels of the first and second sets of genes in the first and second cell populations.

In any of the embodiments herein, the probe mixture can comprise: (iii) a third panel of probes or probe sets, wherein each probe or probe set of the third panel comprises a recognition sequence complementary to a target sequence of an RNA transcript or product thereof of a third set of genes expressed in both the first cell population and the second cell population, and wherein the method comprises allowing the probes or probe sets of the third panel to hybridize to their target sequences in the test biological sample, detecting the hybridized probes or probe sets or products of the hybridized probes or probe sets of the third panel at locations in the biological sample, thereby detecting RNA transcripts of the third set of genes in the test biological sample; and comparing the detected RNA transcripts of the third set of genes in the cells to expected expression levels of the third set of genes in the first and second cell populations.

In any of the embodiments herein, the first, and/or second sets of genes can comprise genes having at least two different expression levels. In any of the embodiments herein, the third set of genes can comprise genes having at least two different expression levels. In any of the embodiments herein, the at least two different expression levels can be selected from the group consisting of low expression, medium expression, and high expression. In any of the embodiments herein, the first and/or second sets of genes can comprise (i) genes that have low expression, (ii) genes that have medium expression, and (iii) genes that have high expression. In any of the embodiments herein, the third set of genes can comprise (i) genes that have low expression, (ii) genes that have medium expression, and (iii) genes that have high expression.

Disclosed, but outside the scope of the claims, is a method, comprising: (a) providing a test biological sample, wherein the test biological sample is a section of a cell pellet comprising a defined mixture of a first cell population and a second cell population; (b) contacting the test sample with a probe or probe set mixture comprising probes or probe sets complementary to target sequences of RNA transcripts or products thereof of a set of genes including genes having at least two different expression levels selected from the group consisting of low, medium, and high expression in the first cell population and/or second cell population, and allowing the probes or probe sets to hybridize to their target sequences in the sample, (c) detecting the hybridized probes or probe sets or products of the hybridized probes or probe sets at locations in the biological sample, thereby detecting RNA transcripts of the set of genes in the test biological sample; (d) identifying discrete cells in the test biological sample and comparing the detected RNA transcripts of set of genes in the cells to expected expression levels set of genes in the first and second cell populations.

In any of the embodiments herein, the method can comprise analyzing sensitivity of the RNA transcript detection by comparing the detected RNA transcripts to the expected expression levels of the genes. In any of the embodiments herein, the method can comprise analyzing specificity of the RNA transcript detection by comparing the detected RNA transcripts to the expected expression levels of the genes.

In any of the embodiments herein, genes that have low expression can be genes detected at a mean count of 1-5 transcripts per cell by single cell RNA sequencing. In any of the embodiments herein, genes that have medium expression can be genes detected at a mean count of 5-20 transcripts per cell by single cell RNA sequencing. In any of the embodiments herein, genes that have high expression can be detected at a mean count of more than 20 transcripts per cell by single cell RNA sequencing.

In any of the embodiments herein, genes that have low expression can be detected at a mean count of 1-5 transcripts per cell at the locations in the biological sample. In any of the embodiments herein, genes that have medium expression can be detected at a mean count of 5-20 transcripts per cell at the locations in the biological sample. In any of the embodiments herein, genes that have low expression can be detected at a mean count of more than 20 transcripts per cell at the locations in the biological sample. In any of the embodiments herein, the expected expression levels of genes that have low expression can be a mean count of 1-5 transcripts per cell detected at the locations in the biological sample. In any of the embodiments herein, the expected expression levels of genes that have medium expression can be a mean count of 5-20 transcripts per cell detected at the locations in the biological sample. In any of the embodiments herein, the expected expression levels of genes that have low expression can be a mean count of more than 20 transcripts per cell detected at the locations in the biological sample.

Disclosed, but outside the scope of the claims, is a method, comprising: (a) providing a test biological sample, wherein the test biological sample is a section of a cell pellet comprising a defined mixture of a first cell population and a second cell population; (b) contacting the test sample with a panel of probes or probe sets, wherein the probes or probe sets individually comprise recognition sequences complementary to target sequences of RNA transcripts or product thereof of a panel of genes and allowing the probes or probe sets to hybridize to their target sequences in the test biological sample, wherein the panel of genes comprises genes that are detectably expressed in the first cell population but not the second cell population and genes that are expressed in the second cell population but not the first cell population; (c) detecting the hybridized probes or probe sets or products of the hybridized probes or probe sets at locations in the biological sample, thereby detecting RNA transcripts of the genes expressed in the test biological sample; (d) identifying discrete cells in the test biological sample and comparing the detected RNA transcripts of the genes in the cells to expected expression levels of the genes in the first and second cell populations, thereby analyzing the sensitivity and/or specificity of the RNA transcript detection.

In any of the embodiments herein, the panel of genes can comprise genes that are expressed in both the first and second cell populations. In any of the embodiments herein, the panel of probes or probe sets can be a panel of circularizable probes or probe sets. In any of the embodiments herein, the method can comprise ligating the circularizable probes or probe sets hybridized to their target sequences, thereby generating circularized probes at locations in the test biological sample.

In any of the embodiments herein, the probes or probe sets can comprise barcode sequences corresponding to the RNA transcripts comprising their corresponding target sequences. In any of the embodiments herein, the method can comprise performing rolling circle amplification of the circularized probes to generate rolling circle amplification products at locations in the test biological sample. In any of the embodiments herein, the rolling circle amplification products can comprise complements of the barcode sequences of the circularizable probes or probe sets. In any of the embodiments herein, detecting the hybridized probes or probe sets or products of the hybridized probes or probe sets can comprise detecting the barcode sequences in the hybridized probes or probe sets. In any of the embodiments herein, detecting the hybridized probes or probe sets or products of the hybridized probes or probe sets can comprise detecting the rolling circle amplification products. In any of the embodiments herein, detecting the rolling circle amplification products can comprise detecting the complements of the barcode sequences. In any of the embodiments herein, detecting the barcode sequences or complements thereof can comprise binding intermediate probes directly or indirectly to the barcode sequences or complements thereof, binding a detectably labeled probes directly or indirectly to detection regions of the intermediate probes, and detecting signals associated with the detectably labeled probes. In any of the embodiments herein, the method can comprise performing one or more wash steps to remove unbound and/or nonspecifically bound intermediate probe molecules from the test biological sample. In any of the embodiments herein, detecting the barcode sequences or complements thereof can comprise sequencing all or a portion of the barcode sequences or complements thereof.

In any of the embodiments herein, detecting the barcode sequences or complements thereof can comprises: contacting the test biological sample with a universal pool of detectably labeled probes and a first pool of intermediate probes, wherein the intermediate probes of the first pool of intermediate probes comprise hybridization regions complementary to the barcode sequences or complements thereof and reporter regions complementary to a detectably labeled probe of the universal pool of detectably labeled probes; detecting complexes formed between the barcode sequences or complements thereof, the intermediate probes of the first pool of intermediate probes, and the detectably labeled probes; and removing the intermediate probes of the first pool of intermediate probes and the detectably labeled probes.

In any of the embodiments herein, detecting the barcode sequences or complements thereof can further comprise: contacting the test biological sample with the universal pool of detectably labeled probes and a second pool of intermediate probes, wherein the intermediate probes of the second pool of intermediate probes comprise hybridization regions complementary to the barcode sequences or complements thereof and reporter regions complementary to a detectably labeled probe of the universal pool of detectably labeled probes; and detecting complexes formed between the barcode sequences or complements thereof, the intermediate probes of the second pool of intermediate probes, and the detectably labeled probes.

In any of the embodiments herein, each barcode sequence or complement thereof can be assigned a sequence of signal codes that identifies the barcode sequence or complement thereof, and detecting the barcode sequences or complements thereof can comprise decoding the barcode sequences of complements thereof by detecting the corresponding sequences of signal codes detected from sequential hybridization, detection, and removal of sequential pools of intermediate probes and the universal pool of detectably labeled probes.

In any of the embodiments herein, the sequences of signal codes can be fluorophore sequences assigned to the corresponding barcode sequences or complements thereof. In any of the embodiments herein, the detectably labeled probes can be fluorescently labeled.

In any of the embodiments herein, identifying the discrete cells can comprise performing cell segmentation. In any of the embodiments herein, the cell segmentation can comprise identifying nuclei of the discrete cells. In any of the embodiments herein, the cell segmentation can comprise identifying membranes of the discrete cells. In any of the embodiments herein, identifying the nuclei of the discrete cells can comprise detecting a nuclear stain. In any of the embodiments herein, the cell segmentation can comprise performing nuclear expansion. In any of the embodiments herein, identifying membrane of the discrete cells can comprise detecting a membrane stain. In any of the embodiments herein, the cell segmentation can comprise identifying an area of maximum distance 5 µm, 10 µm, or 15 µm from the nucleus. In any of the embodiments herein, the cell segmentation can comprise identifying the boundaries of the discrete cells.

In any of the embodiments herein, the detected RNA transcripts can be assigned to the identified discrete cells. In any of the embodiments herein, the method can comprise clustering the identified discrete cells using the detected RNA transcripts. In any of the embodiments herein, the method can comprise comparing the detected RNA transcripts assigned to the identified discrete cells. In some embodiments, the identified discrete cells are associated with the first cell population or the second cell population.

In any of the embodiments herein, the method can comprise analyzing one or more success metrics selected from the group consisting of: a quality score for barcode or barcode complement sequencing or decoding, median number of different genes detected per cell, mean number of different genes detected per cell, median number of transcripts detected per cell, mean number of transcripts detected per cell, number of cells detected, number of transcripts decoded per 100 µm², nuclear transcript density per area (e.g., per 100 µm²), total high quality decoded transcripts, RNA transcript density, and percent of detected transcripts within identified cells. In any of the embodiments herein, the method can comprise determining a quality score for the sequenced or decoded barcode sequence or complement thereof. In any of the embodiments herein, the quality score can be at least q20. In any of the embodiments herein, the median number of different genes detected per cell can be at least 5 or 10. In any of the embodiments herein, the median number of transcripts detected per cell can be at least 15, at least 20, at least 30, at least 40, at least 60, at least 80, or at least 100. In any of the embodiments herein, the mean number of transcripts detected per cell can be at least 15, at least 20, at least 30, at least 40, at least 60, at least 80, or at least 100. In any of the embodiments herein, the density of detected RNA transcripts having a quality score of at least 20 can be at least 0.15 per µM². In any of the embodiments herein, the percent of detected transcripts within identified cells can be at least any one of 95%, 96%, 97%, 98%, 99%, and 99.5%. In any of the embodiments herein, the method can comprise analyzing median number of transcripts detected per cell, number of cells detected, and number of transcripts decoded per 100 µm². In any of the embodiments herein, the method can comprise analyzing median number of transcripts detected per cell, number of cells detected, and nuclear transcript density per area (e.g., per 100 µm²),

In any of the embodiments herein, the method can comprise contacting the test biological sample with a negative control probe or probe set comprising a negative control barcode sequence, and detecting the presence or absence of the negative control probe or probe set or a product thereof in the test biological sample. In any of the embodiments herein, the detecting the presence or absence of the negative control probe or probe set can comprise detecting the presence or absence of the negative control barcode sequence or a complement thereof in the negative control probe or probe set or a product thereof. In any of the embodiments herein, the method can comprise analyzing the detection rate of the negative control probe or probe set or product thereof.

In any of the embodiments herein, the density of the identified discrete cells does not need to be more than 0.5 cells per 100 µm².

In any of the embodiments herein, the ratio of the first cell population and the second cell population in the cell pellet can be between about 10:1 and about 1:10. In any of the embodiments herein, the ratio of the first cell population and the second cell population in the cell pellet can be between about 2:1 and about 1:2. In any of the embodiments herein, the first cell population and the second cell population can be of the same species. In any of the embodiments herein, the first cell population and the second cell population can be derived from different tissue types. In any of the embodiments herein, the first cell population and the second cell population can be of hematopoietic origin. In any of the embodiments herein, the first cell population can be a first cultured cell line, and the second cell population can be a second cultured cell line. In any of the embodiments herein, the first cell population can a T cell line, and the second population can be a B cell line. In any of the embodiments herein, the first cell population can be Jurkat cells, and the second cell population can be Raji cells. In any of the embodiments herein, the first cell population and the second cell population can be human cells. In any of the embodiments herein, the first cell population and the second cell population can be selected from the group consisting of 293 cells, A549 cells, BHL-100 cells, Caco-2 cells, Chang cells, Daudi cells, H9 cells, HCT-15 cells, HCT116 cells, HeLA cells, Hep-2 cells, HepG2 cells, HL-60 cells, HT-1080 cells, HT-29 cells, HUVEC cells, IM-9 cells, JEG-2 cells, Jurkat cells, K-562 cells, KB cells, KG-1 cells, MCF7 cells, Raji cells, Ramos B cells, THP-1 monocytes, WI-38 cells, and WISH cells. In any of the embodiments herein, the first cell population and the second cell population can be mouse cells.

In any of the embodiments herein, the cell pellet can be a formalin-fixed, paraffin-embedded (FFPE) cell pellet. In any of the embodiments herein, the cell pellet can be a fresh frozen cell pellet. In any of the embodiments herein, the method can comprise embedding the cell pellet in an embedding medium and sectioning the embedded cell pellet to provide the test biological sample. In any of the embodiments herein, the test biological sample can be on a substrate, optionally wherein the substrate is a glass substrate.

### BRIEF DESCRIPTION OF THE DRAWINGS

The drawings illustrate certain features and advantages of this disclosure.
**FIG. 1** shows schematics illustrating an example performance test based on detection of mutually exclusive gene expression patterns in a mixed population of known cell types.
**FIG. 2** shows schematics illustrating example configurations of slides with sections from cell blocks comprising unmixed or mixed populations of known cell types.
**FIGS. 3A-3B** show example cell-type-specific genes and commonly expressed "housekeeping" (HK) genes for Jurkat and Raji cells based on relative expression data. The Jurkat-specific, housekeeping, and Raji-specific genes labeled in FIG. 3B are listed in FIG. 3A.
**FIG. 4** shows an exemplary imaged section of a cell block containing a mixed population of Jurkat and Raji cells. DAPI staining reveals individual nuclei, which are segmented (outlines).
**FIG. 5** shows an exemplary imaged section of a cell block containing a mixed population of Jurkat and Raji cells during a sequential hybridization/detection cycle. DAPI staining reveals individual nuclei and fluorescence at 647 nm reveals signals from detection oligonucleotides.
**FIG. 6** shows an example imaged nucleus (DAPI) with decoded objects corresponding to specific transcripts.
**FIGS. 7A** **and** **7B** shows example UMAP projections based on clustering of single cell gene expression profiles from a mixed population of Jurkat and Raji cells, with cells assigned to 2 clusters based on k-means clustering analysis. Gene expression data for each cluster is shown in **FIG. 7B****,** with Jurkat-specific, Raji-specific, and housekeeping genes highlighted.
**FIG. 8** shows an example imaged section of a cell block containing a mixed population of Jurkat and Raji cells that are segmented and identified according to gene expression obtained from an *in situ* sequencing assay.
**FIG. 9** is an example workflow of analysis of a biological sample (e.g., a cell or tissue sample) using an opto-fluidic instrument, according to various embodiments.

### DETAILED DESCRIPTION

If a definition set forth herein is contrary to or otherwise inconsistent with a definition set forth in the patents, applications, published applications and other publications that are referenced herein, the definition set forth herein prevails over the definition that is present in the patents, applications, published applications and other publications referenced herein.

### I. OVERVIEW

Transcriptomic profiling of biological samples using microscopic imaging (i.e. *in situ* analyte detection), provides valuable information regarding analyte abundance and localization *in situ.* In situ platforms for analyte detection provides an important tool, for example, for understanding the molecular basis of cell identity and developing treatment for diseases.

There is a need for methods to assess performance of *in situ* analysis platforms (e.g., methods, assays, workflows, and/or instruments for *in situ* analyte detection) in various scenarios. For example, the performance of an *in situ* analyte detection platform might be assessed as a quality control measure to ensure that the platform is working as expected and producing reliable data. In another example, performance of two different *in situ* analyte detection platforms might be compared. However, assessing and/or comparing the performance of *in situ* analyte detection platforms can be challenging, in part because the results are dependent on the biological sample analyzed, and biological samples such as tissue sections can have high sample-to-sample variance.

In some aspects, provided herein is a simple and robust performance test for *in situ* analyte detection and/or analysis platforms. In some aspects, the performance test includes parameters for the biological sample, the gene panel (e.g. transcripts) to be analyzed, and the analysis to be performed. In some embodiments, aspects of the performance test are alterable, including the biological sample, the gene panel, and the analysis, as described in detail herein. In some aspects, the performance test provides a standardized workflow to assess performance within and/or across *in situ* analyte detection and/or platforms. In some aspects, provided herein are readouts and/or criteria for assessing performance of an *in situ* analyte detection assay.

In some aspects, the performance test is highly reproducible. In some aspects, the performance test can be used to validate and verify protocols and reagents for quality control. In some aspects, the workflow of the performance test is simple and robust. In some aspects, the simple and robust workflow is advantageous, for example, in allowing inexperienced users to become familiar with an in situ assay, and/or to determine that the platform (e.g., an instrument or system for performing the assay) is being properly utilized to produce expected results. In some aspects, the performance test is applicable to various different *in situ* assays and/or platforms, which may be advantageous for assessing and/or comparing the different platforms.

The test biological sample is a section of a cell pellet comprising a defined mixture of a first cell population and a second cell population. Disclosed, but outside the scope of the claims, the test biological sample comprises a section of a first cell pellet comprising a first cell population, and a second cell pellet comprising a second cell population. In some embodiments, the first cell pellet and the second cell pellet are embedded in the same medium. The first cell population and the second cell population are contained contacted with each of the reagents simultaneously. The method comprises contacting the test sample with a probe mixture. The probe mixture comprises a first panel of probes or probe sets. Each probe or probe set of the first panel comprises a recognition sequence complementary to a target sequence of an RNA transcript or product thereof of a first set of genes expressed in the first cell population. The probe mixture comprises a second panel of probes or probe sets. Each probe or probe set of the second panel comprises a recognition sequence complementary to a target sequence of an RNA transcript or product thereof of a second set of genes expressed in the second cell population. The method comprises allowing the probes or probe sets to hybridize to their target sequences in the test biological sample. The first cell population does not detectably express the second set of genes. And the second cell population does not detectably express the first set of genes. The method comprises detecting the hybridized probes or probe sets or products of the hybridized probes or probe sets at locations in the biological sample, thereby detecting RNA transcripts of the first and second sets of genes in the test biological sample. In some embodiments, products of the hybridized probes or probe sets are amplification products of the hybridized probes or probe sets. In some embodiments, products of the hybridized probes or probe sets are rolling circle amplification products (RCPs). The method comprises identifying discrete cells in the test biological sample and comparing the detected RNA transcripts of the first and second sets of genes in the cells to expected expression levels of the first and second sets of genes in the first and second cell populations.

In some embodiments, provided herein is a method, comprising providing a test biological sample. In some embodiments, the test biological sample is a section of a cell pellet comprising a defined mixture of a first cell population and a second cell population.

In some embodiments, provided herein is a method, comprising providing a test biological sample comprising a section of a cell pellet comprising a defined mixture of a cell population of Jurkat cells and a cell population of Raji cells. The population of Jurkat cells and the population of Raji cells are contained contacted with each of the reagents. The method comprises contacting the test sample comprising a population of Jurkat cells and a population of Raji cells with a probe mixture. The probe mixture comprises a first panel of probes or probe sets. In some embodiments, each probe or probe set of the first panel comprises a recognition sequence complementary to a target sequence of an RNA transcript or product thereof of a first set of genes expressed in the population of Jurkat cells and the probe mixture comprises a second panel of probes or probe sets. In some embodiments, each probe or probe set of the second panel comprises a recognition sequence complementary to a target sequence of an RNA transcript or product thereof of a second set of genes expressed in the population of Raji cells. In some embodiments, the method comprises allowing the probes or probe sets to hybridize to their target sequences in the test biological sample comprising a population of Jurkat cells and a population of Raji cells. The population of Jurkat cells does not detectably express the second set of genes. The population of Raji cells does not detectably express the first set of genes. The method comprises detecting the hybridized probes or probe sets or products of the hybridized probes or probe sets at locations in the biological sample comprising a population of Jurkat cells and a population of Raji cells, thereby detecting RNA transcripts of the first and second sets of genes in the test biological sample. In some embodiments, products of the hybridized probes or probe sets are amplification products of the hybridized probes or probe sets. In some embodiments, products of the hybridized probes or probe sets are rolling circle amplification products (RCPs). In some embodiments, the method comprises identifying discrete cells in the test biological sample comprising a population of Jurkat cells and a population of Raji cells and comparing the detected RNA transcripts of the first and second sets of genes in the population of Jurkat cells and the population of Raji cells to expected expression levels of the first and second sets of genes in Jurkat cells and in Raji cells.

In some embodiments, the method comprises contacting the test sample with a probe or probe set mixture comprising probes or probe sets complementary to target sequences of RNA transcripts or products thereof of a set of genes including genes having at least two different expression levels. In some embodiments, the at least two different expression levels are selected from the group consisting of low, medium, and high expression in the first cell population and/or second cell population. In some embodiments, the method comprises identifying discrete cells in the test biological sample and comparing the detected RNA transcripts of set of genes in the cells to expected expression levels set of genes in the first and second cell populations.

In some embodiments, provided herein is a method, comprising providing a test biological sample, wherein the test biological sample is a section of a cell pellet comprising a defined mixture of a population of Jurkat cells and a population of Raji cells. In some embodiments, the method comprises contacting the test sample with a probe or probe set mixture comprising probes or probe sets complementary to target sequences of RNA transcripts or products thereof of a set of genes including genes having at least two different expression levels. In some embodiments, the at least two different expression levels are selected from the group consisting of low, medium, and high expression in Jurkat cells and/or Raji cells. In some embodiments, the method comprises allowing the probes or probe sets to hybridize to their target sequences in the sample. In some embodiments, the method comprises detecting the hybridized probes or probe sets or products of the hybridized probes or probe sets at locations in the biological sample, thereby detecting RNA transcripts of the set of genes in the test biological sample. In some embodiments, products of the hybridized probes or probe sets are amplification products of the hybridized probes or probe sets. In some embodiments, products of the hybridized probes or probe sets are rolling circle amplification products (RCPs). In some embodiments, the method comprises identifying discrete cells in the test biological sample and comparing the detected RNA transcripts of set of genes in the cells to expected expression levels set of genes in Jurkat cells and Raji cells.

In some embodiments, the method comprises identifying discrete cells in the test biological sample and comparing the detected RNA transcripts of the genes in the cells to expected expression levels of the genes in the first and second cell populations, thereby analyzing the sensitivity and/or specificity of the RNA transcript detection. In some cases, the assessment of the performance of the *in situ* analysis assay can indicate if the assay encountered an unexpected failure mode due to any steps in processing of the biological sample. For example, if the test biological sample detaches from the substrate (e.g., due to an incorrectly performed sample preparation procedure), this may result in lower cell density detected in the performance of the *in situ* analysis assay. In some cases, if the test biological sample in the cyclic assay of providing and removing probes for hybridization encounters suboptimal stripping, this may result in residual signals from previous cycles. In some aspects, the assessment of the performance of the assay can indicate if one or more steps in processing and assaying of the test biological sample resulted in unexpected results (e.g., unsatisfactory performance). In some aspects, the assessment of the performance of the assay provides pass/fail criteria (e.g., based on metrics described herein) for evaluating the processing and assaying of the test biological sample.

In some cases, the assessment of the performance of the *in situ* analysis assay can indicate if the assay encountered an unexpected failure mode due to any steps in processing of the biological sample. For example, if the test biological sample detaches from the substrate (e.g., due to an incorrectly performed sample preparation procedure), this may result in lower cell density detected in the performance of the *in situ* analysis assay. In some cases, if the test biological sample in the cyclic assay of providing and removing probes for hybridization encounters suboptimal stripping, this may result in residual signals from previous cycles. In some aspects, the assessment of the performance of the assay can indicate if one or more steps in processing and assaying of the test biological sample resulted in unexpected results (e.g., unsatisfactory performance). In some aspects, the assessment of the performance of the assay provides pass/fail criteria (e.g., based on metrics described herein) for evaluating the processing and assaying of the test biological sample.

In some embodiments, provided herein is a method, comprising providing a test biological sample. In some embodiments, the test biological sample is a section of a cell pellet comprising a defined mixture of a first cell population and a second cell population. In some embodiments, the method comprises contacting the test sample with a panel of probe sets.

In some embodiments, the test biological sample is a section of a cell pellet comprising a defined mixture of a population of Jurkat cells and a population of Raji cells. In some embodiments, the panel of genes comprises genes that are detectably expressed in Jurkat cells but not in Raji cells and genes that are expressed in the Raji cells but not in Jurkat cells. In some embodiments, the method comprises identifying discrete cells in the test biological sample and comparing the detected RNA transcripts of the genes in the cells to expected expression levels of the genes in Jurkat cells and Raji cells, thereby analyzing the sensitivity and/or specificity of the RNA transcript detection. In some aspects, the assessment of the performance of the assay can indicate if one or more steps in processing and assaying of the test biological sample comprising Jurkat cells and Raji cells resulted in unexpected results (e.g., unsatisfactory performance). In some aspects, the assessment of the performance of the assay provides pass/fail criteria (e.g., based on metrics described herein) for evaluating the processing and assaying of the test biological sample comprising a population of Jurkat cells and a population of Raji cells.

### II. TEST BIOLOGICAL SAMPLE

Provided herein are test biological samples for use in the performance test. The test biological sample is a cell pellet or section of a cell pellet. In some embodiments, the cell pellet is a formalin-fixed, paraffin-embedded (FFPE) cell pellet. In some embodiments, the cell pellet is a fresh frozen cell pellet. In some embodiments, the cell pellet is embedded in an embedding medium and sectioned to provide the test biological sample. In some embodiments, the test biological sample is on a substrate, optionally wherein the substrate is a glass substrate.

The test biological sample comprises a defined mixture of a first cell population and a second cell population. In some embodiments, the first and second cell population are intermixed within the test biological sample. In some embodiments, the ratio of the first cell population and the second cell population in the test biological sample is between about 10:1 and about 1:10, between about 2:1 and about 1:2, or at or about 1:1. In some cases, a ratio of the ratio of the first cell population and the second cell population in the test biological sample at about 1:1 may maximize the confidence of the specificity analysis.

The test biological sample comprises a defined mixture of a first cell population and a second cell population. In some embodiments, the first cell population comprises a population of Jurkat cells and the second cell population comprises a population of Raji cells. In some embodiments, the population of Jurkat cells and the population of Raji cells are intermixed within the test biological sample. In some embodiments, the ratio of Jurkat cells and Raji cells in the test biological sample is between about 10:1 and about 1:10, between about 2:1 and about 1:2, or at or about 1:1. In some cases, a ratio of the ratio of Jurkat cells and Raji cells in the test biological sample at about 1:1 may maximize the confidence of the specificity analysis.

In some embodiments, the density of cells in the biological sample is any suitable density for analysis. In some embodiments, the density of cells in the biological sample is between about 0.1x10⁸ cells per mL and about 10x10⁸ cells per mL. In some embodiments, the density of cells in the biological sample is between about 1x10⁸ cells per mL and about 2x10⁸ cells per mL. In some embodiments, the density of cells in the test biological sample is approximately 1.4x10⁸ cells per mL.

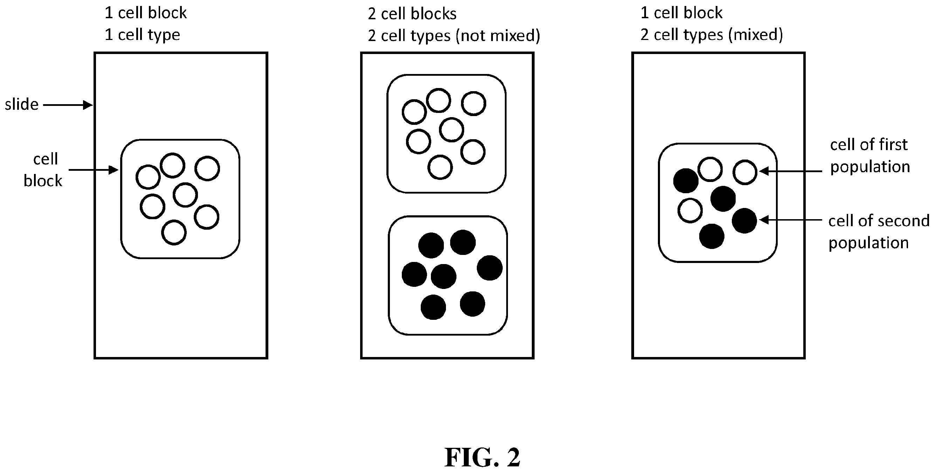

Disclosed but outside the scope of the claims, the first and second cell population are prepared in separate cell pellets. In some embodiments, the test biological sample comprises sections of the first and second cell population, respectively, which are placed in close proximity on the same slide such that they can be analyzed simultaneously for *in situ* analyte detection of both cell populations, for example as shown in **FIG. 2** (middle). In some embodiments, the first and second cell population are prepared in the same cell pellet and are intermixed, for example as shown in **FIG. 2** (right).

The first cell population comprising Jurkat cells and the second cell population comprising Raji cells are prepared in the same cell pellet and are intermixed, for example as shown in **FIG. 2** (right).

In some aspects, the test biological sample is standardized. For example, in some embodiments, the test biological sample meets certain criteria or standards that allow the sample to be used in the performance test to produce expected results. In some aspects, standardization of the test biological sample allows for a plurality of test biological samples to be produced for use in one or more performance test. In some embodiments, standardization of the test biological sample allows for a plurality of the test biological samples to be used in comparing performance of different *in situ* analyte detection assays and/or platforms.

A cell pellet is advantageous for using as the test biological sample. In some aspects, using a cell pellet as the biological sample allows for high reproducibility, spatially expected distribution of signals, ability to assess sensitivity of the performance test, and ability to assess sample preparation conditions. In contrast, using a section from an endogenous tissue sample, such as an intact mouse brain, may reduce the reproducibility of the test biological sample, due to higher sample-to-sample variance. Alternatively, using a printed array for the test biological sample may reduce the ability to assess sensitivity, and reduce ability to assess sample preparation conditions, in comparison to using a cell pellet as the biological sample.A cell pellet comprising a mixture of a first cell population and a second cell population is advantageous for assessing specificity of the assay since mixed cells of different populations are close in proximity in the sample. In some examples, a cell pellet comprising a mixture of a first cell population and a second cell population is advantageous in preparation since it can be easily grown and mixed in a controllable manner (e.g., for cell density in the mix).

The sample comprises two defined cell populations. In some embodiments, the sample comprises a first cell population of Jurkat cells and a second cell population of Raji cells. In some embodiments, the Jurkat cells and Raji cells are intermixed at a ratio of at or about 1:1, such as between about 1:2 and about 2:1.

The cell populations may be from any suitable species. In some embodiments, the first and second cell population are of the same species. In some embodiments, the first cell population and the second cell population are human cells. In some embodiments, the first cell population and the second cell population are mouse cells. Other suitable species may include human, non-human primate, cow, dog, fish, hamster, monkey, mouse, rabbit, rat, frog, bird, or insect species such as fall army worm, cabbage looper, or fruit fly. In some embodiments, the first and second cell population are of different species. For example, the sample comprises a mixture of cells from two different species, e.g., a barnyard sample. In some embodiments, the sample comprises a mixture of human and mouse cells.

A cell population may be from any suitable tissue and/or cell type. In some embodiments, the first cell population and the second cell population are derived from different tissue types. In some embodiments, the first and second cell population are of the same species and of two different cell lineages. In some embodiments, the first cell population and the second cell population are derived from the same tissue type. In some embodiments, the first cell population and the second cell population are of hematopoietic origin.

Suitable tissue/cell types include a blood cell, a hematopoietic cell, an immune cell, a neural cell, a glial cell, a stem cell, an endothelial cell, an epithelial cell, or a fibroblast. Other suitable tissues and/or cell types may include adipose, adrenal gland and/or cortex, aorta, astrocyte, bladder, blood, bone, bone marrow, brain, breast/mammary, bursa, cervix, colon, connective, embryo, endothelial, epithelial, fibroblast, foreskin, glial, heart, keratinocyte, kidney, liver, lung, lymphocyte, macrophage, melanoma, monocyte, muscle, myoblast, neuroblastoma, osteoblast, ovary, pancreas, peritoneum, pituitary, preadipocyte, prostate, rectum, retina, skin, sperm, spleen, stomach, testes, thymus, thyroid, tracheal, umbilicus, or uterus tissue.

A cell population may be from any suitable cell line, such as a cultured cell line. In some embodiments, the first cell population is a first cultured cell line, and the second cell population is a second cultured cell line. In some embodiments, the first cell population is a T cell line, and the second population is a B cell line. In some embodiments, the first cell population are Jurkat cells, and the second cell population are Raji cells. In some embodiments, one or both of the first cell population and second cell population are selected from the group consisting of 293 cells, A549 cells, BHL-100 cells, Caco-2 cells, Chang cells, Daudi cells, H9 cells, HCT-15 cells, HCT116 cells, HeLA cells, Hep-2 cells, HepG2 cells, HL-60 cells, HT-1080 cells, HT-29 cells, HUVEC cells, IM-9 cells, JEG-2 cells, Jurkat cells, K-562 cells, KB cells, KG-1 cells, MCF7 cells, Raji cells, Ramos B cells, THP-1 monocytes, WI-38 cells, and WISH cells.

In some embodiments, one or both of the first cell population and second cell population is an immortalized cell line, such as 3T3 cells, A549 cells, HeLa cells, HEK293 cells, Jurkat cells, Raji cells, OK cells, Ptk2 cells, or Vero cells. In some embodiments, one or both of the first cell population and second cell population can be from any of the following cell lines, for which established culture methods are known: 293, 3T6, A549, A9, AtT-20, BALB/3T3, BHK-21, BHL-100, BT, Caco-2, Chang, CHO-K1, Clone 9, Clone M-3, COS-1, COS-3, COS-7, CRFK, CV-1, D-17, Daudi, GH1, GH3, H9, HaK, HCT-15, HeLa, HEp-2, HL-60, HT-1080, HT-29, HUVEC, I-10, IM-9, JEG-2, Jensen, Jurkat, K-562, KB, KG-1, L2, LLC-WRC 256, McCoy, MCF7, WI-38, WISH, XC, Y-1, Sf9, Sf21, High Five^{™} (BTI-TN-5B1-4), Schneider 2 (S2).

In some aspects, a cell population is selected based on advantageous properties for producing the test biological sample. For example, in some embodiments, the population can be obtained in, or cultured to produce, large numbers of cells. In some embodiments, the cell population is a non-adherent cell line. In some aspects, non-adherent cell lines are suitable for producing large numbers of cells, because they are not limited to growth on vessel surfaces like adherent cell lines. Consequently, a higher cell number to culture medium volume ratio can be obtained in culture.

The test biological sample comprises two cell populations. The two cell populations have different gene expression profiles. Each cell population expresses one or more population-specific genes. In some embodiments, the first and/or second cell population expresses one or more genes that is not expressed in the other cell population. The first and second cell population expresses one or more genes that is not detectably expressed in the other cell population. The first and second population detectably expresses one or more genes that is not detectably expressed in the other cell population. In some embodiments, the first and/or second cell population expresses one or more genes at a significantly higher level (such as at least 2-fold higher, at least 5-fold higher, at least 10-fold higher, at least 50-fold higher, or at least 100-fold higher) than the other cell population. In some embodiments, any of the genes described above can be said to be specific to the first or second cell population, or population-specific (e.g., population-specific, cell-specific, cell-type specific, or cell-line specific). In some aspects, a population-specific gene described herein is population-specific with reference to the cell populations in the test biological sample. For example, the population-specific gene is specific to one population within the test biological sample. It will be understood that the gene may be expressed in other cell types which are not included in the test biological sample, and therefore are not relevant to and do not affect the performance test.

In some embodiments, the two cell populations comprise a population of Jurkat cells and a population of Raji cells. In some embodiments, any of the genes described above can be said to be specific to the population of Jurkat cells or the population of Raji cells, or population-specific (e.g., population-specific, cell-specific, cell-type specific, or cell-line specific). In some aspects, a population-specific gene described herein is population-specific with reference to the population of Jurkat cells in the test biological sample. For example, the population-specific gene with reference to the population of Jurkat cells is specific to the population of Jurkat cells within the test biological sample. It will be understood that the gene that is population-specific with reference to the population of Jurkat cells may be expressed in other cell types which are not included in the test biological sample (i.e., other cell types not including Raji cells), and therefore are not relevant to and do not affect the performance test. In some aspects, a population-specific gene described herein is population-specific with reference to the population of Raji cells in the test biological sample. For example, the population-specific gene with reference to the population of Raji cells is specific to the population of Raji cells within the test biological sample. It will be understood that the gene that is population-specific with reference to the population of Raji cells may be expressed in other cell types which are not included in the test biological sample (i.e., other cell types not including Jurkat cells), and therefore are not relevant to and do not affect the performance test.In some embodiments, a population-specific gene is expressed in one of the two cell populations in the test biological sample and not expressed in the other. In some embodiments, the population-specific gene is expressed at detectable levels in one of the two cell populations and not expressed at detectable levels in the other cell population. In some embodiments, the population-specific gene is expressed at detectable levels in one of the two cell populations and not expressed at detectable levels in the other cell population. In some embodiments, a population-specific gene is selected based on relative expression of the gene in the two cell populations of the biological sample. For example, a population-specific gene may be expressed at a significantly higher level in one population than another population (such as at least 2-fold higher, at least 5-fold higher, at least 10-fold higher, at least 50-fold higher, or at least 100-fold higher) than the other cell population. In some aspects, publicly available gene expression datasets can be used to identify genes that are population-specific with respect to any two populations. In some embodiments, the population-specific gene is present in the genome of one population and not present in the genome of the other.

In some embodiments, the first population of cells comprises a population of Jurkat cells and the second population of cells comprises a population of Raji cells. In some embodiments, a population-specific gene is expressed in the population of Jurkat cells in the test biological sample and not expressed in the population of Raji cells in the test biological sample. In some embodiments, the population-specific gene is expressed at detectable levels in the population of Jurkat cells and not expressed at detectable levels in the population of Raji cells. In some embodiments, a population-specific gene is selected based on relative expression of the gene in Jurkat cells compared to Raji cells. For example, a population-specific gene may be expressed at a significantly higher level in Jurkat cells (such as at least 2-fold higher, at least 5-fold higher, at least 10-fold higher, at least 50-fold higher, or at least 100-fold higher) than in Raji cells.

In some embodiments, the first population of cells comprises a population of Jurkat cells and the second population of cells comprises a population of Raji cells. In some embodiments, a population-specific gene is expressed in the population of Raji cells in the test biological sample and not expressed in the population of Jurkat cells. In some embodiments, a population-specific gene is selected based on relative expression of the gene in Raji cells compared to Jurkat cells. For example, a population-specific gene may be expressed at a significantly higher level in Raji cells (such as at least 2-fold higher, at least 5-fold higher, at least 10-fold higher, at least 50-fold higher, or at least 100-fold higher) than in Jurkat cells.

In some embodiments, population-specific genes can include known markers of a cell population. Markers can be identified for a wide variety of cell populations such as tissues, cell types, and cell lines, for example in assays for gene expression such as RNA-sequencing and flow cytometry. In some aspects, the first cell population and the second cell population can be selected based on characterizations from single-cell RNA sequencing data and mutually exclusive genes can be identified in the first and second cell populations. In some cases, cancerous cell lines may have known deletions in the genome (e.g., identified as a gene that is population-specific).

In some embodiments, population-specific genes can include known markers of a cell population. Markers can be identified for a wide variety of cell populations such as tissues, cell types, and cell lines, for example in assays for gene expression such as RNA-sequencing and flow cytometry. In some aspects, the first cell population and the second cell population can be selected based on characterizations from single-cell RNA sequencing data and mutually exclusive genes can be identified in the first and second cell populations. In some embodiments, the first cell population is a population of Jurkat cells and the second cell population is a population of Raji cells. In some aspects, Jurkat cells and Raji cells can be selected based on characterizations from single-cell RNA sequencing data and mutually exclusive genes can be identified in the cell population of Jurkat cells and the cell population of Raji cells.

Exemplary cell types (and known cell markers) include but are not limited to: astrocytes (GFAP, CD40); basophils (pro-major basic protein 1; CD13); B cells (Pax-5, CD19); embryonic stem cells (c-Myc, Sox2, CD9); endothelial cells (CD31, CD34, CD62E); epithelial cells (CD24, CD44R), fibroblasts (CD10, CD29), hematopoietic stem cells (CD34, Thy1, CD93), macrophages (CD14, CD11b), neurons (NCAM, Thy1, N-cadherin), and T cells (CD3).

In some embodiments, a population-specific gene for a cell population of Jurkat cells is ALDH1A2, BEX3, CD3D, CD3E, CEBPE, DUXAP8, ENO2, FYB1, GSTM3, GSTP1, GTSF1, LEF1, or SIX6. In some embodiments, the gene is population-specific for a cell population of Jurkat cells in a test biological sample comprising a cell population of Jurkat cells and a cell population of Raji cells. In some embodiments, a population-specific gene for a cell population of Raji cells is CDKN2A, ECI1, ELL3, MTAP, POU2AF1, or SPIB. In some embodiments, the gene is population-specific for a cell population of Raji cells in a test biological sample comprising a cell population of Jurkat cells and a cell population of Raji cells.

In some embodiments, two cell populations in the test biological sample, such as the first and second cell population, express one or more of the same gene. In some embodiments, genes that are expressed in both the first cell population and the second cell population are genes that are expressed in a wide variety of cell types, such as genes commonly known as "housekeeping genes." In some embodiments, a gene that is expressed in both the first cell population and the second cell population is AKT1, LAPTM4A, MED28, POLR2A, RAB1B, RFC2, SLC39A3, or SNHG32. In some embodiments, the gene is expressed in both a first cell population of Jurkat cells and a second cell population of Raji cells. In some embodiments, two cell populations in the test biological sample, such as a population of Jurkat cells and a population of Raji cells, express one or more of the same gene. In some embodiments, genes that are expressed in both the population of Jurkat cells and the population of Raji cells are genes that are expressed in a wide variety of cell types, such as genes commonly known as "housekeeping genes." In some embodiments, a gene that is expressed in both the population of Jurkat cells and the population of Raji cells is AKT1, LAPTM4A, MED28, POLR2A, RAB1B, RFC2, SLC39A3, or SNHG32. In some embodiments, the gene is expressed in both a first cell population of Jurkat cells and a second cell population of Raji cells.

In some embodiments, population-specific genes, and/or genes that are expressed in both the first cell population and the second cell population, comprise genes having at least two different expression levels. In some embodiments, the at least two different expression levels are selected from the group consisting of low expression, medium expression, and high expression. In some embodiments, the at least two different expression levels are low expression and high expression. In some embodiments, the at least two different expression levels are medium expression and high expression. In some embodiments, population-specific genes, and/or genes that are expressed in the both the first cell population and the second cell population, comprise (i) genes that have low expression, (ii) genes that have medium expression, and (iii) genes that have high expression.

In some embodiments, genes that have low expression are genes detected at a mean count of 1-5 transcripts per cell by single cell RNA sequencing. In some embodiments, genes that have medium expression are genes detected at a mean count of 5-20 transcripts per cell by single cell RNA sequencing. In some embodiments, genes that have high expression are detected at a mean count of more than 20 transcripts per cell by single cell RNA sequencing.

In some embodiments, genes that have low expression are detected at a mean count of 1-5 transcripts per cell. In some embodiments, genes that have medium expression are detected at a mean count of 5-20 transcripts per cell. In some embodiments, genes that have low expression are detected at a mean count of more than 20 transcripts per cell.

In some embodiments, the expected expression levels of genes that have low expression are a mean count of 1-5 transcripts per cell. In some embodiments, the expected expression levels of genes that have medium expression are a mean count of 5-20 transcripts per cell. In some embodiments, the expected expression levels of genes that have low expression are a mean count of more than 20 transcripts per cell.

In some embodiments, population-specific genes are expressed at a high level in the population of cells that they are specific to. In some embodiments, population-specific genes are expressed at a medium level in the population of cells they are specific to. In some embodiments, population-specific genes are genes specifically expressed in Jurkat cells. In some embodiments, genes specifically expressed in Jurkat cells comprise ALDH1A2, BEX3, CD3D, CD3E, CEBPE, DUXAP8, ENO2, FYB1, GSTM3, GSTP1, GTSF1, LEF1, or SIX6. In some embodiments, genes comprising ALDH1A2, BEX3, CD3D, CD3E, CEBPE, DUXAP8, ENO2, FYB1, GSTM3, GSTP1, GTSF1, LEF1, or SIX6 are expressed at a high level in Jurkat cells. In some embodiments, genes comprising ALDH1A2, BEX3, CD3D, CD3E, CEBPE, DUXAP8, ENO2, FYB1, GSTM3, GSTP1, GTSF1, LEF1, or SIX6 are expressed at a medium level in Jurkat cells. In some embodiments, genes comprising CDKN2A, ECI1, ELL3, MTAP, POU2AF1, or SPIB are not expressed in Jurkat cells. In some embodiments, genes comprising CDKN2A, ECI1, ELL3, MTAP, POU2AF1, or SPIB are not detectably expressed in Jurkat cells. In some embodiments, genes comprising CDKN2A, ECI1, ELL3, MTAP, POU2AF1, or SPIB are expressed at low levels in Jurkat cells.

In some embodiments, population-specific genes are expressed at a high level in the population of cells that they are specific to. In some embodiments, population-specific genes are expressed at a medium level in the population of cells they are specific to. In some embodiments, population-specific genes are genes specifically expressed in Raji cells. In some embodiments, genes specifically expressed in Raji cells comprise CDKN2A, ECI1, ELL3, MTAP, POU2AF1, or SPIB. In some embodiments, genes comprising CDKN2A, ECI1, ELL3, MTAP, POU2AF1, or SPIB are expressed at a high level in Raji cells. In some embodiments, genes comprising CDKN2A, ECI1, ELL3, MTAP, POU2AF1, or SPIB are expressed at a medium level in Raji cells. In some embodiments, genes comprising ALDH1A2, BEX3, CD3D, CD3E, CEBPE, DUXAP8, ENO2, FYB1, GSTM3, GSTP1, GTSF1, LEF1, or SIX6 are not expressed in Raji cells. In some embodiments, genes comprising ALDH1A2, BEX3, CD3D, CD3E, CEBPE, DUXAP8, ENO2, FYB1, GSTM3, GSTP1, GTSF1, LEF1, or SIX6 are not detectably expressed in Raji cells. In some embodiments, genes comprising ALDH1A2, BEX3, CD3D, CD3E, CEBPE, DUXAP8, ENO2, FYB1, GSTM3, GSTP1, GTSF1, LEF1, or SIX6 are expressed at low levels in Raji cells.

In some embodiments, target sequences of RNA transcripts or product thereof of a panel of genes comprising POLR2A, DUXAP8, RFC2, CD3D, FYB1, MTAP, AKT1, CDKN2A, and SPIB is detected. In some embodiments, the panel of genes comprises genes specific for a first population and genes specific for a second population.

In some aspects, a cell population of the sample can be a modified cell population, such as a modified cell population derived from any cell population described herein. In some embodiments, the cell population is modified to affect gene expression. For example, the cell population may comprise one or more mutations that reduce or eliminate expression of a gene, such as a deletion and/or loss-of-function mutation. In some embodiments, the cell population is modified to express a gene that is not endogenous, such as a transgene. Any suitable transgene can be selected. In some embodiments, the modification allows the cell population to be distinguished from another cell population in the biological sample based on gene expression. For example, in a mixture of cells comprising a first population and a second population, eliminating expression of a gene in the first population can allow expression of the gene in the second population to be specific to the second population. Alternatively, expression of a transgene in the first population but not the second population allows expression of the transgene to be specific to the first population.

In some embodiments, the test biological sample can be attached to a substrate. In some embodiments, a substrate herein can be any support that is insoluble in aqueous liquid and which allows for positioning of biological samples, analytes, features, and/or reagents (e.g., probes) on the support. Attachment of the test biological sample can be irreversible or reversible, depending upon the nature of the sample and subsequent steps in the analytical method. In certain embodiments, the test biological sample can be attached to the substrate reversibly by applying a suitable polymer coating to the substrate, and contacting the sample to the polymer coating. The test biological sample can then be detached from the substrate, e.g., using an organic solvent that at least partially dissolves the polymer coating. Hydrogels are examples of polymers that are suitable for this purpose. In some embodiments, the substrate can be coated or functionalized with one or more substances to facilitate attachment of the test biological sample to the substrate. Suitable substances that can be used to coat or functionalize the substrate include, but are not limited to, lectins, poly-lysine, antibodies, and polysaccharides.

In some embodiments, the test biological sample is a section of a cell pellet. The thickness of the section can be a fraction of (e.g., less than 0.9, 0.8, 0.7, 0.6, 0.5, 0.4, 0.3, 0.2, or 0.1) the maximum cross-sectional dimension of a cell. However, sections having a thickness that is larger than the maximum cross-section cell dimension can also be used. For example, cryostat sections can be used, which can be, e.g., 10-20 µm thick. In some embodiments, the thickness of the section can be at least 0.1, 0.2, 0.3, 0.4, 0.5, 0.7, 1.0, 1.5, 2, 3, 4, 5, 6, 7, 8, 9, 10, 12, 13, 14, 15, 20, 30, 40, or 50 µm. Thicker sections can also be used if desired or convenient, e.g., at least 70, 80, 90, or 100 µm or more. Typically, the thickness of a tissue section is between 1-100 µm, 1-50 µm, 1-30 µm, 1-25 µm, 1-20 µm, 1-15 µm, 1-10 µm, 2-8 µm, 3-7 µm, or 4-6 µm, but as mentioned above, sections with thicknesses larger or smaller than these ranges can also be analysed. Multiple sections can also be obtained from a single cell pellet. In some embodiments, a section of a biological sample is prepared and stored.

In some embodiments, the test biological sample (e.g., the cell pellet), can be prepared by deep freezing. The frozen test biological sample can be sectioned, e.g., thinly sliced, onto a substrate surface (e.g., a slide) using any number of suitable methods. For example, the test biological sample can be prepared using a chilled microtome (e.g., a cryostat) set at a temperature suitable to maintain the chemical properties of the nucleic acids in the sample. Such a temperature can be, e.g., less than -15°C, less than -20°C, or less than -25°C.

In some embodiments, the test biological sample can be prepared using formalin-fixation and paraffin-embedding (FFPE), which are established methods. In some embodiments, the test biological sample can be prepared using formalin-fixation and paraffin-embedding. Following fixation of the test biological sample and embedding in a paraffin or resin block, the sample can be sectioned as described above. Prior to analysis, the paraffin-embedding material can be removed from the section (e.g., deparaffinization) by incubating the section in an appropriate solvent (e.g., xylene) followed by a rinse (e.g., 99.5% ethanol for 2 minutes, 96% ethanol for 2 minutes, and 70% ethanol for 2 minutes).

As an alternative to formalin fixation described above, the test biological sample can be fixed in any of a variety of other fixatives. For example, the test biological sample can be fixed via immersion in ethanol, methanol, acetone, paraformaldehyde (PFA)-Triton, and combinations thereof. When acetone fixation is performed, pre-permeabilization steps (described below) may not be performed. Alternatively, acetone fixation can be performed in conjunction with permeabilization steps.

In some embodiments, the methods provided herein comprises one or more post-fixing (also referred to as postfixation) steps. In some embodiments, one or more post-fixing step is performed after contacting the test biological sample with a polynucleotide disclosed herein, e.g., one or more probes such as a circular or padlock probe. In some embodiments, one or more post-fixing step is performed after a hybridization complex comprising a probe and a target is formed in the test biological sample. In some embodiments, one or more post-fixing step is performed prior to a ligation reaction disclosed herein, such as the ligation to circularize a padlock probe.

In some embodiments, one or more post-fixing step is performed after contacting the test biological sample with a binding or labeling agent (e.g., an antibody or antigen binding fragment thereof) for a non-nucleic acid analyte such as a protein analyte. The labeling agent can comprise a nucleic acid molecule (e.g., reporter oligonucleotide) comprising a sequence corresponding to the labeling agent and therefore corresponds to (e.g., uniquely identifies) the analyte. In some embodiments, the labeling agent can comprise a reporter oligonucleotide comprising one or more barcode sequences.

A post-fixing step may be performed using any suitable fixation reagent disclosed herein, for example, 3% (w/v) paraformaldehyde in DEPC-PBS.

As an alternative to paraffin embedding described above, the test biological sample can be embedded in any of a variety of other embedding materials. In some cases, the embedding material can be removed e.g., prior to analysis of sections obtained from the sample. Suitable embedding materials include, but are not limited to, waxes, resins (e.g., methacrylate resins), epoxies, and agar.

In some embodiments, the test biological sample can be embedded in a matrix (e.g., a hydrogel matrix). Embedding the sample in this manner typically involves contacting the biological sample with a hydrogel such that the test biological sample becomes surrounded by the hydrogel. For example, the test biological sample can be embedded by contacting the test biological sample with a suitable polymer material, and activating the polymer material to form a hydrogel. In some embodiments, the hydrogel is formed such that the hydrogel is internalized within the test biological sample.

In some embodiments, the test biological sample is immobilized in the hydrogel via cross-linking of the polymer material that forms the hydrogel. Cross-linking can be performed chemically and/or photochemically, or alternatively by any other suitable hydrogel-formation method.

Additional methods and aspects of hydrogel embedding of biological samples are described for example in Chen et al., Science 347(6221):543-548, 2015.

To facilitate visualization, the test biological sample can be stained using a wide variety of stains and staining techniques. In some embodiments, for example, the test biological sample can be stained using any number of stains and/or immunohistochemical reagents. One or more staining steps may be performed to prepare or process the test biological sample for an assay described herein (e.g., the performance test) or may be performed during and/or after an assay. In some embodiments, the test biological sample can be contacted with one or more nucleic acid stains, membrane stains (e.g., cellular or nuclear membrane), cytological stains, or combinations thereof. In some examples, the stain may be specific to proteins, phospholipids, DNA (e.g., dsDNA, ssDNA), RNA, an organelle or compartment of the cell. The test biological sample may be contacted with one or more labeled antibodies (e.g., a primary antibody specific for the analyte of interest and a labeled secondary antibody specific for the primary antibody). In some embodiments, cells in the test biological sample can be segmented using one or more images taken of the stained sample.

In some embodiments, the stain is performed using a lipophilic dye. In some examples, the staining is performed with a lipophilic carbocyanine or aminostyryl dye, or analogs thereof (e.g, DiI, DiO, DiR, DiD). Other cell membrane stains may include FM and RH dyes or immunohistochemical reagents specific for cell membrane proteins. In some examples, the stain may include but is not limited to, acridine orange, acid fuchsin, Bismarck brown, carmine, coomassie blue, cresyl violet, DAPI, eosin, ethidium bromide, acid fuchsine, haematoxylin, Hoechst stains, iodine, methyl green, methylene blue, neutral red, Nile blue, Nile red, osmium tetroxide, ruthenium red, propidium iodide, rhodamine (e.g., rhodamine B), or safranine, or derivatives thereof. In some embodiments, the sample may be stained with haematoxylin and eosin (H&E).

The test biological sample can be stained using hematoxylin and eosin (H&E) staining techniques, using Papanicolaou staining techniques, Masson's trichrome staining techniques, silver staining techniques, Sudan staining techniques, and/or using Periodic Acid Schiff (PAS) staining techniques. PAS staining is typically performed after formalin or acetone fixation. In some embodiments, the sample can be stained using Romanowsky stain, including Wright's stain, Jenner's stain, Can-Grunwald stain, Leishman stain, and Giemsa stain.

In some embodiments, the test biological sample can be destained. Any suitable methods of destaining or discoloring a biological sample may be utilized and generally depend on the nature of the stain(s) applied to the test biological sample. For example, in some embodiments, one or more immunofluorescent stains are applied to the test biological sample via antibody coupling. Such stains can be removed using techniques such as cleavage of disulfide linkages via treatment with a reducing agent and detergent washing, chaotropic salt treatment, treatment with antigen retrieval solution, and treatment with an acidic glycine buffer. Methods for multiplexed staining and destaining are described, for example, in Bolognesi et al., J. Histochem. Cytochem. 2017; 65(8): 431-444, Lin et al., Nat Commun. 2015; 6:8390, Pirici et al., J. Histochem. Cytochem. 2009; 57:567-75, and Glass et al., J. Histochem. Cytochem. 2009; 57:899-905.

In some embodiments, the test biological sample is embedded in a matrix (e.g., a hydrogel) can be isometrically expanded. Isometric expansion methods that can be used include hydration, a preparative step in expansion microscopy, as described in, e.g., Chen et al., Science 347(6221):543-548, 2015 and U.S. Pat. 10,059,990.

Isometric expansion can be performed by anchoring one or more components of the test biological sample to a gel, followed by gel formation, proteolysis, and swelling. In some embodiments, analytes in the sample, products of the analytes, and/or probes associated with analytes in the sample can be anchored to the matrix (e.g., hydrogel). Isometric expansion of the test biological sample can occur prior to immobilization of the test biological sample on a substrate, or after the test biological sample is immobilized to a substrate. In some embodiments, the isometrically expanded test biological sample can be removed from the substrate prior to contacting the substrate with probes disclosed herein.

In some embodiments, proteins in the test biological sample are anchored to a swellable gel such as a polyelectrolyte gel. An antibody can be directed to the protein before, after, or in conjunction with being anchored to the swellable gel. DNA and/or RNA in a biological sample can also be anchored to the swellable gel via a suitable linker. Examples of such linkers include, but are not limited to, 6-((Acryloyl)amino) hexanoic acid (Acryloyl-X SE) (available from ThermoFisher, Waltham, MA), Label-IT Amine (available from MirusBio, Madison, WI) and Label X (described for example in Chen et al., Nat. Methods 13:679-684, 2016 and U.S. Pat. 10,059,990.

Isometric expansion of the test biological sample can increase the spatial resolution of the subsequent analysis of the sample. The increased resolution in spatial profiling can be determined by comparison of an isometrically expanded test biological sample with a sample that has not been isometrically expanded.

In some embodiments, the test biological sample is isometrically expanded to a size at least 2x, 2.1x, 2.2x, 2.3x, 2.4x, 2.5x, 2.6x, 2.7x, 2.8x, 2.9x, 3x, 3.1x, 3.2x, 3.3x, 3.4x, 3.5x, 3.6x, 3.7x, 3.8x, 3.9x, 4x, 4.1x, 4.2x, 4.3x, 4.4x, 4.5x, 4.6x, 4.7x, 4.8x, or 4.9x its non-expanded size. In some embodiments, the sample is isometrically expanded to at least 2x and less than 20x of its non-expanded size.

In some embodiments, the test biological sample is reversibly cross-linked prior to or during an *in situ* assay. In some aspects, the analytes, polynucleotides and/or amplification product (e.g., amplicon) of an analyte or a probe bound thereto can be anchored to a polymer matrix. For example, the polymer matrix can be a hydrogel. In some embodiments, one or more of the polynucleotide probe(s) and/or amplification product (e.g., amplicon) thereof can be modified to contain functional groups that can be used as an anchoring site to attach the polynucleotide probes and/or amplification product to a polymer matrix. In some embodiments, a modified probe comprising oligo dT may be used to bind to mRNA molecules of interest, followed by reversible or irreversible crosslinking of the mRNA molecules.

In some embodiments, the test biological sample is immobilized in a hydrogel via cross-linking of the polymer material that forms the hydrogel. Cross-linking can be performed chemically and/or photochemically, or alternatively by any other suitable hydrogel-formation method. A hydrogel may include a macromolecular polymer gel including a network. Within the network, some polymer chains can optionally be cross-linked, although cross-linking does not always occur.

In some embodiments, a hydrogel can include hydrogel subunits, such as, but not limited to, acrylamide, bis-acrylamide, polyacrylamide and derivatives thereof, poly(ethylene glycol) and derivatives thereof (e.g. PEG-acrylate (PEG-DA), PEG-RGD), gelatin-methacryloyl (GelMA), methacrylated hyaluronic acid (MeHA), polyaliphatic polyurethanes, polyether polyurethanes, polyester polyurethanes, polyethylene copolymers, polyamides, polyvinyl alcohols, polypropylene glycol, polytetramethylene oxide, polyvinyl pyrrolidone, polyacrylamide, poly(hydroxyethyl acrylate), and poly(hydroxyethyl methacrylate), collagen, hyaluronic acid, chitosan, dextran, agarose, gelatin, alginate, protein polymers, methylcellulose, and the like, and combinations thereof.

In some embodiments, a hydrogel includes a hybrid material, e.g., the hydrogel material includes elements of both synthetic and natural polymers. Examples of suitable hydrogels are described, for example, in U.S. Patent Nos. 6,391,937, 9,512,422, and 9,889,422, and in U.S. Patent Application Publication Nos. 2017/0253918, 2018/0052081 and 2010/0055733.

In some embodiments, the hydrogel can form the substrate. In some embodiments, the substrate includes a hydrogel and one or more second materials. In some embodiments, the hydrogel is placed on top of one or more second materials. For example, the hydrogel can be pre-formed and then placed on top of, underneath, or in any other configuration with one or more second materials. In some embodiments, hydrogel formation occurs after contacting one or more second materials during formation of the substrate. Hydrogel formation can also occur within a structure (e.g., wells, ridges, projections, and/or markings) located on a substrate.

In some embodiments, hydrogel formation on a substrate occurs before, contemporaneously with, or after probes are provided to the sample. For example, hydrogel formation can be performed on the substrate already containing the probes.

In some embodiments, hydrogel formation occurs within the test biological sample. In some embodiments, the test biological sample (i.e., cell pellet) is embedded in a hydrogel. In some embodiments, hydrogel subunits are infused into the test biological sample, and polymerization of the hydrogel is initiated by an external or internal stimulus.

In embodiments in which a hydrogel is formed within the test biological sample, functionalization chemistry can be used. In some embodiments, functionalization chemistry includes hydrogel-tissue chemistry (HTC). Any hydrogel-tissue backbone (e.g., synthetic or native) suitable for HTC can be used for anchoring biological macromolecules and modulating functionalization. Non-limiting examples of methods using HTC backbone variants include CLARITY, PACT, ExM, SWITCH and ePACT. In some embodiments, hydrogel formation within the test biological sample is permanent. For example, biological macromolecules can permanently adhere to the hydrogel allowing multiple rounds of interrogation. In some embodiments, hydrogel formation within the test biological sample is reversible.

In some embodiments, additional reagents are added to the hydrogel subunits before, contemporaneously with, and/or after polymerization. For example, additional reagents can include but are not limited to oligonucleotides (e.g., probes), endonucleases to fragment DNA, fragmentation buffer for DNA, DNA polymerase enzymes, dNTPs used to amplify the nucleic acid and to attach the barcode to the amplified fragments. Other enzymes can be used, including without limitation, RNA polymerase, ligase, proteinase K, and DNAse. Additional reagents can also include reverse transcriptase enzymes, including enzymes with terminal transferase activity, primers, and switch oligonucleotides. In some embodiments, optical labels are added to the hydrogel subunits before, contemporaneously with, and/or after polymerization.

In some embodiments, HTC reagents are added to the hydrogel before, contemporaneously with, and/or after polymerization. In some embodiments, a cell labeling agent is added to the hydrogel before, contemporaneously with, and/or after polymerization. In some embodiments, a cell-penetrating agent is added to the hydrogel before, contemporaneously with, and/or after polymerization.

Hydrogels embedded within the test biological sample can be cleared using any suitable method. For example, electrophoretic tissue clearing methods can be used to remove biological macromolecules from the hydrogel-embedded sample. In some embodiments, a hydrogel-embedded the test biological sample is stored before or after clearing of hydrogel, in a medium (e.g., a mounting medium, methylcellulose, or other semi-solid mediums).

In some embodiments, a method disclosed herein comprises de-crosslinking the reversibly cross-linked test biological sample. The de-crosslinking does not need to be complete. In some embodiments, only a portion of crosslinked molecules in the reversibly cross-linked test biological sample are de-crosslinked and allowed to migrate.

In some embodiments, the test biological sample can be permeabilized to facilitate transfer of species (such as probes) into the sample. If a sample is not permeabilized sufficiently, the transfer of species (such as probes) into the sample may be too low to enable adequate analysis. Conversely, if a sample is too permeable, the relative spatial relationship of the analytes within the sample can be lost. Hence, a balance between permeabilizing the test biological sample enough to obtain good signal intensity while still maintaining the spatial resolution of the analyte distribution in the test biological sample is desirable.

In general, a biological sample can be permeabilized by exposing the sample to one or more permeabilizing agents. Suitable agents for this purpose include, but are not limited to, organic solvents (e.g., acetone, ethanol, and methanol), cross-linking agents (e.g., paraformaldehyde), detergents (e.g., saponin, Triton X-100^{™} or Tween-20^{™}), and enzymes (e.g., trypsin, proteases). In some embodiments, the test biological sample can be incubated with a cellular permeabilizing agent to facilitate permeabilization of the sample. Additional methods for sample permeabilization are described, for example, in Jamur et al., Method Mol. Biol. 588:63-66, 2010. Any suitable method for sample permeabilization can generally be used in connection with the test biological sample described herein.

In some embodiments, the test biological sample can be permeabilized by adding one or more lysis reagents to the sample. Examples of suitable lysis agents include, but are not limited to, bioactive reagents such as lysis enzymes that are used for lysis of different cell types, such as lysozymes, achromopeptidase, lysostaphin, labiase, kitalase, lyticase, and a variety of other commercially available lysis enzymes.

Other lysis agents can additionally or alternatively be added to the test biological sample to facilitate permeabilization. For example, surfactant-based lysis solutions can be used to lyse sample cells. Lysis solutions can include ionic surfactants such as, for example, sarcosyl and sodium dodecyl sulfate (SDS). More generally, chemical lysis agents can include, without limitation, organic solvents, chelating agents, detergents, surfactants, and chaotropic agents.

In some embodiments, the test biological sample can be permeabilized by non-chemical permeabilization methods. For example, non-chemical permeabilization methods that can be used include, but are not limited to, physical lysis techniques such as electroporation, mechanical permeabilization methods (e.g., bead beating using a homogenizer and grinding balls to mechanically disrupt sample tissue structures), acoustic permeabilization (e.g., sonication), and thermal lysis techniques such as heating to induce thermal permeabilization of the sample.

Additional reagents can be added to the test biological sample to perform various functions prior to analysis of the sample. In some embodiments, DNase and RNase inactivating agents or inhibitors such as proteinase K, and/or chelating agents such as EDTA, can be added to the test biological sample. For example, a method disclosed herein may comprise a step for increasing accessibility of a nucleic acid for binding, e.g., a denaturation step to open up DNA in a cell for hybridization by a probe. For example, proteinase K treatment may be used to free up DNA with proteins bound thereto.

### III. PROBES, DETECTION, AND ANALYSIS

Disclosed herein in some aspects are nucleic acid probes and/or probe sets and immobilization oligonucleotides that are introduced into a cell or used to otherwise contact a biological sample such as a tissue sample. The probes may comprise any of a variety of entities that can hybridize to a nucleic acid, typically by Watson-Crick base pairing, such as DNA, RNA, LNA, PNA, etc., depending on the application. The nucleic acid probe(s) and immobilization oligonucleotides typically contains a hybridization region that is able to bind to at least a portion of a target nucleic acid, in some embodiments specifically. The nucleic acid probe may be able to bind to a specific target nucleic acid (e.g., an mRNA, or other nucleic acids as discussed herein). In some embodiments, the nucleic acid probes may be detected using a detectable label, and/or by using detectably labeled nucleic acid probes able to bind to the nucleic acid probes or amplification products thereof, directly or via an intermediate probe. In some embodiments, products of the hybridized probes or probe sets are amplification products of the hybridized probes or probe sets. In some embodiments, products of the hybridized probes or probe sets are rolling circle amplification products (RCPs). In some embodiments, the nucleic acid probes are compatible with one or more biological and/or chemical reactions. For instance, a primary nucleic acid probe disclosed herein can serve as a template or primer for a polymerase (e.g., for rolling circle amplification), a template or substrate for a ligase, a substrate for a click chemistry reaction, and/or a substrate for a nuclease (e.g., endonuclease for cleavage).

### A. Panels of Probes or Probe Sets

In some aspects, provided herein are panels of probes or probe sets for detecting a plurality of analytes in the biological sample (e.g., RNA transcripts) for assessing performance of *in situ* analyte detection assay. In some embodiments, the panels of probes or probe sets are for detection of population-specific genes of the first cell population and the second cell population. For example, the target analytes (e.g., genes) for detection can comprise any of the population-specific (e.g., population-specific, cell-specific, cell-type specific, or cell-line specific) genes described in Section II. In some cases, the target analytes (e.g., genes) for detection can comprise genes that are expressed at least two different expression levels (e.g., high, medium, or low expression level) as described in Section II. In some aspects, the target analytes (e.g., genes) can be selected based on expression in the first cell population and the second cell population. For example, the panels of probes or probe sets are used to detect selected genes that are population-specific, cell-specific, cell-type specific, or cell-line specific. In some examples, the panels of probes or probe sets are used to detect selected genes that are expressed in a high, medium, or low level in the first cell population or the second cell population.

In some aspects, provided herein are panels of probes for detecting a plurality of analytes in the biological sample (e.g., RNA transcripts) for assessing performance of *in situ* analyte detection assay. In some embodiments, the panels of probes are for detection of population-specific genes of the first cell population and the second cell population. For example, the target analytes (e.g., genes) for detection can comprise any of the population-specific (e.g., population-specific, cell-specific, cell-type specific, or cell-line specific) genes described in Section II. In some cases, the target analytes (e.g., genes) for detection can comprise genes that are expressed at least two different expression levels (e.g., high, medium, or low expression level) as described in Section II. In some aspects, the target analytes (e.g., genes) can be selected based on expression in the first cell population and the second cell population. For example, the panels of probes are used to detect selected genes that are population-specific, cell-specific, cell-type specific, or cell-line specific. In some examples, the panels of probes are used to detect selected genes that are expressed in a high, medium, or low level in the first cell population or the second cell population.

In some aspects, provided herein are panels of probe sets for detecting a plurality of analytes in the biological sample (e.g., RNA transcripts) for assessing performance of *in situ* analyte detection assay. In some embodiments, the panels of probe sets are for detection of population-specific genes of the first cell population and the second cell population. For example, the target analytes (e.g., genes) for detection can comprise any of the population-specific (e.g., population-specific, cell-specific, cell-type specific, or cell-line specific) genes described in Section II. In some cases, the target analytes (e.g., genes) for detection can comprise genes that are expressed at least two different expression levels (e.g., high, medium, or low expression level) as described in Section II. In some aspects, the target analytes (e.g., genes) can be selected based on expression in the first cell population and the second cell population. For example, the panels of probe sets are used to detect selected genes that are population-specific, cell-specific, cell-type specific, or cell-line specific. In some examples, the panels of probe sets are used to detect selected genes that are expressed in a high, medium, or low level in the first cell population or the second cell population.

In some aspects, provided herein are panels of probes for detecting a plurality of analytes in the biological sample (e.g., RNA transcripts) for assessing performance of *in situ* analyte detection assay. In some embodiments, the panels of probes are for detection of population-specific genes of Jurkat cells and/or Raji cells. For example, the target analytes (e.g., genes) for detection can comprise any of the population-specific (e.g., population-specific, cell-specific, cell-type specific, or cell-line specific) genes described in Section II. In some cases, the target analytes (e.g., genes) for detection can comprise genes that are expressed at least two different expression levels (e.g., high, medium, or low expression level) as described in Section II. In some aspects, the target analytes (e.g., genes) can be selected based on expression in Jurkat cells and/or Raji cells. For example, the panels of probes are used to detect selected genes that are population-specific, cell-specific, cell-type specific, or cell-line specific. In some examples, the panels of probes or probe sets are used to detect selected genes that are expressed in a high, medium, or low level in Jurkat cells or Raji cells.

In some aspects, provided herein are panels of probe sets for detecting a plurality of analytes in the biological sample (e.g., RNA transcripts) for assessing performance of *in situ* analyte detection assay. In some embodiments, the panels of probe sets are for detection of population-specific genes of Jurkat cells and/or Raji cells. For example, the target analytes (e.g., genes) for detection can comprise any of the population-specific (e.g., population-specific, cell-specific, cell-type specific, or cell-line specific) genes described in Section II. In some cases, the target analytes (e.g., genes) for detection can comprise genes that are expressed at least two different expression levels (e.g., high, medium, or low expression level) as described in Section II. In some aspects, the target analytes (e.g., genes) can be selected based on expression in Jurkat cells and/or Raji cells. For example, the panels of probe sets are used to detect selected genes that are population-specific, cell-specific, cell-type specific, or cell-line specific. In some examples, the panels of probe sets are used to detect selected genes that are expressed in a high, medium, or low level in Jurkat cells and/or Raji cells.

In some embodiments, a first panel of probes or probe sets is provided, wherein each probe or probe set of the first panel comprises a recognition sequence complementary to a target analyte (e.g., sequence of an RNA transcript or product thereof) of a first set of genes expressed in the first cell population. In some embodiments, the first set of genes is specifically expressed in the first population (e.g., at least 10 fold, at least 50-fold, or at least 100-fold higher expression in the first cell population than in the second cell population). In some embodiments, first set of genes are not detectably expressed in the second cell population. In some embodiments, the first set of genes are not detectably expressed in the second cell population by single cell RNA sequencing. In some embodiments, the first set of genes are not detectably expressed in the second cell population by bulk RNA sequencing of the second cell population. In some embodiments, the first set of genes are not detectably expressed in the second cell population by *in situ* detection of a probe or probe set or a product thereof (e.g., an RCA product thereof) specifically associated with an RNA transcript of the gene in the second cell population. In some embodiments, products of the hybridized probes or probe sets are amplification products of the hybridized probes or probe sets. In some embodiments, products of the hybridized probes or probe sets are rolling circle amplification products (RCPs). Thus, in some aspects, detection of a probe or probe set or product thereof comprising a barcode sequence corresponding to a gene of the first set of genes in a cell of the second cell population indicates non-specific probe hybridization, ligation, amplification, and/or detection. In some embodiments, detection of the probes or probe sets of the first panel of probes or probe sets specifically in the first cell population indicates specificity of the assay. In some embodiments, detection of rolling circle amplification products of the probes or probe sets of the first panel of probes or probe sets specifically in the first cell population indicates specificity of the assay.

In some embodiments, a first panel of probes is provided, wherein each probe of the first panel comprises a recognition sequence complementary to a target analyte (e.g., sequence of an RNA transcript or product thereof) of a first set of genes expressed in the first cell population. In some embodiments, the first set of genes is specifically expressed in the first population (e.g., at least 10 fold, at least 50-fold, or at least 100-fold higher expression in the first cell population than in the second cell population). In some embodiments, first set of genes are not detectably expressed in the second cell population. In some embodiments, the first set of genes are not detectably expressed in the second cell population by single cell RNA sequencing. In some embodiments, the first set of genes are not detectably expressed in the second cell population by bulk RNA sequencing of the second cell population. In some embodiments, the first set of genes are not detectably expressed in the second cell population by *in situ* detection of a probe or a product thereof (e.g., an RCA product thereof) specifically associated with an RNA transcript of the gene in the second cell population. In some embodiments, products of the hybridized probes or probe sets are amplification products of the hybridized probes or probe sets. In some embodiments, products of the hybridized probes or probe sets are rolling circle amplification products (RCPs). Thus, in some aspects, detection of a probe or product thereof comprising a barcode sequence corresponding to a gene of the first set of genes in a cell of the second cell population indicates non-specific probe hybridization, ligation, amplification, and/or detection. In some embodiments, detection of the probes of the first panel of probes specifically in the first cell population indicates specificity of the assay. In some embodiments, detection of rolling circle amplification products of the probes of the first panel of probes specifically in the first cell population indicates specificity of the assay.

In some embodiments, a first panel of probe sets is provided, wherein each probe set of the first panel comprises a recognition sequence complementary to a target analyte (e.g., sequence of an RNA transcript or product thereof) of a first set of genes expressed in the first cell population. In some embodiments, the first set of genes is specifically expressed in the first population (e.g., at least 10 fold, at least 50-fold, or at least 100-fold higher expression in the first cell population than in the second cell population). In some embodiments, first set of genes are not detectably expressed in the second cell population. In some embodiments, the first set of genes are not detectably expressed in the second cell population by single cell RNA sequencing. In some embodiments, the first set of genes are not detectably expressed in the second cell population by bulk RNA sequencing of the second cell population. In some embodiments, the first set of genes are not detectably expressed in the second cell population by *in situ* detection of a probe set or a product thereof (e.g., an RCA product thereof) specifically associated with an RNA transcript of the gene in the second cell population. In some embodiments, products of the hybridized probes or probe sets are amplification products of the hybridized probes or probe sets. In some embodiments, products of the hybridized probes or probe sets are rolling circle amplification products (RCPs). Thus, in some aspects, detection of a probe set or product thereof comprising a barcode sequence corresponding to a gene of the first set of genes in a cell of the second cell population indicates non-specific probe hybridization, ligation, amplification, and/or detection. In some embodiments, detection of the probe sets of the first panel of probe sets specifically in the first cell population indicates specificity of the assay. In some embodiments, detection of rolling circle amplification products of the probe sets of the first panel of probe sets specifically in the first cell population indicates specificity of the assay.

In some embodiments, a first panel of probes is provided, wherein each probe of the first panel comprises a recognition sequence complementary to a target analyte (e.g., sequence of an RNA transcript or product thereof) of a first set of genes expressed in Jurkat cells. In some embodiments, the first set of genes is specifically expressed in Jurkat cells (e.g., at least 10 fold, at least 50-fold, or at least 100-fold higher expression in Jurkat cells than in Raji cells). In some embodiments, the first set of genes are not detectably expressed in Raji cells. In some embodiments, the first set of genes are not detectably expressed in Raji cells by single cell RNA sequencing. In some embodiments, the first set of genes are not detectably expressed in Raji cells by bulk RNA sequencing of the population of Raji cells. In some embodiments, the first set of genes are not detectably expressed in Raji cells by *in situ* detection of a probe or probe set or a product thereof (e.g., an RCA product thereof) specifically associated with an RNA transcript of the gene in the population of Raji cells. In some embodiments, products of the hybridized probes are amplification products of the hybridized probes. In some embodiments, products of the hybridized probes are rolling circle amplification products (RCPs). Thus, in some aspects, detection of a probe or product thereof (e.g., an RCP) comprising a barcode sequence corresponding to a gene of the first set of genes in a cell of the population of Raji cells indicates non-specific probe hybridization, ligation, amplification, and/or detection. In some embodiments, detection of the probes of the first panel of probes specifically in the population of Jurkat cells indicates specificity of the assay. In some embodiments, detection of rolling circle amplification products of the probes of the first panel of probes specifically in the population of Jurkat cells indicates specificity of the assay.

In some embodiments, a first panel of probe sets is provided, wherein each probe set of the first panel comprises a recognition sequence complementary to a target analyte (e.g., sequence of an RNA transcript or product thereof) of a first set of genes expressed in Jurkat cells. In some embodiments, the first set of genes is specifically expressed in Jurkat cells (e.g., at least 10 fold, at least 50-fold, or at least 100-fold higher expression in Jurkat cells than in Raji cells). In some embodiments, first set of genes are not detectably expressed in Raji cells. In some embodiments, the first set of genes are not detectably expressed in Raji cells by single cell RNA sequencing. In some embodiments, the first set of genes are not detectably expressed in Raji cells by bulk RNA sequencing of the population of Raji cells. In some embodiments, the first set of genes are not detectably expressed in Raji cells by *in situ* detection of a probe set or a product thereof (e.g., an RCA product thereof) specifically associated with an RNA transcript of the gene in the population of Raji cells. In some embodiments, products of the hybridized probe sets are amplification products of the hybridized probe sets. In some embodiments, products of the hybridized probe sets are rolling circle amplification products (RCPs). Thus, in some aspects, detection of a probe set or product thereof (e.g., an RCP) comprising a barcode sequence corresponding to a gene of the first set of genes in a cell of the population of Raji cells indicates non-specific probe hybridization, ligation, amplification, and/or detection. In some embodiments, detection of the probe sets of the first panel of probe sets specifically in the population of Jurkat cells indicates specificity of the assay. In some embodiments, detection of rolling circle amplification products of the probe sets of the first panel of probe sets specifically in the population of Jurkat cells indicates specificity of the assay.

In some embodiments, a second panel of probes or probe sets is provided, wherein each probe or probe set of the second panel comprises a recognition sequence complementary to a target analyte (e.g., sequence of an RNA transcript or product thereof) of a second set of genes expressed in the second cell population. In some embodiments, the second set of genes is specifically expressed in the second population (e.g., at least 10 fold, at least 50-fold, or at least 100-fold higher expression in the second cell population than in the first cell population). In some embodiments, second set of genes are not detectably expressed in the first cell population. In some embodiments, the second set of genes are not detectably expressed in the first cell population by single cell RNA sequencing. In some embodiments, the second set of genes are not detectably expressed in the first cell population by bulk RNA sequencing of the first cell population. In some embodiments, the second set of genes are not detectably expressed in the first cell population by *in situ* detection of a probe or probe set or a product thereof (e.g., an RCA product thereof) specifically associated with an RNA transcript of the gene in the first cell population. In some embodiments, products of the hybridized probes or probe sets are amplification products of the hybridized probes or probe sets. In some embodiments, products of the hybridized probes or probe sets are rolling circle amplification products (RCPs). Thus, in some aspects, detection of a probe or probe set or product thereof comprising a barcode sequence corresponding to a gene of the second set of genes in a cell of the first cell population indicates non-specific probe hybridization, ligation, amplification, and/or detection. In some embodiments, detection of the probes or probe sets of the second panel of probes or probe sets specifically in the second cell population indicates specificity of the assay. In some embodiments, detection of rolling circle amplification products of the probes or probe sets of the second panel of probes or probe sets specifically in the second cell population indicates specificity of the assay.

In some embodiments, a second panel of probes is provided, wherein each probe of the second panel comprises a recognition sequence complementary to a target analyte (e.g., sequence of an RNA transcript or product thereof) of a second set of genes expressed in the second cell population. In some embodiments, the second set of genes is specifically expressed in the second population (e.g., at least 10 fold, at least 50-fold, or at least 100-fold higher expression in the second cell population than in the first cell population). In some embodiments, second set of genes are not detectably expressed in the first cell population. In some embodiments, the second set of genes are not detectably expressed in the first cell population by single cell RNA sequencing. In some embodiments, the second set of genes are not detectably expressed in the first cell population by bulk RNA sequencing of the first cell population. In some embodiments, the second set of genes are not detectably expressed in the first cell population by *in situ* detection of a probe or probe set or a product thereof (e.g., an RCA product thereof) specifically associated with an RNA transcript of the gene in the first cell population. In some embodiments, products of the hybridized probes or probe sets are amplification products of the hybridized probes or probe sets. In some embodiments, products of the hybridized probes or probe sets are rolling circle amplification products (RCPs). Thus, in some aspects, detection of a probe or product thereof comprising a barcode sequence corresponding to a gene of the second set of genes in a cell of the first cell population indicates non-specific probe hybridization, ligation, amplification, and/or detection. In some embodiments, detection of the probes or of the second panel of probes specifically in the second cell population indicates specificity of the assay. In some embodiments, detection of rolling circle amplification products of the probes of the second panel of probes specifically in the second cell population indicates specificity of the assay.

In some embodiments, a second panel of probe sets is provided, wherein each probe set of the second panel comprises a recognition sequence complementary to a target analyte (e.g., sequence of an RNA transcript or product thereof) of a second set of genes expressed in the second cell population. In some embodiments, the second set of genes is specifically expressed in the second population (e.g., at least 10 fold, at least 50-fold, or at least 100-fold higher expression in the second cell population than in the first cell population). In some embodiments, second set of genes are not detectably expressed in the first cell population. In some embodiments, the second set of genes are not detectably expressed in the first cell population by single cell RNA sequencing. In some embodiments, the second set of genes are not detectably expressed in the first cell population by bulk RNA sequencing of the first cell population. In some embodiments, the second set of genes are not detectably expressed in the first cell population by *in situ* detection of a probe set or a product thereof (e.g., an RCA product thereof) specifically associated with an RNA transcript of the gene in the first cell population. In some embodiments, products of the hybridized probe sets are amplification products of the hybridized probe sets. In some embodiments, products of the hybridized probe sets are rolling circle amplification products (RCPs). Thus, in some aspects, detection of a probe set or product thereof comprising a barcode sequence corresponding to a gene of the second set of genes in a cell of the first cell population indicates non-specific probe hybridization, ligation, amplification, and/or detection. In some embodiments, detection of the probe sets of the second panel of probe sets specifically in the second cell population indicates specificity of the assay. In some embodiments, detection of rolling circle amplification products of the probe sets of the second panel of probe sets specifically in the second cell population indicates specificity of the assay.

In some embodiments, a second panel of probes is provided, wherein each probe of the second panel comprises a recognition sequence complementary to a target analyte (e.g., sequence of an RNA transcript or product thereof) of a second set of genes expressed in Raji cells. In some embodiments, the second set of genes is specifically expressed in Raji cells (e.g., at least 10 fold, at least 50-fold, or at least 100-fold higher expression in Raji cells than in Jurkat cells). In some embodiments, second set of genes are not detectably expressed in the population of Jurkat cells. In some embodiments, the second set of genes are not detectably expressed in Jurkat cells by single cell RNA sequencing. In some embodiments, the second set of genes are not detectably expressed in Jurkat cells by bulk RNA sequencing of the population of Jurkat cells. In some embodiments, the second set of genes are not detectably expressed in the population of Jurkat cells by *in situ* detection of a probe or a product thereof (e.g., an RCA product thereof) specifically associated with an RNA transcript of the gene in the population of Jurkat cells. In some embodiments, products of the hybridized probes are amplification products of the hybridized probes. In some embodiments, products of the hybridized probes are rolling circle amplification products (RCPs). Thus, in some aspects, detection of a probe or product thereof comprising a barcode sequence corresponding to a gene of the second set of genes in a cell of the population of Jurkat cells indicates non-specific probe hybridization, ligation, amplification, and/or detection. In some embodiments, detection of the probes of the second panel of probes specifically in the population of Raji cells indicates specificity of the assay. In some embodiments, detection of rolling circle amplification products of the probes of the second panel of probes specifically in the population of Raji cells indicates specificity of the assay.

In some embodiments, a second panel of probe sets is provided, wherein each probe set of the second panel comprises a recognition sequence complementary to a target analyte (e.g., sequence of an RNA transcript or product thereof) of a second set of genes expressed in Raji cells. In some embodiments, the second set of genes is specifically expressed in Raji cells (e.g., at least 10 fold, at least 50-fold, or at least 100-fold higher expression in Raji cells than in Jurkat cells). In some embodiments, second set of genes are not detectably expressed in the population of Jurkat cells. In some embodiments, the second set of genes are not detectably expressed in Jurkat cells by single cell RNA sequencing. In some embodiments, the second set of genes are not detectably expressed in Jurkat cells by bulk RNA sequencing of the population of Jurkat cells. In some embodiments, the second set of genes are not detectably expressed in the population of Jurkat cells by *in situ* detection of a probe set or a product thereof (e.g., an RCA product thereof) specifically associated with an RNA transcript of the gene in the population of Jurkat cells. In some embodiments, products of the hybridized probe sets are amplification products of the hybridized probe sets. In some embodiments, products of the hybridized probe sets are rolling circle amplification products (RCPs). Thus, in some aspects, detection of a probe set or product thereof comprising a barcode sequence corresponding to a gene of the second set of genes in a cell of the population of Jurkat cells indicates non-specific probe hybridization, ligation, amplification, and/or detection. In some embodiments, detection of the probe sets of the second panel of probe sets specifically in the population of Raji cells indicates specificity of the assay. In some embodiments, detection of rolling circle amplification products of the probe sets of the second panel of probe sets specifically in the population of Raji cells indicates specificity of the assay.

In some embodiments, provided herein is a third panel of probes or probe sets, wherein each probe or probe set of the third panel comprises a recognition sequence complementary to a target analyte (e.g., sequence of an RNA transcript or product thereof) of a third set of genes. In some embodiments, the third set of genes is expressed in both the first cell population and the second cell population. In some embodiments, the third set of genes comprises one or more housekeeping genes. In some embodiments, the third set of genes is expressed in both the first cell population and the second cell population at a level of at least 1, 2, 5, 10, 20, 40, or 100 transcripts per cell. In some embodiments, expected expression levels of the third set of genes are based on available RNA sequencing data and/or *in situ* detection of RNA transcripts of the third set of genes in a control assay.

In some embodiments, provided herein is a third panel of probes, wherein each probe of the third panel comprises a recognition sequence complementary to a target analyte (e.g., sequence of an RNA transcript or product thereof) of a third set of genes. In some embodiments, the third set of genes is expressed in both the first cell population and the second cell population. In some embodiments, the third set of genes comprises one or more housekeeping genes. In some embodiments, the third set of genes is expressed in both the first cell population and the second cell population at a level of at least 1, 2, 5, 10, 20, 40, or 100 transcripts per cell. In some embodiments, expected expression levels of the third set of genes are based on available RNA sequencing data and/or *in situ* detection of RNA transcripts of the third set of genes in a control assay.

In some embodiments, provided herein is a third panel of probe sets, wherein each probe set of the third panel comprises a recognition sequence complementary to a target analyte (e.g., sequence of an RNA transcript or product thereof) of a third set of genes. In some embodiments, the third set of genes is expressed in both the first cell population and the second cell population. In some embodiments, the third set of genes comprises one or more housekeeping genes. In some embodiments, the third set of genes is expressed in both the first cell population and the second cell population at a level of at least 1, 2, 5, 10, 20, 40, or 100 transcripts per cell. In some embodiments, expected expression levels of the third set of genes are based on available RNA sequencing data and/or *in situ* detection of RNA transcripts of the third set of genes in a control assay.

In some embodiments, provided herein is a third panel of probes, wherein each probe of the third panel comprises a recognition sequence complementary to a target analyte (e.g., sequence of an RNA transcript or product thereof) of a third set of genes. In some embodiments, the third set of genes is expressed in both Jurkat cells and Raji cells. In some embodiments, the third set of genes comprises one or more housekeeping genes. In some embodiments, the third set of genes is expressed in both the population of Jurkat cells and the population of Raji cells at a level of at least 1, 2, 5, 10, 20, 40, or 100 transcripts per cell. In some embodiments, expected expression levels of the third set of genes are based on available RNA sequencing data and/or *in situ* detection of RNA transcripts of the third set of genes in a control assay.

In some embodiments, provided herein is a third panel of probe sets, wherein each probe set of the third panel comprises a recognition sequence complementary to a target analyte (e.g., sequence of an RNA transcript or product thereof) of a third set of genes. In some embodiments, the third set of genes is expressed in both Jurkat cells and Raji cells. In some embodiments, the third set of genes comprises one or more housekeeping genes. In some embodiments, the third set of genes is expressed in both the population of Jurkat cells and the population of Raji cells at a level of at least 1, 2, 5, 10, 20, 40, or 100 transcripts per cell. In some embodiments, expected expression levels of the third set of genes are based on available RNA sequencing data and/or *in situ* detection of RNA transcripts of the third set of genes in a control assay.

In some embodiments, provided herein is a probe mixture comprising the first panel of probes or probe sets and the second panel of probes or probe sets. In some embodiments, provided herein is a probe mixture comprising the first, second, and third panels of probes or probe sets. In some embodiments, the probe mixture comprises probes or probe sets targeting transcripts of genes having at least two different expression levels in the first cell population. In some embodiments, the probe mixture comprises probes or probe sets targeting transcripts of genes having at least two different expression levels in the second cell population. In some embodiments, the at least two different expression levels are selected from the group consisting of low expression, medium expression, and high expression. In some embodiments, genes that have low expression are genes that are detected at a mean count of 1-3, 1-4, or 1-5, transcripts per cell (e.g., by RNA sequencing). In some embodiments, genes that have medium expression are genes that are detected at a mean count of 5-20, 6-20, 6-19, 10-20, or 10-19 transcripts per cell (e.g., by RNA sequencing). In some embodiments, genes that have medium expression are genes that are detected at a mean count of more than 20, more than 21, more than 30, more than 40, or more than 50 transcripts per cell (e.g., by RNA sequencing).

In some embodiments, provided herein is a probe mixture comprising the first panel of probes and the second panel of probes. In some embodiments, provided herein is a probe mixture comprising the first, second, and third panels of probes. In some embodiments, the probe mixture comprises probes or probe sets targeting transcripts of genes having at least two different expression levels in the first cell population. In some embodiments, the probe mixture comprises probes targeting transcripts of genes having at least two different expression levels in the second cell population. In some embodiments, the at least two different expression levels are selected from the group consisting of low expression, medium expression, and high expression. In some embodiments, genes that have low expression are genes that are detected at a mean count of 1-3, 1-4, or 1-5, transcripts per cell (e.g., by RNA sequencing). In some embodiments, genes that have medium expression are genes that are detected at a mean count of 5-20, 6-20, 6-19, 10-20, or 10-19 transcripts per cell (e.g., by RNA sequencing). In some embodiments, genes that have medium expression are genes that are detected at a mean count of more than 20, more than 21, more than 30, more than 40, or more than 50 transcripts per cell (e.g., by RNA sequencing).

In some embodiments, provided herein is a probe mixture comprising the first panel of probe sets and the second panel of probe sets. In some embodiments, provided herein is a probe mixture comprising the first, second, and third panels of probe sets. In some embodiments, the probe mixture comprises probe sets targeting transcripts of genes having at least two different expression levels in the first cell population. In some embodiments, the probe mixture comprises probe sets targeting transcripts of genes having at least two different expression levels in the second cell population. In some embodiments, the at least two different expression levels are selected from the group consisting of low expression, medium expression, and high expression. In some embodiments, genes that have low expression are genes that are detected at a mean count of 1-3, 1-4, or 1-5, transcripts per cell (e.g., by RNA sequencing). In some embodiments, genes that have medium expression are genes that are detected at a mean count of 5-20, 6-20, 6-19, 10-20, or 10-19 transcripts per cell (e.g., by RNA sequencing). In some embodiments, genes that have medium expression are genes that are detected at a mean count of more than 20, more than 21, more than 30, more than 40, or more than 50 transcripts per cell (e.g., by RNA sequencing).

In some embodiments, provided herein is a probe mixture comprising the first panel of probes and the second panel of probes. In some embodiments, provided herein is a probe mixture comprising the first, second, and third panels of probes. In some embodiments, the probe mixture comprises probes targeting transcripts of genes having at least two different expression levels in Jurkat cells. In some embodiments, the probe mixture comprises probes targeting transcripts of genes having at least two different expression levels in Raji cells. In some embodiments, the at least two different expression levels are selected from the group consisting of low expression, medium expression, and high expression. In some embodiments, genes that have low expression are genes that are detected at a mean count of 1-3, 1-4, or 1-5, transcripts per cell (e.g., by RNA sequencing). In some embodiments, genes that have medium expression are genes that are detected at a mean count of 5-20, 6-20, 6-19, 10-20, or 10-19 transcripts per cell (e.g., by RNA sequencing). In some embodiments, genes that have medium expression are genes that are detected at a mean count of more than 20, more than 21, more than 30, more than 40, or more than 50 transcripts per cell (e.g., by RNA sequencing).

In some embodiments, provided herein is a probe mixture comprising the first panel of probe sets and the second panel of probe sets. In some embodiments, provided herein is a probe mixture comprising the first, second, and third panels of probe sets. In some embodiments, the probe mixture comprises probe sets targeting transcripts of genes having at least two different expression levels in Jurkat cells. In some embodiments, the probe mixture comprises probe sets targeting transcripts of genes having at least two different expression levels in Raji cells. In some embodiments, the at least two different expression levels are selected from the group consisting of low expression, medium expression, and high expression. In some embodiments, genes that have low expression are genes that are detected at a mean count of 1-3, 1-4, or 1-5, transcripts per cell (e.g., by RNA sequencing). In some embodiments, genes that have medium expression are genes that are detected at a mean count of 5-20, 6-20, 6-19, 10-20, or 10-19 transcripts per cell (e.g., by RNA sequencing). In some embodiments, genes that have medium expression are genes that are detected at a mean count of more than 20, more than 21, more than 30, more than 40, or more than 50 transcripts per cell (e.g., by RNA sequencing).

In some cases, a probe or probe set of the first, second, and/or third panels of is a barcoded probe or probe set. Exemplary barcoded probes or probe sets may comprise a circularizable probe or probe set (e.g., based on a padlock probe, a gapped padlock probe, a SNAIL (Splint Nucleotide Assisted Intramolecular Ligation) probe set), a PLISH (Proximity Ligation *in situ* Hybridization) probe set, a RollFISH probe set, or a PLAYR (Proximity Ligation Assay for RNA) probe set). In some embodiments, an exemplary barcoded probe or probe set is not circular or circularizable. Examples of barcoded probes or probe sets include, but are not limited to, L-shaped probes (e.g., a probe comprising a target-hybridizing sequence and a 5' or 3' overhang upon hybridization to its target sequence), or U-shaped probes (e.g., a probe comprising a target-hybridizing sequence and a 5' overhang and a 3' overhang upon hybridization to its target sequence). The specific probe or probe set design can vary.

In some cases, a probe of the first, second, and/or third panels of is a barcoded probe. Exemplary barcoded probes may comprise a circularizable probe (e.g., based on a padlock probe, a gapped padlock probe, a SNAIL (Splint Nucleotide Assisted Intramolecular Ligation) probe set), a PLISH (Proximity Ligation *in situ* Hybridization) probe set, a RollFISH probe set, or a PLAYR (Proximity Ligation Assay for RNA) probe set). In some embodiments, an exemplary barcoded probe is not circular or circularizable. Examples of barcoded probes include, but are not limited to, L-shaped probes (e.g., a probe comprising a target-hybridizing sequence and a 5' or 3' overhang upon hybridization to its target sequence), or U-shaped probes (e.g., a probe comprising a target-hybridizing sequence and a 5' overhang and a 3' overhang upon hybridization to its target sequence). The specific probe design can vary.

In some cases, a probe set of the first, second, and/or third panels of is a barcoded probe set. Exemplary barcoded probe sets may comprise a circularizable probe set (e.g., based on a padlock probe, a gapped padlock probe, a SNAIL (Splint Nucleotide Assisted Intramolecular Ligation) probe set), a PLISH (Proximity Ligation *in situ* Hybridization) probe set, a RollFISH probe set, or a PLAYR (Proximity Ligation Assay for RNA) probe set). In some embodiments, an exemplary barcoded probe set is not circular or circularizable. Examples of barcoded probe sets include, but are not limited to, L-shaped probes (e.g., a probe comprising a target-hybridizing sequence and a 5' or 3' overhang upon hybridization to its target sequence), or U-shaped probes (e.g., a probe comprising a target-hybridizing sequence and a 5' overhang and a 3' overhang upon hybridization to its target sequence). The specific probe set design can vary.

In some embodiments, a probe or probe set of the first, second, and/or third panels of probes is a probe comprising a 3' or 5' overhang upon hybridization to the target nucleic acid (e.g., an L-shaped probe). In some embodiments, the overhang comprises one or more barcode sequences corresponding to the target nucleic acid (e.g., the target RNA transcript). In some embodiments, a plurality of probes are designed to hybridize to the target nucleic acid (e.g., at least 20, 30, or 40 probes can hybridize to the target nucleic acid). In some embodiments, the probe or probe set is a probe comprising a 3' overhang and a 5' overhang upon hybridization to the target nucleic acid (a U-shaped probe). In some embodiments, the 3' overhang and the 5' overhang each independently comprises one or more detectable labels and/or barcode sequences. In some embodiments, the 3' and/or 5' overhang comprises one or more detectable labels and/or barcode sequences. In some embodiments, multiple probes comprising one or more overhang regions are hybridized to a plurality of target sequences within a particular target nucleic acid molecule (e.g., tiling across multiple regions in the target nucleic acid molecule). Such tiling of probes can provide signal amplification by increasing the number of detectable labels and/or barcode sequences per target nucleic acid. For example, between 10 and 20, between 10 and 30, or between 20 and 40 probes can be hybridized per target nucleic acid molecule. In some embodiments according to the methods described herein, each target nucleic acid molecule can be hybridized by no more than 1, 2, 3, 4, 5, 6, 7, 8, 9, or 10 primary probes. In some embodiments, a single probe is hybridized per target nucleic acid molecule (e.g., per target RNA). Any suitable method of signal amplification can be used to detect a barcode sequence in the overhang region of the primary probe (e.g., RCA of a probe that directly or indirectly binds to the primary probe or probe set and/or the amplification product thereof; hybridization chain reaction (HCR) directly or indirectly on the primary probe or probe set and/or the amplification product thereof; linear oligonucleotide hybridization chain reaction (LO-HCR) directly or indirectly on the primary probe or probe set and/or the amplification product thereof; primer exchange reaction (PER) directly or indirectly on the primary probe or probe set and/or the amplification product thereof; assembly of branched structures directly or indirectly on the primary probe or probe set and/or the amplification product thereof; hybridization of a plurality of detectably labeled probes directly or indirectly on the primary probe or probe set and/or the amplification product thereof, or any combination thereof).

In some embodiments, a probe of the first, second, and/or third panels of probes is a probe comprising a 3' or 5' overhang upon hybridization to the target nucleic acid (e.g., an L-shaped probe). In some embodiments, the overhang comprises one or more barcode sequences corresponding to the target nucleic acid (e.g., the target RNA transcript). In some embodiments, a plurality of probes are designed to hybridize to the target nucleic acid (e.g., at least 20, 30, or 40 probes can hybridize to the target nucleic acid). In some embodiments, the probe is a probe comprising a 3' overhang and a 5' overhang upon hybridization to the target nucleic acid (a U-shaped probe). In some embodiments, the 3' overhang and the 5' overhang each independently comprises one or more detectable labels and/or barcode sequences. In some embodiments, the 3' and/or 5' overhang comprises one or more detectable labels and/or barcode sequences. In some embodiments, multiple probes comprising one or more overhang regions are hybridized to a plurality of target sequences within a particular target nucleic acid molecule (e.g., tiling across multiple regions in the target nucleic acid molecule). Such tiling of probes can provide signal amplification by increasing the number of detectable labels and/or barcode sequences per target nucleic acid. For example, between 10 and 20, between 10 and 30, or between 20 and 40 probes can be hybridized per target nucleic acid molecule. In some embodiments according to the methods described herein, each target nucleic acid molecule can be hybridized by no more than 1, 2, 3, 4, 5, 6, 7, 8, 9, or 10 primary probes. In some embodiments, a single probe is hybridized per target nucleic acid molecule (e.g., per target RNA). Any suitable method of signal amplification can be used to detect a barcode sequence in the overhang region of the primary probe (e.g., RCA of a probe that directly or indirectly binds to the primary probe or probe set and/or the amplification product thereof; hybridization chain reaction (HCR) directly or indirectly on the primary probe or probe set and/or the amplification product thereof; linear oligonucleotide hybridization chain reaction (LO-HCR) directly or indirectly on the primary probe or probe set and/or the amplification product thereof; primer exchange reaction (PER) directly or indirectly on the primary probe and/or the amplification product thereof; assembly of branched structures directly or indirectly on the primary probe and/or the amplification product thereof; hybridization of a plurality of detectably labeled probes directly or indirectly on the primary probe and/or the amplification product thereof, or any combination thereof).

In some embodiments, a probe set of the first, second, and/or third panels of probes is a probe comprising a 3' or 5' overhang upon hybridization to the target nucleic acid (e.g., an L-shaped probe). In some embodiments, the overhang comprises one or more barcode sequences corresponding to the target nucleic acid (e.g., the target RNA transcript). In some embodiments, a plurality of probes are designed to hybridize to the target nucleic acid (e.g., at least 20, 30, or 40 probes can hybridize to the target nucleic acid). In some embodiments, the probe set is a probe comprising a 3' overhang and a 5' overhang upon hybridization to the target nucleic acid (a U-shaped probe). In some embodiments, the 3' overhang and the 5' overhang each independently comprises one or more detectable labels and/or barcode sequences. In some embodiments, the 3' and/or 5' overhang comprises one or more detectable labels and/or barcode sequences. In some embodiments, multiple probes comprising one or more overhang regions are hybridized to a plurality of target sequences within a particular target nucleic acid molecule (e.g., tiling across multiple regions in the target nucleic acid molecule). Such tiling of probes can provide signal amplification by increasing the number of detectable labels and/or barcode sequences per target nucleic acid. For example, between 10 and 20, between 10 and 30, or between 20 and 40 probes can be hybridized per target nucleic acid molecule. In some embodiments according to the methods described herein, each target nucleic acid molecule can be hybridized by no more than 1, 2, 3, 4, 5, 6, 7, 8, 9, or 10 primary probes. In some embodiments, a single probe is hybridized per target nucleic acid molecule (e.g., per target RNA). Any suitable method of signal amplification can be used to detect a barcode sequence in the overhang region of the primary probe (e.g., RCA of a probe that directly or indirectly binds to the primary probe set and/or the amplification product thereof; hybridization chain reaction (HCR) directly or indirectly on the primary probe set and/or the amplification product thereof; linear oligonucleotide hybridization chain reaction (LO-HCR) directly or indirectly on the primary probe set and/or the amplification product thereof; primer exchange reaction (PER) directly or indirectly on the primary probe set and/or the amplification product thereof; assembly of branched structures directly or indirectly on the probe set and/or the amplification product thereof; hybridization of a plurality of detectably labeled probes directly or indirectly on the primary probe set and/or the amplification product thereof, or any combination thereof).

In some embodiments, a probe or probe set of the first, second, and/or third panel is a circular probe. In some embodiments, the probe or probe set is a circularizable probe or probe set. In some embodiments, the probe or probe set is designed for RNA-templated ligation. See, e.g., U.S. Pat. Pub. 2020/0224244. In any of the embodiments herein, the circularizable probe or probe set can comprise one, two, three, four, or more ribonucleotides. In some embodiments, the circularizable probe or probe set is designed to be circularized using the target nucleic acid (e.g., a DNA or RNA target nucleic acid) as a template. In some embodiments, the circularizable probe or probe set is designed to be circularized using another probe as a template (e.g., as in the case of SNAIL or RollFISH probes). In some embodiments, the probe used as a template for circularization is also used as a primer for amplification of the circularized probe or probe set. In some embodiments, a separate primer is provided for amplification of the circularized probe or probe set. Any other modifications or variations of circularizable probe or probe sets can be used.

In some embodiments, a probe of the first, second, and/or third panel is a circular probe. In some embodiments, the probe is a circularizable probe. In some embodiments, the probe is designed for RNA-templated ligation. See, e.g., U.S. Pat. Pub. 2020/0224244. In any of the embodiments herein, the circularizable probe can comprise one, two, three, four, or more ribonucleotides. In some embodiments, the circularizable probe is designed to be circularized using the target nucleic acid (e.g., a DNA or RNA target nucleic acid) as a template. In some embodiments, the circularizable probe is designed to be circularized using another probe as a template (e.g., as in the case of SNAIL or RollFISH probes). In some embodiments, the probe used as a template for circularization is also used as a primer for amplification of the circularized probe. In some embodiments, a separate primer is provided for amplification of the circularized probe. Any other modifications or variations of circularizable probe can be used.

In some embodiments, a probe set of the first, second, and/or third panel is a circular probe. In some embodiments, the probe set is a circularizable probe set. In some embodiments, the probe set is designed for RNA-templated ligation. See, e.g., U.S. Pat. Pub. 2020/0224244. In any of the embodiments herein, the circularizable probe set can comprise one, two, three, four, or more ribonucleotides. In some embodiments, the circularizable probe set is designed to be circularized using the target nucleic acid (e.g., a DNA or RNA target nucleic acid) as a template. In some embodiments, the circularizable probe set is designed to be circularized using another probe as a template (e.g., as in the case of SNAIL or RollFISH probes). In some embodiments, the probe used as a template for circularization is also used as a primer for amplification of the circularized probe set. In some embodiments, a separate primer is provided for amplification of the circularized probe set. Any other modifications or variations of circularizable probe sets can be used.

In some embodiments, the probe or probe set comprises a primer binding site. In some embodiments, the probes or probe sets of the first panel and the second panel comprise common primer binding sites. In some embodiments, the probes or probe sets of the first panel, the second panel, and the third panel comprise common primer binding sites. In some embodiments, a primer is provided for hybridization to the primer binding site, wherein the primer can be extended to form an amplification product of the probe or probe set (e.g., a rolling circle amplification product of a circular or circularized probe). A primer is generally a single-stranded nucleic acid sequence having a 3' end that can be used as a substrate for a nucleic acid polymerase in a nucleic acid extension reaction. RNA primers are formed of RNA nucleotides, and are used in RNA synthesis, while DNA primers are formed of DNA nucleotides and used in DNA synthesis. Primers can also include both RNA nucleotides and DNA nucleotides (e.g., in a random or designed pattern). Primers can also include other natural or synthetic nucleotides described herein that can have additional functionality. In some examples, DNA primers can be used to prime RNA synthesis and vice versa (e.g., RNA primers can be used to prime DNA synthesis). Primers can vary in length. For example, primers can be about 6 bases to about 120 bases. For example, primers can include up to about 25 bases. A primer extension reaction generally refers to any method where two nucleic acid sequences become linked (e.g., hybridized) by an overlap of their respective terminal complementary nucleic acid sequences (e.g., for example, 3' termini). Such linking can be followed by nucleic acid extension (e.g., an enzymatic extension) of one, or both termini using the other nucleic acid sequence as a template for extension. Enzymatic extension can be performed by an enzyme including, but not limited to, a polymerase and/or a reverse transcriptase.

In some embodiments, the probe comprises a primer binding site. In some embodiments, the probes of the first panel and the second panel comprise common primer binding sites. In some embodiments, the probes of the first panel, the second panel, and the third panel comprise common primer binding sites. In some embodiments, a primer is provided for hybridization to the primer binding site, wherein the primer can be extended to form an amplification product of the probe (e.g., a rolling circle amplification product of a circular or circularized probe). A primer is generally a single-stranded nucleic acid sequence having a 3' end that can be used as a substrate for a nucleic acid polymerase in a nucleic acid extension reaction. RNA primers are formed of RNA nucleotides, and are used in RNA synthesis, while DNA primers are formed of DNA nucleotides and used in DNA synthesis. Primers can also include both RNA nucleotides and DNA nucleotides (e.g., in a random or designed pattern). Primers can also include other natural or synthetic nucleotides described herein that can have additional functionality. In some examples, DNA primers can be used to prime RNA synthesis and vice versa (e.g., RNA primers can be used to prime DNA synthesis). Primers can vary in length. For example, primers can be about 6 bases to about 120 bases. For example, primers can include up to about 25 bases. A primer extension reaction generally refers to any method where two nucleic acid sequences become linked (e.g., hybridized) by an overlap of their respective terminal complementary nucleic acid sequences (e.g., for example, 3' termini). Such linking can be followed by nucleic acid extension (e.g., an enzymatic extension) of one, or both termini using the other nucleic acid sequence as a template for extension. Enzymatic extension can be performed by an enzyme including, but not limited to, a polymerase and/or a reverse transcriptase.

In some embodiments, the probe set comprises a primer binding site. In some embodiments, the probe sets of the first panel and the second panel comprise common primer binding sites. In some embodiments, the probe sets of the first panel, the second panel, and the third panel comprise common primer binding sites. In some embodiments, a primer is provided for hybridization to the primer binding site, wherein the primer can be extended to form an amplification product of the probe set (e.g., a rolling circle amplification product of a circular or circularized probe). A primer is generally a single-stranded nucleic acid sequence having a 3' end that can be used as a substrate for a nucleic acid polymerase in a nucleic acid extension reaction. RNA primers are formed of RNA nucleotides, and are used in RNA synthesis, while DNA primers are formed of DNA nucleotides and used in DNA synthesis. Primers can also include both RNA nucleotides and DNA nucleotides (e.g., in a random or designed pattern). Primers can also include other natural or synthetic nucleotides described herein that can have additional functionality. In some examples, DNA primers can be used to prime RNA synthesis and vice versa (e.g., RNA primers can be used to prime DNA synthesis). Primers can vary in length. For example, primers can be about 6 bases to about 120 bases. For example, primers can include up to about 25 bases. A primer extension reaction generally refers to any method where two nucleic acid sequences become linked (e.g., hybridized) by an overlap of their respective terminal complementary nucleic acid sequences (e.g., for example, 3' termini). Such linking can be followed by nucleic acid extension (e.g., an enzymatic extension) of one, or both termini using the other nucleic acid sequence as a template for extension. Enzymatic extension can be performed by an enzyme including, but not limited to, a polymerase and/or a reverse transcriptase.

In some embodiments, a probe or probe set of the first panel, second panel, and/or third panel comprises a first probe and a second probe that can be ligated to generate a ligated first-second probe (e.g., a linear ligated probe). In some embodiments, a linear ligated probe can be circularized using an additional bridge probe that is ligated to either end of the ligated linear probe (e.g., in a templated or non-templated ligation). In some embodiments, the first and/or second probe comprises an overhang region, which may optionally comprise one or more barcode sequences for detection of the first and/or second probe or the ligated first-second probe. In some embodiments, the probe or probe set is a circularizable probe or probe set (e.g., a padlock probe). In some embodiments, the circularizable probe or probe set comprises one or more barcode sequences for detection of circularizable probe or probe set, the circularized probe or probe set, or an amplification product thereof.

In some embodiments, a probe of the first panel, second panel, and/or third panel comprises a first probe and a second probe that can be ligated to generate a ligated first-second probe (e.g., a linear ligated probe). In some embodiments, a linear ligated probe can be circularized using an additional bridge probe that is ligated to either end of the ligated linear probe (e.g., in a templated or non-templated ligation). In some embodiments, the first and/or second probe comprises an overhang region, which may optionally comprise one or more barcode sequences for detection of the first and/or second probe or the ligated first-second probe. In some embodiments, the probe is a circularizable probe (e.g., a padlock probe). In some embodiments, the circularizable probe comprises one or more barcode sequences for detection of circularizable probe, the circularized probe, or an amplification product thereof. In some embodiments, products of the hybridized probes are amplification products of the hybridized probes. In some embodiments, products of the hybridized probes are rolling circle amplification products (RCPs).

In some embodiments, a probe set of the first panel, second panel, and/or third panel comprises a first probe and a second probe that can be ligated to generate a ligated first-second probe (e.g., a linear ligated probe). In some embodiments, a linear ligated probe can be circularized using an additional bridge probe that is ligated to either end of the ligated linear probe (e.g., in a templated or non-templated ligation). In some embodiments, the first and/or second probe comprises an overhang region, which may optionally comprise one or more barcode sequences for detection of the first and/or second probe or the ligated first-second probe. In some embodiments, the probe set is a circularizable probe set (e.g., a padlock probe). In some embodiments, the circularizable probe set comprises one or more barcode sequences for detection of the circularizable probe set, the circularized probe set, or an amplification product thereof. In some embodiments, products of the hybridized probe sets are amplification products of the hybridized probe sets. In some embodiments, products of the hybridized probe sets are rolling circle amplification products (RCPs).

In some embodiments, a probe or probe set of the first, second, and/or third panel disclosed herein can comprise one, two, three, four, or more ribonucleotides in a DNA backbone. In any of the embodiments herein, the one or more ribonucleotides can be at and/or near a ligatable 3' end of a circularizable probe or probe set. The probe or probe set may comprise an optional 3' RNA base. In some embodiments, a probe or probe set disclosed herein can comprise a 5' flap which may be recognized by a structure-specific cleavage enzyme (e.g. an enzyme capable of recognizing the junction between single-stranded 5' overhang and a DNA duplex and cleaving the single-stranded overhang). In some embodiments, the flap is positioned between a 3' end and 5' end of a split hybridization region upon hybridization of the primary probe or probe set to the target sequence, and cleavage of the flap allows ligation of the 3' end to the 5' end of the split hybridization region. Methods of ligating a first and second hybridization region with or without flap cleavage are described in U.S. Pat. Pub. 20200224244.

In some embodiments, a probe of the first, second, and/or third panel disclosed herein can comprise one, two, three, four, or more ribonucleotides in a DNA backbone. In any of the embodiments herein, the one or more ribonucleotides can be at and/or near a ligatable 3' end of a circularizable probe. The probe may comprise an optional 3' RNA base. In some embodiments, a probe disclosed herein can comprise a 5' flap which may be recognized by a structure-specific cleavage enzyme (e.g. an enzyme capable of recognizing the junction between single-stranded 5' overhang and a DNA duplex and cleaving the single-stranded overhang). In some embodiments, the flap is positioned between a 3' end and 5' end of a split hybridization region upon hybridization of the primary probe to the target sequence, and cleavage of the flap allows ligation of the 3' end to the 5' end of the split hybridization region.

In some embodiments, a probe set of the first, second, and/or third panel disclosed herein can comprise one, two, three, four, or more ribonucleotides in a DNA backbone. In any of the embodiments herein, the one or more ribonucleotides can be at and/or near a ligatable 3' end of a circularizable probe set. The probe set may comprise an optional 3' RNA base. In some embodiments, a probe set disclosed herein can comprise a 5' flap which may be recognized by a structure-specific cleavage enzyme (e.g. an enzyme capable of recognizing the junction between single-stranded 5' overhang and a DNA duplex and cleaving the single-stranded overhang). In some embodiments, the flap is positioned between a 3' end and 5' end of a split hybridization region upon hybridization of the primary probe set to the target sequence, and cleavage of the flap allows ligation of the 3' end to the 5' end of the split hybridization region.

In some embodiments, a probe or probe set of the first, second, and/or third panel comprises a split hybridization region configured to hybridize to a splint. In some embodiments, the split hybridization region comprises one or more barcode sequences. For example, a probe set can comprise two probes that hybridize to adjacent portions of the target sequence, wherein each probe comprises an overhang region that does not hybridize to the target nucleic acid. The overhang regions can together form a split-hybridization region. The split hybridization region can comprise one or more barcode sequences specific to the target sequence, so that the target sequence can be identified by hybridizing a detectable splint to the split hybridization region. The splint may be directly or indirectly labeled. In some embodiments, the splint is a bridge probe. In some embodiments, the splint is ligated to one or more other probes (e.g., to form a circularized probe), and optionally amplified by rolling circle amplification. In some embodiments, the splint comprises a barcode sequence (e.g., in an overhang region) that can be detected using any of the signal amplification and detection methods described herein, such as assembly of branched DNA structures, HCR, LO-HCR, RCA, PER, etc. Examples of probes or probe sets comprising split hybridization regions (e.g., Z-probes, proximity ligation *in situ* hybridization (PLISH) probes, or split-FISH probes) have been described, for example, in U.S. Pat. Pub. 20160115555, U.S. Pat. Pub. US20200224243, U.S. Pat. Pub. 20160108458, and WO2021/167526.

In some embodiments, a probe of the first, second, and/or third panel comprises a split hybridization region configured to hybridize to a splint. In some embodiments, the split hybridization region comprises one or more barcode sequences. For example, a probe set can comprise two probes that hybridize to adjacent portions of the target sequence, wherein each probe comprises an overhang region that does not hybridize to the target nucleic acid. The overhang regions can together form a split-hybridization region. The split hybridization region can comprise one or more barcode sequences specific to the target sequence, so that the target sequence can be identified by hybridizing a detectable splint to the split hybridization region. The splint may be directly or indirectly labeled. In some embodiments, the splint is a bridge probe. In some embodiments, the splint is ligated to one or more other probes (e.g., to form a circularized probe), and optionally amplified by rolling circle amplification. In some embodiments, the splint comprises a barcode sequence (e.g., in an overhang region) that can be detected using any of the signal amplification and detection methods described herein, such as assembly of branched DNA structures, HCR, LO-HCR, RCA, PER, etc. Examples of probes comprising split hybridization regions (e.g., Z-probes, proximity ligation *in situ* hybridization (PLISH) probes, or split-FISH probes) have been described, for example, in U.S. Pat. Pub. 20160115555, U.S. Pat. Pub. US20200224243, U.S. Pat. Pub. 20160108458, and WO2021/167526.

In some embodiments, a probe set of the first, second, and/or third panel comprises a split hybridization region configured to hybridize to a splint. In some embodiments, the split hybridization region comprises one or more barcode sequences. For example, a probe set can comprise two probes that hybridize to adjacent portions of the target sequence, wherein each probe comprises an overhang region that does not hybridize to the target nucleic acid. The overhang regions can together form a split-hybridization region. The split hybridization region can comprise one or more barcode sequences specific to the target sequence, so that the target sequence can be identified by hybridizing a detectable splint to the split hybridization region. The splint may be directly or indirectly labeled. In some embodiments, the splint is a bridge probe. In some embodiments, the splint is ligated to one or more other probes (e.g., to form a circularized probe), and optionally amplified by rolling circle amplification. In some embodiments, the splint comprises a barcode sequence (e.g., in an overhang region) that can be detected using any of the signal amplification and detection methods described herein, such as assembly of branched DNA structures, HCR, LO-HCR, RCA, PER, etc. Examples of probe sets comprising split hybridization regions (e.g., Z-probes, proximity ligation *in situ* hybridization (PLISH) probes, or split-FISH probes) have been described, for example, in U.S. Pat. Pub. 20160115555, U.S. Pat. Pub. US20200224243, U.S. Pat. Pub. 20160108458, and WO2021/167526.

In some embodiments described herein, the probe or probe set comprises a target recognition region (optionally a split target recognition region) capable of hybridizing to a target sequence in or associated with an analyte in a biological sample. In some embodiments, the target recognition region is complementary to the target sequence. In some embodiments, the target recognition region is at least about 10, at least about 15, at least about 20, at least about 25, at least about 30, at least about 35, at least about 40, at least about 45, at least about 50, at least about 70, at least about 80, at least about 90, or at least about 100 nucleotides in length. In some embodiments, the target recognition region is between or between about any one of 5 and 200, 10 and 200, 15 and 200, 20 and 200, 5 and 100, 10 and 100, 15 and 100, 20 and 100, 5 and 50, 10 and 50, 15 and 50, 20 and 50, 5 and 20, or 10 and 40 nucleotides in length. In some embodiments, the target recognition region is a split target recognition region.

In some embodiments described herein, the probe comprises a target recognition region (optionally a split target recognition region) capable of hybridizing to a target sequence in or associated with an analyte in a biological sample. In some embodiments, the target recognition region of the probe is complementary to the target sequence. In some embodiments, the target recognition region of the probe is at least about 10, at least about 15, at least about 20, at least about 25, at least about 30, at least about 35, at least about 40, at least about 45, at least about 50, at least about 70, at least about 80, at least about 90, or at least about 100 nucleotides in length. In some embodiments, the target recognition region of the probe is between or between about any one of 5 and 200, 10 and 200, 15 and 200, 20 and 200, 5 and 100, 10 and 100, 15 and 100, 20 and 100, 5 and 50, 10 and 50, 15 and 50, 20 and 50, 5 and 20, or 10 and 40 nucleotides in length. In some embodiments, the target recognition region of the probe is a split target recognition region.

In some embodiments described herein, the probe set comprises a target recognition region (optionally a split target recognition region) capable of hybridizing to a target sequence in or associated with an analyte in a biological sample. In some embodiments, the target recognition region of the probe set is complementary to the target sequence. In some embodiments, the target recognition region of the probe set is at least about 10, at least about 15, at least about 20, at least about 25, at least about 30, at least about 35, at least about 40, at least about 45, at least about 50, at least about 70, at least about 80, at least about 90, or at least about 100 nucleotides in length. In some embodiments, the target recognition region of the probe set is between or between about any one of 5 and 200, 10 and 200, 15 and 200, 20 and 200, 5 and 100, 10 and 100, 15 and 100, 20 and 100, 5 and 50, 10 and 50, 15 and 50, 20 and 50, 5 and 20, or 10 and 40 nucleotides in length. In some embodiments, the target recognition region of the probe set is a split target recognition region.

In some embodiments, the split target recognition region comprises a first target recognition region and a second target recognition region. In some embodiments, the first target recognition region is at a first end of a probe or probe set and the second target recognition region is at a second end of a probe or probe set. In some embodiments, the first target recognition region is at a 3' end of a probe or probe set and the second target recognition region is at a 5' end of a probe or probe set, or vice versa. In some embodiments, the first and second target recognition regions are at a first and second end of a circularizable probe. In some embodiments, the first and second target recognition regions are in a first and second probe. In some embodiments, the 3' or 5' end of the probe or probe set comprises a flap (e.g., an overhang region that does not hybridize to the target nucleic acid) that is cleaved prior to ligation of the probe or probe set. In some embodiments, the first target recognition region and the second target recognition region are independently between or between about any one of 5 and 200, 10 and 200, 15 and 200, 20 and 200, 5 and 100, 10 and 100, 15 and 100, 20 and 100, 5 and 50, 10 and 50, 15 and 50, 20 and 50, 5 and 20, or 10 and 40 nucleotides in length.

In some embodiments, the split target recognition region comprises a first target recognition region and a second target recognition region. In some embodiments, the first target recognition region is at a first end of a probe and the second target recognition region is at a second end of a probe. In some embodiments, the first target recognition region is at a 3' end of a probe and the second target recognition region is at a 5' end of a probe, or vice versa. In some embodiments, the first and second target recognition regions are at a first and second end of a circularizable probe. In some embodiments, the first and second target recognition regions are in a first and second probe. In some embodiments, the 3' or 5' end of the probe or probe set comprises a flap (e.g., an overhang region that does not hybridize to the target nucleic acid) that is cleaved prior to ligation of the probe or probe set. In some embodiments, the first target recognition region and the second target recognition region are independently between or between about any one of 5 and 200, 10 and 200, 15 and 200, 20 and 200, 5 and 100, 10 and 100, 15 and 100, 20 and 100, 5 and 50, 10 and 50, 15 and 50, 20 and 50, 5 and 20, or 10 and 40 nucleotides in length.

In some embodiments, the split target recognition region comprises a first target recognition region and a second target recognition region. In some embodiments, the first target recognition region is at a first end of a probe set and the second target recognition region is at a second end of a probe set. In some embodiments, the first target recognition region is at a 3' end of a probe set and the second target recognition region is at a 5' end of a probe set, or vice versa. In some embodiments, the first and second target recognition regions are at a first and second end of a circularizable probe. In some embodiments, the first and second target recognition regions are in a first and second probe. In some embodiments, the 3' or 5' end of the probe set comprises a flap (e.g., an overhang region that does not hybridize to the target nucleic acid) that is cleaved prior to ligation of the probe set. In some embodiments, the first target recognition region and the second target recognition region are independently between or between about any one of 5 and 200, 10 and 200, 15 and 200, 20 and 200, 5 and 100, 10 and 100, 15 and 100, 20 and 100, 5 and 50, 10 and 50, 15 and 50, 20 and 50, 5 and 20, or 10 and 40 nucleotides in length.

In some embodiments, the probe or probe set comprises an anchor sequence, which can be a common sequence among a plurality of probes or probe sets for a plurality of target sequences. In some embodiments, the anchor sequence is common among the first and second panels of probes or probe sets. In some embodiments, the anchor sequence is common among the first, second, and third panels of probes or probe sets. In some embodiments, the method comprises contacting the sample with an anchor probe configured to hybridize to the anchor sequence or a complement thereof (e.g., in a rolling circle amplification product). In some embodiments, the anchor probe is complementary to the anchor sequence or complement thereof. In some embodiments, the anchor probe is a detectable probe. The anchor probe can be directly labeled or indirectly labeled (e.g., by direct or indirect hybridization of one or more detectably labeled probes to the anchor probe). In some embodiments, the method comprises imaging the sample to detect hybridization of the anchor probe, thereby detecting a plurality of analytes simultaneously.

In some embodiments, the probe comprises an anchor sequence, which can be a common sequence among a plurality of probes for a plurality of target sequences. In some embodiments, the anchor sequence is common among the first and second panels of probes. In some embodiments, the anchor sequence is common among the first, second, and third panels of probes. In some embodiments, the method comprises contacting the sample with an anchor probe configured to hybridize to the anchor sequence or a complement thereof (e.g., in a rolling circle amplification product). In some embodiments, the anchor probe is complementary to the anchor sequence or complement thereof. In some embodiments, the anchor probe is a detectable probe. The anchor probe can be directly labeled or indirectly labeled (e.g., by direct or indirect hybridization of one or more detectably labeled probes to the anchor probe). In some embodiments, the method comprises imaging the sample to detect hybridization of the anchor probe, thereby detecting a plurality of analytes simultaneously.

In some embodiments, the probe set comprises an anchor sequence, which can be a common sequence among a plurality of probe sets for a plurality of target sequences. In some embodiments, the anchor sequence is common among the first and second panels of probe sets. In some embodiments, the anchor sequence is common among the first, second, and third panels of probe sets. In some embodiments, the method comprises contacting the sample with an anchor probe set configured to hybridize to the anchor sequence or a complement thereof (e.g., in a rolling circle amplification product). In some embodiments, the anchor probe set is complementary to the anchor sequence or complement thereof. In some embodiments, the anchor probe set is a detectable probe. The anchor probe set can be directly labeled or indirectly labeled (e.g., by direct or indirect hybridization of one or more detectably labeled probes to the anchor probe). In some embodiments, the method comprises imaging the sample to detect hybridization of the anchor probe set, thereby detecting a plurality of analytes simultaneously.

In some embodiments, the target sequence is a marker sequence for a particular analyte (e.g., an RNA transcript), which identifies the particular analyte (e.g., alone or in combination with one or more other marker sequences). Thus, in some embodiments, a target sequence for a given target analyte is specific to that analyte, or unique, such that multiple target analytes can be distinguished from each other. In some embodiments, the analyte is an RNA molecule (e.g., an endogenous RNA molecule). Various analytes that may comprise target sequences, and methods of associating target sequences with different analytes, are described in Section VI.B below.

In some embodiments, the target sequence is present in a group of related molecules, e.g. isoforms or variants or mutants of an RNA transcript for a given gene. In some embodiments, the target sequence is specific to a particular subset of molecules (e.g., specific to a particular variant or mutant of an endogenous analyte such as an RNA molecule. For example, in some embodiments, the target sequence comprises a particular single nucleotide variant. In some embodiments, the target sequence may be unique or specific to the particular variant. In this way different variants, or isoforms, or mutants may be identified or distinguished from one another using the primary probes or probe sets.

Where the analyte is a nucleic acid molecule, the target sequence (e.g., a marker sequence) may be a sequence present in the target analyte molecule, or a complement thereof (e.g. a reverse complement thereof). It may therefore be or comprise a variant or mutant sequence etc. present in the analyte, or a conserved sequence present in an analyte group which is specific to that group. The target sequence (e.g., a marker sequence) may alternatively be present in or incorporated into a product of an endogenous analyte or labeling agent as a tag or identifier (ID) sequence (e.g. a barcode) for the analyte or labeling agent. It may thus be a synthetic or artificial sequence.

In some embodiments, the probe or probe set comprises one or more barcode sequences or complements thereof. The barcode sequences may be positioned anywhere within the nucleic acid probe or probe set. If more than one barcode sequence is present, the barcode sequences may be positioned next to each other, and/or interspersed with other sequences. In some embodiments, two or more of the barcode sequences may also at least partially overlap. In some embodiments, two or more of the barcode sequences in the same probe do not overlap. In some embodiments, all of the barcode sequences in the same probe are separated from one another by at least a phosphodiester bond (e.g., they may be immediately adjacent to each other but do not overlap), such as 1, 2, 3, 4, 5, 6, 7, 8, 9, 10 or more nucleotides apart. In some embodiments, one or more barcodes are indicative of the target sequence in the target nucleic acid, such as a single nucleotide of interest (e.g., SNPs or point mutations), a dinucleotide sequence, or a short sequence of about 5 nucleotides in length in the target sequence.

In some embodiments, the probe comprises one or more barcode sequences or complements thereof. The barcode sequences may be positioned anywhere within the nucleic acid probe. If more than one barcode sequence is present, the barcode sequences may be positioned next to each other, and/or interspersed with other sequences. In some embodiments, two or more of the barcode sequences may also at least partially overlap. In some embodiments, two or more of the barcode sequences in the same probe do not overlap. In some embodiments, all of the barcode sequences in the same probe are separated from one another by at least a phosphodiester bond (e.g., they may be immediately adjacent to each other but do not overlap), such as 1, 2, 3, 4, 5, 6, 7, 8, 9, 10 or more nucleotides apart. In some embodiments, one or more barcodes are indicative of the target sequence in the target nucleic acid, such as a single nucleotide of interest (e.g., SNPs or point mutations), a dinucleotide sequence, or a short sequence of about 5 nucleotides in length in the target sequence.

In some embodiments, the probe set comprises one or more barcode sequences or complements thereof. The barcode sequences may be positioned anywhere within the nucleic acid probe set. If more than one barcode sequence is present, the barcode sequences may be positioned next to each other, and/or interspersed with other sequences. In some embodiments, two or more of the barcode sequences may also at least partially overlap. In some embodiments, two or more of the barcode sequences in the same probe set do not overlap. In some embodiments, all of the barcode sequences in the same probe set are separated from one another by at least a phosphodiester bond (e.g., they may be immediately adjacent to each other but do not overlap), such as 1, 2, 3, 4, 5, 6, 7, 8, 9, 10 or more nucleotides apart. In some embodiments, one or more barcodes are indicative of the target sequence in the target nucleic acid, such as a single nucleotide of interest (e.g., SNPs or point mutations), a dinucleotide sequence, or a short sequence of about 5 nucleotides in length in the target sequence.

The barcode sequences, if present, may be of any length. If more than one barcode sequence is used, the barcode sequences may independently have the same or different lengths, such as at least 5, at least 10, at least 15, at least 20, at least 25, at least 30, at least 35, at least 40, at least 50 nucleotides in length. In some embodiments, the barcode sequence may be no more than 120, no more than 112, no more than 104, no more than 96, no more than 88, no more than 80, no more than 72, no more than 64, no more than 56, no more than 48, no more than 40, no more than 32, no more than 24, no more than 16, or no more than 8 nucleotides in length. Combinations of any of these are also possible, e.g., the barcode sequence may be between 5 and 10 nucleotides, between 8 and 15 nucleotides, etc.

The barcode sequence may be arbitrary or random. In certain cases, the barcode sequences are chosen so as to reduce or minimize homology with other components in a sample, e.g., such that the barcode sequences do not themselves bind to or hybridize with other nucleic acids suspected of being within the cell or other sample. In some embodiments, between a particular barcode sequence and another sequence (e.g., a cellular nucleic acid sequence in a sample or other barcode sequences in probes added to the sample), the homology may be less than 10%, less than 8%, less than 7%, less than 6%, less than 5%, less than 4%, less than 3%, less than 2%, or less than 1%. In some embodiments, the homology may be less than 20, 19, 18, 17, 16, 15, 14, 13, 12, 11, 10, 9, 8, 7, 6, 5, 4, 3, or 2 bases, and in some embodiments, the bases are consecutive bases.

In some embodiments, a group of nucleic acid probes hybridize to a group of target sequences in an RNA analyte (e.g., mRNA). For example, a group of nucleic acid probes described herein can be designed to hybridize to a plurality of target sequences (e.g., marker sequences) present in the nucleic acid analyte. In some embodiments, the analyte comprises between or between about 10 and 20, 10 and 15, 10 and 30, 20 and 30, 20 and 40, 20 and 50, 40 and 50, or 45 and 60 target sequences (e.g., marker sequences). The target sequences can comprise copies of the same target sequences and/or can comprise different target sequences. Hybridization of a plurality of nucleic acid probes to a given nucleic acid analyte increases the number of binding sites available for oligonucleotide probes associated with labels or with the absence of a label, resulting in a strong signal intensity, and thus an increase in the signal to noise ratio. In turn, this allows for highly sensitive detection of target analytes, and enables, for example, the detection of rare transcripts or mutations. In some embodiments, a plurality of nucleic acid probes that hybridizes to each given target nucleic acid shares the same barcode sequences.

In some embodiments, target sequences in an analyte such as an RNA (e.g., mRNA) are designed to optimize hybridization of a plurality of nucleic acid probes under a constant set of hybridization conditions, e.g., incubation temperature. For example, target sequences can be designed to cover a relatively narrow range of GC content and melting temperatures (TM) with the target binding regions of the corresponding nucleic acid probes. In some embodiments, target sequences for endogenous RNAs have limited homology to other RNAs in the transcriptome, reducing the probability that a nucleic acid probe will bind to the wrong RNA. Design considerations for target sequences have been described, for example, in U.S. Patent Application Publication Nos. 2017/0220733 and 2017/0212986; and U.S. Patent No. 11,098,303.

### B. Rolling Circle Amplification

In some embodiments, the methods provided herein comprise generating a product of a primary probe or probe set (e.g., described in Section III.A.). In some embodiments, a product is an amplification product of a primary probe or probe set bound to an RNA transcript (e.g., a rolling circle amplification product of a circularizable probe or probe set).

In some embodiments, the methods provided herein comprise generating a product of a primary probe. In some embodiments, a product of a primary probe is an amplification product of a primary probe bound to an RNA transcript (e.g., a rolling circle amplification product of a circularizable probe). In some embodiments, the product of a primary probe is a rolling circle amplification product (RCP). In some embodiments, the methods provided herein comprise generating a product of a primary probe set. In some embodiments, a product of a primary probe set is an amplification product of a primary probe set bound to an RNA transcript (e.g., a rolling circle amplification product of a circularizable probe set). In some embodiments, the product of a primary probe set is a rolling circle amplification product (RCP).

In some embodiments, the method comprises circularizing a circularizable probe or probe set that hybridizes to a target sequence in the test biological sample. In some embodiments, the method comprises circularizing probes or probe sets of the probe mixture that hybridize to target RNA transcripts or products thereof (e.g., cDNA of the RNA transcripts) in the test biological sample. In some embodiments, the probes or probe sets are circularizable by ligation.

In some embodiments, the method comprises circularizing a circularizable probe or probe set that hybridizes to a target sequence in the test biological sample, wherein the biological sample comprises a first cell population and a second cell population comprising a population of Jurkat cells and a population of Raji cells. In some embodiments, the method comprises circularizing probes or probe sets of the probe mixture that hybridize to target RNA transcripts or products thereof (e.g., cDNA of the RNA transcripts) in the test biological sample comprising the cell population of Jurkat cells and the cell population of Raji cells.

In some embodiments, the method comprises circularizing a circularizable probe that hybridizes to a target sequence in the test biological sample. In some embodiments, the method comprises circularizing probes of the probe mixture that hybridize to target RNA transcripts or products thereof (e.g., cDNA of the RNA transcripts) in the test biological sample. In some embodiments, the probes are circularizable by ligation.

In some embodiments, the method comprises circularizing a circularizable probe set that hybridizes to a target sequence in the test biological sample. In some embodiments, the method comprises circularizing probe sets of the probe mixture that hybridize to target RNA transcripts or products thereof (e.g., cDNA of the RNA transcripts) in the test biological sample. In some embodiments, the probe sets are circularizable by ligation.

In some embodiments, the method comprises circularizing a circularizable probe or probe set that hybridizes to a target sequence in the test biological sample, wherein the target sequence comprises a gene that is expressed by a population of Jurkat cells. In some embodiments, the method comprises circularizing a circularizable probe that hybridizes to a target sequence in the test biological sample, wherein the target sequence comprises a gene that is expressed by a population of Jurkat cells. In some embodiments, the method comprises circularizing a circularizable probe set that hybridizes to a target sequence in the test biological sample, wherein the target sequence comprises a gene that is expressed by a population of Jurkat cells. In some embodiments, the gene is a population-specific gene with reference to the population of Jurkat cells. In some embodiments, the gene is a commonly expressed gene (i.e., a housekeeping gene) that is also expressed in a population of Raji cells.

In some embodiments, the method comprises circularizing a circularizable probe or probe set that hybridizes to a target sequence in the test biological sample, wherein the target sequence comprises a gene that is expressed by a population of Raji cells. In some embodiments, the method comprises circularizing a circularizable probe that hybridizes to a target sequence in the test biological sample, wherein the target sequence comprises a gene that is expressed by a population of Raji cells. In some embodiments, the method comprises circularizing a circularizable probe set that hybridizes to a target sequence in the test biological sample, wherein the target sequence comprises a gene that is expressed by a population of Raji cells. In some embodiments, the gene is a population-specific gene with reference to the population of Raji cells. In some embodiments, the gene is a commonly expressed gene (i.e., a housekeeping gene) that is also expressed in a population of Jurkat cells.

In some embodiments, the method comprises circularizing a circularizable probe or probe set that hybridizes to a target sequence in the test biological sample, wherein the biological sample comprises a first cell population and a second cell population comprising a population of Jurkat cells and a population of Raji cells, and wherein the target sequence comprises a gene that is expressed by a population of Jurkat cells and/or a population of Raji cells. In some embodiments, the method comprises circularizing probes or probe sets of the probe mixture that hybridize to target RNA transcripts or products thereof (e.g., cDNA of the RNA transcripts) in the test biological sample comprising the cell population of Jurkat cells and the cell population of Raji cells.

In some embodiments, the probes or probe sets are circularizable by DNA-templated ligation, such as from a cDNA molecule. See, e.g., U.S. Pat. 8,551,710. In some embodiments, provided herein is a probe or probe set capable of RNA-templated ligation. See, e.g., U.S. Pat. Pub. 2020/0224244. In some embodiments, the probe set is a SNAIL probe set. See, e.g., U.S. Pat. Pub. 20190055594. In some embodiments, provided herein is a multiplexed proximity ligation assay. See, e.g., U.S. Pat. Pub. 20140194311. In some embodiments, provided herein is a probe or probe set capable of proximity ligation, for instance a proximity ligation assay for RNA (e.g., PLAYR) probe set. See, e.g., U.S. Pat. Pub. 20160108458. In some embodiments, a circular probe can be indirectly hybridized to the target nucleic acid. In some embodiments, the circular construct is formed from a probe set capable of proximity ligation, for instance a proximity ligation *in situ* hybridization (PLISH) probe set. See, e.g., U.S. Pat. Pub. 2020/0224243.

In some embodiments, the probes are circularizable by DNA-templated ligation, such as from a cDNA molecule. In some embodiments, provided herein is a probe capable of RNA-templated ligation. In some embodiments, provided herein is a multiplexed proximity ligation assay. In some embodiments, provided herein is a probe capable of proximity ligation, for instance a proximity ligation assay for RNA (e.g., PLAYR) probe. In some embodiments, a circular probe can be indirectly hybridized to the target nucleic acid.

In some embodiments, the probe sets are circularizable by DNA-templated ligation, such as from a cDNA molecule. In some embodiments, provided herein is a probe set capable of RNA-templated ligation. In some embodiments, the probe set is a SNAIL probe set. In some embodiments, provided herein is a multiplexed proximity ligation assay. In some embodiments, provided herein is a probe set capable of proximity ligation, for instance a proximity ligation assay for RNA (e.g., PLAYR) probe set. In some embodiments, a circular probe set can be indirectly hybridized to the target nucleic acid. In some embodiments, the circular construct is formed from a probe set capable of proximity ligation, for instance a proximity ligation *in situ* hybridization (PLISH) probe set.

In some embodiments, the ligation involves chemical ligation *(e.g.,* click chemistry ligation). In some embodiments, the chemical ligation involves template dependent ligation. In some embodiments, the chemical ligation involves template independent ligation. In some embodiments, the click reaction is a template-independent reaction *(see, e.g.,* Xiong and Seela (2011), J. Org. Chem. 76(14): 5584-5597). In some embodiments, the click reaction is a template-dependent reaction or template-directed reaction. In some embodiments, the template-dependent reaction is sensitive to base pair mismatches such that reaction rate is significantly higher for matched versus unmatched templates. In some embodiments, the click reaction is a nucleophilic addition template-dependent reaction. In some embodiments, the click reaction is a cyclopropane-tetrazine template-dependent reaction.

In some embodiments, the ligation involves enzymatic ligation. In some embodiments, the enzymatic ligation involves use of a ligase. In some aspects, the ligase used herein comprises an enzyme that is commonly used to join polynucleotides together or to join the ends of a single polynucleotide. An RNA ligase, a DNA ligase, or another variety of ligase can be used to ligate two nucleotide sequences together. Ligases comprise ATP-dependent double-strand polynucleotide ligases, NAD-i-dependent double-strand DNA or RNA ligases and single-strand polynucleotide ligases, for example any of the ligases described in EC 6.5.1.1 (ATP-dependent ligases), EC 6.5.1.2 (NAD+-dependent ligases), EC 6.5.1.3 (RNA ligases). Specific examples of ligases comprise bacterial ligases such as E. coli DNA ligase, Tth DNA ligase, Thermococcus sp. (strain 9° N) DNA ligase (9°N^{™} DNA ligase, New England Biolabs), Taq DNA ligase, Ampligase^{™} (Epicentre Biotechnologies) and phage ligases such as T3 DNA ligase, T4 DNA ligase and T7 DNA ligase and mutants thereof. In some embodiments, the ligase is a T4 RNA ligase. In some embodiments, the ligase is a splintR ligase. In some embodiments, the ligase is a single stranded DNA ligase. In some embodiments, the ligase is a T4 DNA ligase. In some embodiments, the ligase is a ligase that has an DNA-splinted DNA ligase activity. In some embodiments, the ligase is a ligase that has an RNA-splinted DNA ligase activity.

In some embodiments, the ligation herein is a direct ligation. In some embodiments, the ligation herein is an indirect ligation. "Direct ligation" means that the ends of the polynucleotides hybridize immediately adjacently to one another to form a substrate for a ligase enzyme resulting in their ligation to each other (intramolecular ligation). Alternatively, "indirect" means that the ends of the polynucleotides hybridize non-adjacently to one another, i.e., separated by one or more intervening nucleotides or "gaps". In some embodiments, said ends are not ligated directly to each other, but instead occurs either via the intermediacy of one or more intervening (so-called "gap" or "gap-filling" (oligo)nucleotides) or by the extension of the 3' end of a probe to "fill" the "gap" corresponding to said intervening nucleotides (intermolecular ligation). In some cases, the gap of one or more nucleotides between the hybridized ends of the polynucleotides may be "filled" by one or more "gap" (oligo)nucleotide(s) which are complementary to a splint, padlock probe, or target nucleic acid. The gap may be a gap of 1 to 60 nucleotides or a gap of 1 to 40 nucleotides or a gap of 3 to 40 nucleotides. In specific embodiments, the gap may be a gap of about 1, 2, 3, 4, 5, 6, 7, 8, 9 or 10 or more nucleotides, of any integer (or range of integers) of nucleotides in between the indicated values. In some embodiments, the gap between said terminal regions may be filled by a gap oligonucleotide or by extending the 3' end of a polynucleotide. In some cases, ligation involves ligating the ends of the probe to at least one gap (oligo)nucleotide, such that the gap (oligo)nucleotide becomes incorporated into the resulting polynucleotide. In some embodiments, the ligation herein is preceded by gap filling. In other embodiments, the ligation herein does not require gap filling.

In some embodiments, ligation of the polynucleotides produces polynucleotides with melting temperature higher than that of unligated polynucleotides. Thus, in some aspects, ligation stabilizes the hybridization complex containing the ligated polynucleotides prior to subsequent steps, comprising amplification and detection.

In some aspects, a high fidelity ligase, such as a thermostable DNA ligase (e.g., a *Taq* DNA ligase), is used. Thermostable DNA ligases are active at elevated temperatures, allowing further discrimination by incubating the ligation at a temperature near the melting temperature (Tₘ) of the DNA strands. This selectively reduces the concentration of annealed mismatched substrates (expected to have a slightly lower Tₘ around the mismatch) over annealed fully base-paired substrates. Thus, high-fidelity ligation can be achieved through a combination of the intrinsic selectivity of the ligase active site and balanced conditions to reduce the incidence of annealed mismatched dsDNA.

In some embodiments, the ligation herein is a proximity ligation of ligating two (or more) nucleic acid sequences that are in proximity with each other, e.g., through enzymatic means (e.g., a ligase). In some embodiments, proximity ligation can include a "gap-filling" step that involves incorporation of one or more nucleic acids by a polymerase, based on the nucleic acid sequence of a template nucleic acid molecule, spanning a distance between the two nucleic acid molecules of interest (see, e.g., U.S. Patent No. 7,264,929). A wide variety of different methods can be used for proximity ligating nucleic acid molecules, including (but not limited to) "sticky-end" and "blunt-end" ligations. Additionally, single-stranded ligation can be used to perform proximity ligation on a single-stranded nucleic acid molecule. Sticky-end proximity ligations involve the hybridization of complementary single-stranded sequences between the two nucleic acid molecules to be joined, prior to the ligation event itself. Blunt-end proximity ligations generally do not include hybridization of complementary regions from each nucleic acid molecule because both nucleic acid molecules lack a single-stranded overhang at the site of ligation.

In some embodiments, the method comprises contacting the biological sample with a primer that hybridizes to a primer binding region in the probe or probe set. In some embodiments, the method comprises contacting the biological sample with a primer that hybridizes to a primer binding region in the probe. In some embodiments, the method comprises contacting the biological sample with a primer that hybridizes to a primer binding region in the probe set. In some embodiments, the method comprises contacting the biological sample with a primer that hybridizes to a primer binding region in the probe, wherein the biological sample comprises a cell population of Jurkat cells and a cell population of Raji cells. In some embodiments, the method comprises contacting the biological sample with a primer that hybridizes to a primer binding region in the probe set, wherein the biological sample comprises a cell population of Jurkat cells and a cell population of Raji cells. In some embodiments, the primer is a single-stranded nucleic acid sequence having a 3' end that can be used as a substrate for a nucleic acid polymerase in a nucleic acid extension reaction. RNA primers are formed of RNA nucleotides, and are used in RNA synthesis, while DNA primers are formed of DNA nucleotides and used in DNA synthesis. Primers can also include both RNA nucleotides and DNA nucleotides (e.g., in a random or designed pattern). Primers can also include other natural or synthetic nucleotides described herein that can have additional functionality. In some examples, DNA primers can be used to prime RNA synthesis and vice versa (e.g., RNA primers can be used to prime DNA synthesis). Primers can vary in length. For example, primers can be about 6 bases to about 120 bases. For example, primers can include up to about 25 bases. A primer, may in some cases, refer to a primer binding sequence. A primer extension reaction generally refers to any method where two nucleic acid sequences become linked (e.g., hybridized) by an overlap of their respective terminal complementary nucleic acid sequences (e.g., 3' termini). Such linking can be followed by nucleic acid extension (e.g., an enzymatic extension) of one, or both termini using the other nucleic acid sequence as a template for extension. Enzymatic extension can be performed by an enzyme including, but not limited to, a polymerase and/or a reverse transcriptase.

In some embodiments, the method comprises performing amplification of circularizable probes or probe sets (e.g., following circularization of the probes or probe sets) in the test biological sample. In some embodiments, the method comprises performing amplification of circularizable probes (e.g., following circularization of the probes) in the test biological sample. In some embodiments, the method comprises performing amplification of circularizable probe sets (e.g., following circularization of the probe sets) in the test biological sample. In some embodiments, the method comprises performing amplification of circularizable probes (e.g., following circularization of the probes) in the test biological sample, wherein the test biological sample comprises a cell population of Jurkat cells and a cell population of Raji cells. In some embodiments, the method comprises performing amplification of circularizable probe sets (e.g., following circularization of the probe sets) in the test biological sample, wherein the test biologal sample comprises a cell population of Jurkat cells and a cell population of Raji cells. In some embodiments, the amplification is performed at a temperature between or between about 20°C and about 60°C. In some embodiments, the amplification is performed at a temperature between or between about 30°C and about 40°C. In some aspects, the amplification step, such as the rolling circle amplification (RCA) is performed at a temperature between at or about 25°C and at or about 50°C, such as at or about 25°C, 27°C, 29°C, 31°C, 33°C, 35°C, 37°C, 39°C, 41°C, 43°C, 45°C, 47°C, or 49°C.

In some embodiments, upon addition of a DNA polymerase in the presence of appropriate dNTP precursors and other cofactors, a primer is elongated to produce multiple copies of the circular template (e.g., a circular or circularized probe or probe set). This amplification step can utilize isothermal amplification or non-isothermal amplification. In some embodiments, after the formation of the hybridization complex and association of the amplification probe, the hybridization complex is rolling-circle amplified to generate a cDNA nanoball (i.e., amplicon) containing multiple copies of the cDNA. Techniques for rolling circle amplification (RCA) include linear RCA, a branched RCA, a dendritic RCA, or any combination thereof. (See, e.g., Baner et al, Nucleic Acids Research, 26:5073-5078, 1998; Lizardi et al, Nature Genetics 19:226, 1998; Mohsen et al., Acc Chem Res. 2016 November 15; 49(11): 2540-2550; Schweitzer et al. Proc. Natl Acad. Sci. USA 97:101 13- 1 19, 2000; Faruqi et al, BMC Genomics 2:4, 2000; Nallur et al, Nucl. Acids Res. 29:el 18, 2001; Dean et al. Genome Res. 1 1 :1095- 1099, 2001; Schweitzer et al, Nature Biotech. 20:359-365, 2002; U.S. Patent Nos. 6,054,274, 6,291,187, 6,323,009, 6,344,329 and 6,368,801). Exemplary polymerases for use in RCA comprise DNA polymerase such phi29 (φ29) polymerase, Klenow fragment, *Bacillus stearothermophilus* DNA polymerase (BST), T4 DNA polymerase, T7 DNA polymerase, or DNA polymerase I. In some aspects, DNA polymerases that have been engineered or mutated to have desirable characteristics can be employed. In some embodiments, the polymerase is phi29 DNA polymerase.

In some aspects, during the amplification step, modified nucleotides can be added to the reaction to incorporate the modified nucleotides in the amplification product (e.g., nanoball). Exemplary of the modified nucleotides comprise amine-modified nucleotides. In some aspects of the methods, for example, for anchoring or cross-linking of the generated amplification product (e.g., nanoball) to a scaffold, to cellular structures and/or to other amplification products (e.g., other nanoballs). In some aspects, the amplification products comprises a modified nucleotide, such as an amine-modified nucleotide. In some embodiments, the amine-modified nucleotide comprises an acrylic acid N- hydroxysuccinimide moiety modification. Examples of other amine-modified nucleotides comprise, but are not limited to, a 5-Aminoallyl-dUTP moiety modification, a 5-Propargylamino-dCTP moiety modification, a N6-6-Aminohexyl-dATP moiety modification, or a 7-Deaza-7-Propargylamino-dATP moiety modification.

In some aspects, the polynucleotides and/or amplification product (e.g., amplicon) can be anchored to a polymer matrix. For example, the polymer matrix can be a hydrogel. In some embodiments, one or more of the polynucleotide probe(s) can be modified to contain functional groups that can be used as an anchoring site to attach the polynucleotide probes and/or amplification product to a polymer matrix. Exemplary modification and polymer matrix that can be employed in accordance with the provided embodiments comprise those described in, for example, WO 2014/163886, WO 2017/079406, US 2016/0024555, US 2018/0251833 and US 2017/0219465. In some examples, the scaffold also contains modifications or functional groups that can react with or incorporate the modifications or functional groups of the probe set or amplification product. In some examples, the scaffold can comprise oligonucleotides, polymers or chemical groups, to provide a matrix and/or support structures.

The amplification products may be immobilized within the matrix generally at the location of the nucleic acid being amplified, thereby creating a localized colony of amplicons. The amplification products may be immobilized within the matrix by steric factors. The amplification products may also be immobilized within the matrix by covalent or noncovalent bonding. In this manner, the amplification products may be considered to be attached to the matrix. By being immobilized to the matrix, such as by covalent bonding or cross-linking, the size and spatial relationship of the original amplicons is maintained. By being immobilized to the matrix, such as by covalent bonding or cross-linking, the amplification products are resistant to movement or unraveling under mechanical stress.

In some aspects, the amplification products are copolymerized and/or covalently attached to the surrounding matrix thereby preserving their spatial relationship and any information inherent thereto. For example, if the amplification products are those generated from DNA or RNA within a cell embedded in the matrix, the amplification products can also be functionalized to form covalent attachment to the matrix preserving their spatial information within the cell thereby providing a subcellular localization distribution pattern. In some embodiments, the provided methods involve embedding the one or more polynucleotide probe sets and/or the amplification products in the presence of hydrogel subunits to form one or more hydrogel-embedded amplification products. In some embodiments, the hydrogel-tissue chemistry described comprises covalently attaching nucleic acids to in situ synthesized hydrogel for tissue clearing, enzyme diffusion, and multiple-cycle sequencing while an existing hydrogel-tissue chemistry method cannot. In some embodiments, to enable amplification product embedding in the tissue-hydrogel setting, amine-modified nucleotides are comprised in the amplification step (e.g., RCA), functionalized with an acrylamide moiety using acrylic acid N-hydroxysuccinimide esters, and copolymerized with acrylamide monomers to form a hydrogel.

### C. Detection and Analysis

**In** some aspects, the provided methods involve analyzing, e.g., detecting or determining, one or more sequences present in the probes or probe sets or products thereof (e.g., rolling circle amplification products thereof). In some embodiments, products of the hybridized probes or probe sets are amplification products of the hybridized probes or probe sets. In some embodiments, products of the hybridized probes or probe sets are rolling circle amplification products (RCPs). In some embodiments, the detecting is performed at one or more locations in the test biological sample. In some embodiments, the locations are the locations of RNA transcripts in the test biological sample. In some embodiments, the locations are the locations at which the probes or probe sets hybridize to the RNA transcripts in the biological sample, and are optionally ligated and amplified by rolling circle amplification.

In some embodiments, the provided methods involve analyzing, e.g., detecting or determining, one or more sequences present in the probes or probe sets or products thereof (e.g., rolling circle amplification products thereof) within the biological sample, wherein the biological sample comprises a cell population of Jurkat cells and a cell population of Raji cells. In some embodiments, products of the hybridized probes or probe sets are amplification products of the hybridized probes or probe sets. In some embodiments, products of the hybridized probes or probe sets are rolling circle amplification products (RCPs). In some embodiments, the detecting is performed at one or more locations in the test biological sample, wherein the biological ample comprises a cell population of Jurkat cells and a cell population of Raji cells. In some embodiments, the locations are the locations of RNA transcripts in the test biological sample, wherein the biological sample comprises a cell population of Jurkat cells and a cell population of Raji cells. In some embodiments, the locations are the locations at which the probes or probe sets hybridize to the RNA transcripts in the biological sample comprising a cell population of Jurkat cells and a cell population of Raji cells, and are optionally ligated and amplified by rolling circle amplification.

In some embodiments, detecting the one or more sequences present in the probes or probe sets in the test biological sample is performed, and the detected sequences are compared to an expected set of detected sequences. In some embodiments, the expected set of sequences is based on the barcode sequences of the panels of probes or probe sets in the probe mixture and the known expression levels of the RNA transcripts of the first, second, and/or third sets of genes in the first and second cell populations. In some embodiments, the one or more sequences are one or more barcode sequences or complements thereof. In some embodiments, the expected set of detected sequences include sequences expected to be detected at a high expression level (e.g., more than 20 counts of the detected sequence per cell) in one or both of the first and second cell populations. In some embodiments, the expected set of detected sequences include sequences expected to be detected at a medium expression level (e.g., 5-20 counts of the detected sequence per cell) in one or both of the first and second cell populations. In some embodiments, the expected set of detected sequences include sequences expected to be detected at a low expression level (e.g., 1-5 counts of the detected sequence per cell) in one or both of the first and second cell populations.

In some embodiments, detecting the one or more sequences present in the probes in the test biological sample is performed, and the detected sequences are compared to an expected set of detected sequences. In some embodiments, the expected set of sequences is based on the barcode sequences of the panels of probes in the probe mixture and the known expression levels of the RNA transcripts of the first, second, and/or third sets of genes in the first and second cell populations. In some embodiments, the one or more sequences are one or more barcode sequences or complements thereof. In some embodiments, the expected set of detected sequences include sequences expected to be detected at a high expression level (e.g., more than 20 counts of the detected sequence per cell) in one or both of the first and second cell populations. In some embodiments, the expected set of detected sequences include sequences expected to be detected at a medium expression level (e.g., 5-20 counts of the detected sequence per cell) in one or both of the first and second cell populations. In some embodiments, the expected set of detected sequences include sequences expected to be detected at a low expression level (e.g., 1-5 counts of the detected sequence per cell) in one or both of the first and second cell populations.

In some embodiments, detecting the one or more sequences present in the probe sets in the test biological sample is performed, and the detected sequences are compared to an expected set of detected sequences. In some embodiments, the expected set of sequences is based on the barcode sequences of the panels of probe sets in the probe mixture and the known expression levels of the RNA transcripts of the first, second, and/or third sets of genes in the first and second cell populations. In some embodiments, the one or more sequences are one or more barcode sequences or complements thereof. In some embodiments, the expected set of detected sequences include sequences expected to be detected at a high expression level (e.g., more than 20 counts of the detected sequence per cell) in one or both of the first and second cell populations. In some embodiments, the expected set of detected sequences include sequences expected to be detected at a medium expression level (e.g., 5-20 counts of the detected sequence per cell) in one or both of the first and second cell populations. In some embodiments, the expected set of detected sequences include sequences expected to be detected at a low expression level (e.g., 1-5 counts of the detected sequence per cell) in one or both of the first and second cell populations.

In some embodiments, detecting the one or more sequences present in the probes or probe sets in the test biological sample is performed, and the detected sequences are compared to an expected set of detected sequences, wherein the biological sample comprises a population of Jurkat cells and a population of Raji cells. In some embodiments, the expected set of sequences is based on the barcode sequences of the panels of probes or probe sets in the probe mixture and the known expression levels of the RNA transcripts of the first, second, and/or third sets of genes in the population of Jurkat cells and in the population of Raji cells. In some embodiments, the one or more sequences are one or more barcode sequences or complements thereof. In some embodiments, the expected set of detected sequences include sequences expected to be detected at a high expression level (e.g., more than 20 counts of the detected sequence per cell) in one or both of the population of Jurkat cells and the population of Raji cells. In some embodiments, the expected set of detected sequences include sequences expected to be detected at a medium expression level (e.g., 5-20 counts of the detected sequence per cell) in one or both of the population of Jurkat cells and the population of Raji cells. In some embodiments, the expected set of detected sequences include sequences expected to be detected at a low expression level (e.g., 1-5 counts of the detected sequence per cell) in one or both of the population of Jurkat cells and the population of Raji cells. In some embodiments, the detecting comprises a plurality of repeated cycles of hybridization and removal of probes (e.g., detectably labeled probes, or intermediate probes that bind to detectably labeled probes) to the primary probe or probe set hybridized to the target nucleic acid, or to a rolling circle amplification product generated from the probe or probe set hybridized to the target nucleic acid. In some embodiments, the detecting comprises a plurality of repeated cycles of hybridization and removal of probes (e.g., detectably labeled probes, or intermediate probes that bind to detectably labeled probes) to the primary probe hybridized to the target nucleic acid, or to a rolling circle amplification product generated from the probe hybridized to the target nucleic acid. In some embodiments, the detecting comprises a plurality of repeated cycles of hybridization and removal of probes (e.g., detectably labeled probes, or intermediate probes that bind to detectably labeled probes) to the primary probe set hybridized to the target nucleic acid, or to a rolling circle amplification product generated from the probe set hybridized to the target nucleic acid.

Methods for binding and identifying a target nucleic acid that uses various probes or oligonucleotides have been described in, e.g., US2003/0013091, US2007/0166708, US2010/0015607, US2010/0261026, US2010/0262374, US2010/0112710, US2010/0047924, and US2014/0371088. Detectably-labeled probes can be useful for detecting multiple target nucleic acids and be detected in one or more hybridization cycles (e.g., sequential hybridization assays, or sequencing by hybridization).

In some embodiments, the detecting can comprise binding an intermediate probe directly or indirectly to the primary probe or probe set, binding a detectably labeled probe directly or indirectly to a detection region of the intermediate probe, and detecting a signal associated with the detectably labeled probe. In some embodiments, the method comprises detecting a rolling circle amplification product (RCP) generated using a circular or circularized primary probe or probe set as a template. In some embodiments, the method comprises detecting a rolling circle amplification product (RCP) generated using a circular or circularized probe or probe that binds to a primary probe or probe set as a template. In some embodiments, detecting the RCP comprises binding an intermediate probe directly or indirectly to the RCP, binding a detectably labeled probe directly or indirectly to a detection region of the intermediate probe, and detecting a signal associated with the detectably labeled probe. In some embodiments, the method can comprise performing one or more wash steps to remove unbound and/or nonspecifically bound intermediate probe molecules from the primary probes or the products of the primary probes.

In some embodiments, the detecting can comprise binding an intermediate probe directly or indirectly to the primary probe, binding a detectably labeled probe directly or indirectly to a detection region of the intermediate probe, and detecting a signal associated with the detectably labeled probe. In some embodiments, the method comprises detecting a rolling circle amplification product (RCP) generated using a circular or circularized primary probe as a template. In some embodiments, the method comprises detecting a rolling circle amplification product (RCP) generated using a circular or circularized probe or probe that binds to a primary probe set as a template. In some embodiments, detecting the RCP comprises binding an intermediate probe directly or indirectly to the RCP, binding a detectably labeled probe directly or indirectly to a detection region of the intermediate probe, and detecting a signal associated with the detectably labeled probe. In some embodiments, the method can comprise performing one or more wash steps to remove unbound and/or nonspecifically bound intermediate probe molecules from the primary probes or the products of the primary probes.

In some embodiments, the detecting can comprise binding an intermediate probe directly or indirectly to the primary probe set, binding a detectably labeled probe directly or indirectly to a detection region of the intermediate probe, and detecting a signal associated with the detectably labeled probe. In some embodiments, the method comprises detecting a rolling circle amplification product (RCP) generated using a circular or circularized primary probe set as a template. In some embodiments, the method comprises detecting a rolling circle amplification product (RCP) generated using a circular or circularized probe or probe that binds to a primary probe set as a template. In some embodiments, detecting the RCP comprises binding an intermediate probe directly or indirectly to the RCP, binding a detectably labeled probe directly or indirectly to a detection region of the intermediate probe, and detecting a signal associated with the detectably labeled probe. In some embodiments, the method can comprise performing one or more wash steps to remove unbound and/or nonspecifically bound intermediate probe molecules from the primary probe set or the products of the primary probe set.

In some embodiments, the detecting can comprise binding an intermediate probe directly or indirectly to the primary probe or probe set, binding a detectably labeled probe directly or indirectly to a detection region of the intermediate probe, and detecting a signal associated with the detectably labeled probe within a test biological sample. In some embodiments, the test biological sample comprises a cell population of Jurkat cells and a cell population of Raji cells. In some embodiments, the method comprises detecting a rolling circle amplification product (RCP) generated using a circular or circularized primary probe or probe set as a template within a test biological sample comprising a cell population of Jurkat cells and a cell population of Raji cells. In some embodiments, the method comprises detecting a rolling circle amplification product (RCP) generated using a circular or circularized probe or probe that binds to a primary probe or probe set as a template within a test biological sample comprising a cell population of Jurkat cells and a cell population of Raji cells. In some embodiments, detecting the RCP comprises binding an intermediate probe directly or indirectly to the RCP, binding a detectably labeled probe directly or indirectly to a detection region of the intermediate probe, and detecting a signal associated with the detectably labeled probe within a test biological sample, wherein the test biological sample comprises a cell population of Jurkat cells and a cell population of Raji cells. In some embodiments, the method can comprise performing one or more wash steps to remove unbound and/or nonspecifically bound intermediate probe molecules from the primary probes or the products of the primary probes from the test biological sample, wherein the biological sample comprises a cell population of Jurkat cells and a cell population of Raji cells.

In some embodiments, the detecting can comprise: detecting signals associated with detectably labeled probes that are hybridized to barcode regions or complements thereof in the primary probe or probe set or a product thereof (e.g., an RCP); and/or detecting signals associated with detectably labeled probes that are hybridized to intermediate probes which are in turn hybridized to the barcode regions or complements thereof. In some embodiments, products of the hybridized probes or probe sets are amplification products of the hybridized probes or probe sets. In some embodiments, products of the hybridized probes or probe sets are rolling circle amplification products (RCPs). In some embodiments, products of the hybridized probes are amplification products of the hybridized probes. In some embodiments, products of the hybridized probes are rolling circle amplification products (RCPs). In some embodiments, products of the hybridized probe sets are amplification products of the hybridized probe sets. In some embodiments, products of the hybridized probe sets are rolling circle amplification products (RCPs). In some embodiments, the detecting can comprise: detecting signals associated with detectably labeled probes that are hybridized to barcode regions or complements thereof in the primary probe or a product thereof (e.g., an RCP); and/or detecting signals associated with detectably labeled probes that are hybridized to intermediate probes which are in turn hybridized to the barcode regions or complements thereof. In some embodiments, the detecting can comprise: detecting signals associated with detectably labeled probes that are hybridized to barcode regions or complements thereof in the primary probe set or a product thereof (e.g., an RCP); and/or detecting signals associated with detectably labeled probes that are hybridized to intermediate probes which are in turn hybridized to the barcode regions or complements thereof. In some embodiments, the detectably labeled probes can be fluorescently labeled.

In some embodiments, the methods comprise detecting the sequence in all or a portion of a primary probe or probe set or an RCP, or detecting a sequence of the primary probe or probe set or RCP, such as one or more barcode sequences present in the primary probe or probe set or RCP. In some embodiments, the sequence of the RCP, or barcode thereof, is indicative of a sequence of the target nucleic acid to which the RCP is hybridized. In some embodiments, the analysis and/or sequence determination comprises detecting a sequence in all or a portion of the nucleic acid concatemer and/or *in situ* hybridization to the RCP. In some embodiments, the detection step involves sequencing by hybridization, sequencing by ligation, sequencing by synthesis, sequencing by binding, and/or fluorescent *in situ* sequencing (FISSEQ), and/or hybridization-based *in situ* sequencing. In some embodiments, the detection step is by sequential fluorescent *in situ* hybridization (e.g., for combinatorial decoding of the barcode sequence or complement thereof). In some embodiments, the analysis and/or sequence determination comprises detecting a polymer generated by a hybridization chain reaction (HCR) reaction. In some embodiments, the detection or determination comprises hybridizing to the first overhang a detection oligonucleotide labeled with a fluorophore, an isotope, a mass tag, or a combination thereof. In some embodiments, the detection or determination comprises imaging the probe hybridized to the target nucleic acid (e.g., imaging one or more detectably labeled probes hybridized thereto). In some embodiments, the target nucleic acid is an mRNA in a tissue sample, and the detection or determination is performed when the target nucleic acid and/or the amplification product is *in situ* in the tissue sample. In some embodiments, the target nucleic acid is an amplification product (e.g., a rolling circle amplification product). In some embodiments, the target nucleic acid is an mRNA in a tissue sample, and the detection or determination is performed when the target nucleic acid and/or the amplification product is *in situ* in the tissue sample, wherein the tissue sample is a test biological sample comprising a cell population of Jurkat cells and a cell population of Raji cells.

In some embodiments, the methods comprise detecting the sequence in all or a portion of a primary probe, or detecting a sequence of the primary probe, such as one or more barcode sequences present in the primary probe. In some embodiments, the methods comprise detecting the sequence in all or a portion of a primary probe set, or detecting a sequence of the primary probe set, such as one or more barcode sequences present in the primary probe set. In some embodiments, the methods comprise detecting the sequence in all or a portion of an RCP, or detecting a sequence of the RCP, such as one or more barcode sequences present in the RCP. In some embodiments, the sequence of the RCP, or barcode thereof, is indicative of a sequence of the target nucleic acid to which the RCP is hybridized. In some embodiments, the analysis and/or sequence determination comprises detecting a sequence in all or a portion of the nucleic acid concatemer and/or *in situ* hybridization to the RCP. In some embodiments, the detection step involves sequencing by hybridization, sequencing by ligation, sequencing by synthesis, sequencing by binding, and/or fluorescent *in situ* sequencing (FISSEQ), and/or hybridization-based *in situ* sequencing. In some embodiments, the detection step is by sequential fluorescent *in situ* hybridization (e.g., for combinatorial decoding of the barcode sequence or complement thereof). In some embodiments, the analysis and/or sequence determination comprises detecting a polymer generated by a hybridization chain reaction (HCR) reaction. In some embodiments, the detection or determination comprises hybridizing to the first overhang a detection oligonucleotide labeled with a fluorophore, an isotope, a mass tag, or a combination thereof. In some embodiments, the detection or determination comprises imaging the probe hybridized to the target nucleic acid (e.g., imaging one or more detectably labeled probes hybridized thereto). In some embodiments, the target nucleic acid is an mRNA in a tissue sample, and the detection or determination is performed when the target nucleic acid and/or the amplification product is *in situ* in the tissue sample. In some embodiments, the target nucleic acid is an amplification product (e.g., a rolling circle amplification product).

In some aspects, the provided methods comprise imaging a detectably labeled probe bound directly or indirectly to the primary probe or probe set or product thereof and detecting the detectable label. In some embodiments, products of the hybridized probes or probe sets are amplification products of the hybridized probes or probe sets. In some embodiments, products of the hybridized probes or probe sets are rolling circle amplification products (RCPs). In some embodiments, the detectably labeled probe comprises a detectable label that can be measured and quantitated. The label or detectable label can comprise a directly or indirectly detectable moiety, e.g., any fluorophores, radioactive isotopes, fluorescers, chemiluminescers, enzymes, enzyme substrates, enzyme cofactors, enzyme inhibitors, chromophores, dyes, metal ions, metal sols, ligands (e.g., biotin or haptens) and the like.

In some aspects, the provided methods comprise imaging a detectably labeled probe bound directly or indirectly to the primary probe or product thereof and detecting the detectable label. In some embodiments, products of the hybridized probes or probe sets are amplification products of the hybridized probes. In some embodiments, products of the hybridized probes are rolling circle amplification products (RCPs). In some embodiments, the detectably labeled probe comprises a detectable label that can be measured and quantitated. The label or detectable label can comprise a directly or indirectly detectable moiety, e.g., any fluorophores, radioactive isotopes, fluorescers, chemiluminescers, enzymes, enzyme substrates, enzyme cofactors, enzyme inhibitors, chromophores, dyes, metal ions, metal sols, ligands (e.g., biotin or haptens) and the like.

In some aspects, the provided methods comprise imaging a detectably labeled probe bound directly or indirectly to the primary probe set or product thereof and detecting the detectable label. In some embodiments, products of the hybridized probe sets are amplification products of the hybridized probes or probe sets. In some embodiments, products of the hybridized probe sets are rolling circle amplification products (RCPs). In some embodiments, the detectably labeled probe comprises a detectable label that can be measured and quantitated. The label or detectable label can comprise a directly or indirectly detectable moiety, e.g., any fluorophores, radioactive isotopes, fluorescers, chemiluminescers, enzymes, enzyme substrates, enzyme cofactors, enzyme inhibitors, chromophores, dyes, metal ions, metal sols, ligands (e.g., biotin or haptens) and the like.

A fluorophore can comprise a substance or a portion thereof that is capable of exhibiting fluorescence in the detectable range. Particular examples of labels that may be used in accordance with the provided embodiments comprise, but are not limited to phycoerythrin, Alexa dyes, fluorescein, YPet, CyPet, Cascade blue, allophycocyanin, Cy3, Cy5, Cy7, rhodamine, dansyl, umbelliferone, Texas red, luminol, acradimum esters, biotin, green fluorescent protein (GFP), enhanced green fluorescent protein (EGFP), yellow fluorescent protein (YFP), enhanced yellow fluorescent protein (EYFP), blue fluorescent protein (BFP), red fluorescent protein (RFP), firefly luciferase, Renilla luciferase, NADPH, beta-galactosidase, horseradish peroxidase, glucose oxidase, alkaline phosphatase, chloramphenical acetyl transferase, and urease.

Fluorescence detection in tissue samples can often be hindered by the presence of strong background fluorescence. Background fluorescence can include autofluorescence (that can arise from a variety of sources, including aldehyde fixation, extracellular matrix components, red blood cells, lipofuscin, and the like), as opposed to the desired immunofluorescence from the fluorescently labeled antibodies or probes. Tissue autofluorescence can lead to difficulties in distinguishing the signals due to fluorescent antibodies or probes from the general background. In some embodiments, a method disclosed herein utilizes one or more agents to reduce tissue autofluorescence, for example, Autofluorescence Eliminator (Sigma/EMD Millipore), TrueBlack Lipofuscin Autofluorescence Quencher (Biotium), MaxBlock Autofluorescence Reducing Reagent Kit (MaxVision Biosciences), and/or a very intense black dye (e.g., Sudan Black, or comparable dark chromophore).

Examples of detectable labels comprise but are not limited to various radioactive moieties, enzymes, prosthetic groups, fluorescent markers, luminescent markers, bioluminescent markers, metal particles, protein-protein binding pairs and protein-antibody binding pairs. Examples of fluorescent proteins comprise, but are not limited to, yellow fluorescent protein (YFP), green fluorescence protein (GFP), cyan fluorescence protein (CFP), umbelliferone, fluorescein, fluorescein isothiocyanate, rhodamine, dichlorotriazinylamine fluorescein, dansyl chloride and phycoerythrin.

Examples of bioluminescent markers comprise, but are not limited to, luciferase (e.g., bacterial, firefly and click beetle), luciferin, aequorin and the like. Examples of enzyme systems having visually detectable signals comprise, but are not limited to, galactosidases, glucorimidases, phosphatases, peroxidases and cholinesterases. Identifiable markers also comprise radioactive compounds such as ¹²⁵I, ³⁵S, ¹⁴C, or ³H. Identifiable markers are commercially available from a variety of sources.

Examples of fluorescent labels and nucleotides and/or polynucleotides conjugated to such fluorescent labels comprise those described in, for example, Hoagland, Handbook of Fluorescent Probes and Research Chemicals, Ninth Edition (Molecular Probes, Inc., Eugene, 2002); Keller and Manak, DNA Probes, 2nd Edition (Stockton Press, New York, 1993); Eckstein, editor, Oligonucleotides and Analogues: A Practical Approach (IRL Press, Oxford, 1991); and Wetmur, Critical Reviews in Biochemistry and Molecular Biology, 26:227-259 (1991). In some embodiments, exemplary techniques and methods methodologies applicable to the provided embodiments comprise those described in, for example, US 4,757,141, US 5,151,507 and US 5,091,519. In some embodiments, one or more fluorescent dyes are used as labels for labeled target sequences, for example, as described in US 5,188,934 (4,7-dichlorofluorescein dyes); US 5,366,860 (spectrally resolvable rhodamine dyes); US 5,847,162 (4,7- dichlororhodamine dyes); US 4,318,846 (ether-substituted fluorescein dyes); US 5,800,996 (energy transfer dyes); US 5,066,580 (xanthine dyes); and US 5,688,648 (energy transfer dyes). Labeling can also be carried out with quantum dots, as described in US 6,322,901, US 6,576,291, US 6,423,551, US 6,251,303, US 6,319,426, US 6,426,513, US 6,444,143, US 5,990,479, US 6,207,392, US 2002/0045045 and US 2003/0017264. In some embodiments, a fluorescent label comprises a signaling moiety that conveys information through the fluorescent absorption and/or emission properties of one or more molecules. Exemplary fluorescent properties comprise fluorescence intensity, fluorescence lifetime, emission spectrum characteristics and energy transfer.

Examples of commercially available fluorescent nucleotide analogues readily incorporated into nucleotide and/or polynucleotide sequences comprise, but are not limited to, Cy3-dCTP, Cy3-dUTP, Cy5-dCTP, Cy5-dUTP (Amersham Biosciences, Piscataway, N.J.), fluorescein- !2-dUTP, tetramethylrhodamine-6-dUTP, TEXAS RED^{™}-5-dUTP, CASCADE BLUE^{™}.7-dUTP, BODIPY TMFL-14-dUTP, BODIPY TMR-14-dUTP, BODIPY TMTR-14-dUTP, RHOD AMINE GREEN^{™}-5-dUTP, OREGON GREENR^{™} 488-5-dUTP, TEXAS RED^{™}-12-dUTP, BODIPY^{™} 630/650-14-dUTP, BODIPY^{™} 650/665-14-dUTP, ALEXA FLUOR^{™} 488-5-dUTP, ALEXA FLUOR^{™} 532-5-dUTP, ALEXA FLUOR^{™} 568-5-dUTP, ALEXA FLUOR^{™} 594-5-dUTP, ALEXA FLUOR^{™} 546-14-dUTP, fluorescein- 12-UTP, tetramethylrhodamine-6-UTP, TEXAS RED^{™}-5- UTP, mCherry, CASCADE BLUE^{™}-7-UTP, BODIPY^{™} FL-14-UTP, BODIPY TMR- 14-UTP, BODIPY^{™} TR-14-UTP, RHOD AMINE GREEN^{™}-5-UTP, ALEXA FLUOR^{™} 488-5-UTP, and ALEXA FLUOR^{™} 546-14-UTP (Molecular Probes, Inc. Eugene, Oreg.). Methods are known for custom synthesis of nucleotides having other fluorophores (See, Henegariu et al. (2000) Nature Biotechnol. 18:345).

Other fluorophores available for post-synthetic attachment comprise, but are not limited to, ALEXA FLUOR^{™} 350, ALEXA FLUOR^{™} 532, ALEXA FLUOR^{™} 546, ALEXA FLUOR^{™} 568, ALEXA FLUOR^{™} 594, ALEXA FLUOR^{™} 647, BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethyl rhodamine, Texas Red (available from Molecular Probes, Inc., Eugene, Oreg.), Cy2, Cy3.5, Cy5.5, and Cy7 (Amersham Biosciences, Piscataway, N.J.). FRET tandem fluorophores may also be used, comprising, but not limited to, PerCP-Cy5.5, PE-Cy5, PE-Cy5.5, PE-Cy7, PE-Texas Red, APC-Cy7, PE-Alexa dyes (610, 647, 680), and APC-Alexa dyes.

In some cases, metallic silver or gold particles may be used to enhance signal from fluorescently labeled nucleotide and/or polynucleotide sequences (Lakowicz et al. (2003) Bio Techniques 34:62).

Biotin, or a derivative thereof, may also be used as a label on a nucleotide and/or a polynucleotide sequence, and subsequently bound by a detectably labeled avidin/streptavidin derivative (e.g., phycoerythrin-conjugated streptavidin), or a detectably labeled anti-biotin antibody. Digoxigenin may be incorporated as a label and subsequently bound by a detectably labeled anti-digoxigenin antibody (e.g., fluoresceinated anti-digoxigenin). An aminoallyl-dUTP residue may be incorporated into a polynucleotide sequence and subsequently coupled to an N-hydroxy succinimide (NHS) derivatized fluorescent dye. In general, any member of a conjugate pair may be incorporated into a detection polynucleotide provided that a detectably labeled conjugate partner can be bound to permit detection.

Other suitable labels for a polynucleotide sequence may comprise fluorescein (FAM), digoxigenin, dinitrophenol (DNP), dansyl, biotin, bromodeoxyuridine (BrdU), hexahistidine (6xHis), and phosphor-amino acids (e.g., P-tyr, P-ser, P-thr). In some embodiments the following hapten/antibody pairs are used for detection, in which each of the antibodies is derivatized with a detectable label: biotin/a-biotin, digoxigenin/a- digoxigenin, dinitrophenol (DNP)/a-DNP, 5-Carboxyfluorescein (FAM)/a-FAM.

In some embodiments, a nucleotide and/or a oligonucleotide sequence can be indirectly labeled, especially with a hapten that is then bound by a capture agent, e.g., as disclosed in US 5,344,757, US 5,702,888, US 5,354,657, US 5,198,537 and US 4,849,336, and US 5,073,562. Many different hapten-capture agent pairs are available for use. Exemplary haptens comprise, but are not limited to, biotin, des-biotin and other derivatives, dinitrophenol, dansyl, fluorescein, Cy5, and digoxigenin. For biotin, a capture agent may be avidin, streptavidin, or antibodies. Antibodies may be used as capture agents for the other haptens (many dye-antibody pairs being commercially available, e.g., Molecular Probes, Eugene, Oreg.).

In some aspects, the detecting involves using detection methods such as flow cytometry; sequencing; probe binding and electrochemical detection; pH alteration; catalysis induced by enzymes bound to DNA tags; quantum entanglement; Raman spectroscopy; terahertz wave technology; and/or scanning electron microscopy. In some aspects, the flow cytometry is mass cytometry or fluorescence-activated flow cytometry. In some aspects, the detecting comprises performing microscopy, scanning mass spectrometry or other imaging techniques described herein. In such aspects, the detecting comprises determining a signal, e.g., a fluorescent signal.

In some aspects, the detection (comprising imaging) is carried out using any of a number of different types of microscopy, e.g., confocal microscopy, two-photon microscopy, light-field microscopy, intact tissue expansion microscopy, and/or CLARITY^{™}-optimized light sheet microscopy (COLM).

In some embodiments, fluorescence microscopy is used for detection and imaging of the detection probe. In some aspects, a fluorescence microscope is an optical microscope that uses fluorescence and phosphorescence instead of, or in addition to, reflection and absorption to study properties of organic or inorganic substances. In fluorescence microscopy, a sample is illuminated with light of a wavelength which excites fluorescence in the sample. The fluoresced light, which is usually at a longer wavelength than the illumination, is then imaged through a microscope objective. Two filters may be used in this technique; an illumination (or excitation) filter which ensures the illumination is near monochromatic and at the correct wavelength, and a second emission (or barrier) filter which ensures none of the excitation light source reaches the detector. Alternatively, these functions may both be accomplished by a single dichroic filter. The fluorescence microscope can be any microscope that uses fluorescence to generate an image, whether it is a more simple set up like an epifluorescence microscope, or a more complicated design such as a confocal microscope, which uses optical sectioning to get better resolution of the fluorescent image.

In some embodiments, confocal microscopy is used for detection and imaging of the detection probe. Confocal microscopy uses point illumination and a pinhole in an optically conjugate plane in front of the detector to eliminate out-of-focus signal. As only light produced by fluorescence very close to the focal plane can be detected, the image's optical resolution, particularly in the sample depth direction, is much better than that of wide-field microscopes. However, as much of the light from sample fluorescence is blocked at the pinhole, this increased resolution is at the cost of decreased signal intensity - so long exposures are often required. As only one point in the sample is illuminated at a time, 2D or 3D imaging requires scanning over a regular raster (e.g., a rectangular pattern of parallel scanning lines) in the specimen. The achievable thickness of the focal plane is defined mostly by the wavelength of the used light divided by the numerical aperture of the objective lens, but also by the optical properties of the specimen. The thin optical sectioning possible makes these types of microscopes particularly good at 3D imaging and surface profiling of samples. CLARITY^{™}-optimized light sheet microscopy (COLM) provides an alternative microscopy for fast 3D imaging of large clarified samples. COLM interrogates large immunostained tissues, permits increased speed of acquisition and results in a higher quality of generated data.

Other types of microscopy that can be employed comprise bright field microscopy, oblique illumination microscopy, dark field microscopy, phase contrast, differential interference contrast (DIC) microscopy, interference reflection microscopy (also known as reflected interference contrast, or RIC), single plane illumination microscopy (SPIM), super-resolution microscopy, laser microscopy, electron microscopy (EM), Transmission electron microscopy (TEM), Scanning electron microscopy (SEM), reflection electron microscopy (REM), Scanning transmission electron microscopy (STEM) and low- voltage electron microscopy (LVEM), scanning probe microscopy (SPM), atomic force microscopy (ATM), ballistic electron emission microscopy (BEEM), chemical force microscopy (CFM), conductive atomic force microscopy (C- AFM), electrochemical scanning tunneling microscope (ECSTM), electrostatic force microscopy (EFM), fluidic force microscope (FluidFM), force modulation microscopy (FMM), feature-oriented scanning probe microscopy (FOSPM), kelvin probe force microscopy (KPFM), magnetic force microscopy (MFM), magnetic resonance force microscopy (MRFM), near-field scanning optical microscopy (NSOM) (or SNOM, scanning near-field optical microscopy, SNOM, Piezoresponse Force Microscopy (PFM), PSTM, photon scanning tunneling microscopy (PSTM), PTMS, photothermal microspectroscopy/ microscopy (PTMS), SCM, scanning capacitance microscopy (SCM), SECM, scanning electrochemical microscopy (SECM), SGM, scanning gate microscopy (SGM), SHPM, scanning Hall probe microscopy (SHPM), SICM, scanning ion-conductance microscopy (SICM), SPSM spin polarized scanning tunneling microscopy (SPSM), SSRM, scanning spreading resistance microscopy (SSRM), SThM, scanning thermal microscopy (SThM), STM, scanning tunneling microscopy (STM), STP, scanning tunneling potentiometry (STP), SVM, scanning voltage microscopy (SVM), and synchrotron x-ray scanning tunneling microscopy (SXSTM), and intact tissue expansion microscopy (exM).

In some embodiments, the assay comprises *in situ* sequencing. *In situ* sequencing typically involves incorporation of a labeled nucleotide (e.g., fluorescently labeled mononucleotides or dinucleotides) in a sequential, template-dependent manner or hybridization of a labeled primer (e.g., a labeled random hexamer) to a nucleic acid template such that the identities (e.g., nucleotide sequence) of the incorporated nucleotides or labeled primer extension products can be determined, and consequently, the nucleotide sequence of the corresponding template nucleic acid. Aspects of *in situ* sequencing are described, for example, in Mitra et al., (2003) Anal. Biochem. 320, 55-65, and Lee et al., (2014) Science, 343(6177), 1360-1363. In addition, examples of methods and systems for performing *in situ* sequencing are described in US 2016/0024555, US 2019/0194709, and in US 10,138,509, US 10,494,662 and US 10,179,932. Exemplary techniques for in situ sequencing or in in situ sequence detection comprise, but are not limited to, STARmap (described for example in Wang et al., (2018) Science, 361(6499) 5691), MERFISH (described for example in Moffitt, (2016) Methods in Enzymology, 572, 1-49), hybridization-based *in situ* sequencing (HybISS) (described for example in Gyllborg et al., Nucleic Acids Res (2020) 48(19):e112, and FISSEQ (described for example in US 2019/0032121).

In some embodiments, sequencing can be performed by sequencing-by-synthesis (SBS). In some embodiments, a sequencing primer is complementary to sequences at or near the one or more barcode(s). In such embodiments, sequencing-by-synthesis can comprise reverse transcription and/or amplification in order to generate a template sequence from which a primer sequence can bind. Exemplary SBS methods comprise those described for example, but not limited to, US 2007/0166705, US 2006/0188901, US 7,057,026, US 2006/0240439, US 2006/0281109, US 2011/0059865, US 2005/0100900, US 9,217,178, US 2009/0118128, US 2012/0270305, US 2013/0260372, and US 2013/0079232.

In some embodiments, detection of the barcode sequences is performed by sequential hybridization of probes to the barcode sequences or complements thereof and detecting complexes formed by the probes and barcode sequences or complements thereof. In some cases, each barcode sequence or complement thereof is assigned a sequence of signal codes that identifies the barcode sequence or complement thereof (e.g., a temporal signal signature or code that identifies the analyte), and detecting the barcode sequences or complements thereof can comprise decoding the barcode sequences of complements thereof by detecting the corresponding sequences of signal codes detected from sequential hybridization, detection, and removal of sequential pools of intermediate probes and the universal pool of detectably labeled probes. In some cases, the sequences of signal codes can be fluorophore sequences assigned to the corresponding barcode sequences or complements thereof. In some embodiments, the detectably labeled probes are fluorescently labeled. In some embodiments, the barcode sequence or complement thereof is performed by sequential probe hybridization as described in US 2021/0340618.

In any of the embodiments herein, the detecting step can comprise contacting the biological sample with one or more detectably labeled probes that directly or indirectly hybridize to the barcode sequences or complements thereof (e.g., in amplification products generated using the probes or probe sets), and dehybridizing the one or more detectably labeled probes. In any of the embodiments herein, the contacting and dehybridizing steps can be repeated with the one or more detectably labeled probes and/or one or more other detectably labeled probes that directly or indirectly hybridize to the barcode sequences or complements thereof. In some aspects, the method comprises sequential hybridization of detectably labeled probes to create a spatiotemporal signal signature or code that identifies the analyte.

In any of the embodiments herein, the detecting step can comprise contacting the biological sample with one or more first detectably labeled probes that directly hybridize to the plurality of probes or probe sets. In some instances, the detecting step can comprise contacting the biological sample with one or more first detectably labeled probes that indirectly hybridize to the plurality of probes or probe sets. In any of the embodiments herein, the detecting step can comprise contacting the biological sample with one or more first detectably labeled probes that directly or indirectly hybridize to the plurality of probes or probe sets.

In any of the embodiments herein, the detecting step can comprise contacting the biological sample with one or more first detectably labeled probes that directly hybridize to the plurality of probes. In some instances, the detecting step can comprise contacting the biological sample with one or more first detectably labeled probes that indirectly hybridize to the plurality of probes. In any of the embodiments herein, the detecting step can comprise contacting the biological sample with one or more first detectably labeled probes that directly or indirectly hybridize to the plurality of probes.

In any of the embodiments herein, the detecting step can comprise contacting the biological sample with one or more first detectably labeled probes that directly hybridize to the plurality of probe sets. In some instances, the detecting step can comprise contacting the biological sample with one or more first detectably labeled probes that indirectly hybridize to the plurality of probe sets. In any of the embodiments herein, the detecting step can comprise contacting the biological sample with one or more first detectably labeled probes that directly or indirectly hybridize to the plurality of probe sets.

In any of the embodiments herein, the detecting step can comprise contacting the biological sample with one or more intermediate probes that directly or indirectly hybridize to the barcode sequences or complements thereof (e.g., of the plurality of probes or probe sets or rolling circle amplification product generated using the plurality of probes or probe sets), wherein the one or more intermediate probes are detectable using one or more detectably labeled probes. In any of the embodiments herein, the detecting step can further comprise dehybridizing the one or more intermediate probes and/or the one or more detectably labeled probes from the barcode sequences or complements thereof (e.g., of the plurality of probes or probe sets or rolling circle amplification product generated using the plurality of probes or probe sets). In any of the embodiments herein, the contacting and dehybridizing steps can be repeated with the one or more intermediate probes, the one or more detectably labeled probes, one or more other intermediate probes, and/or one or more other detectably labeled probes. In any of the embodiments herein, the detecting step can comprise contacting the biological sample with one or more intermediate probes that directly or indirectly hybridize to the barcode sequences or complements thereof (e.g., of the plurality of probes or probe sets or rolling circle amplification product generated using the plurality of probes or probe sets), wherein the biological sample comprises a cell pellet comprising a population of Jurkat cells and a population of Raji cells, and wherein the one or more intermediate probes are detectable using one or more detectably labeled probes. In any of the embodiments herein, the detecting step can further comprise dehybridizing the one or more intermediate probes and/or the one or more detectably labeled probes from the barcode sequences or complements thereof (e.g., of the plurality of probes or probe sets or rolling circle amplification product generated using the plurality of probes or probe sets). In any of the embodiments herein, the contacting and dehybridizing steps can be repeated with the one or more intermediate probes, the one or more detectably labeled probes, one or more other intermediate probes, and/or one or more other detectably labeled probes.

In some embodiments, sequencing can be performed using single molecule sequencing by ligation. Such techniques utilize DNA ligase to incorporate oligonucleotides and identify the incorporation of such oligonucleotides. The oligonucleotides typically have different labels that are correlated with the identity of a particular nucleotide in a sequence to which the oligonucleotides hybridize. Aspects and features involved in sequencing by ligation are described, for example, in Shendure et al. Science (2005), 309: 1728-1732, and in US 5,599,675; US 5,750,341; US 6,969,488; US 6,172,218; US and 6,306,597.

In some embodiments, nucleic acid hybridization can be used for sequencing. These methods utilize labeled nucleic acid decoder probes that are complementary to at least a portion of a barcode sequence. Multiplex decoding can be performed with pools of many different probes with distinguishable labels. Non-limiting examples of nucleic acid hybridization sequencing are described for example in US 8,460,865, and in Gunderson et al., Genome Research 14:870-877 (2004).

In some embodiments, real-time monitoring of DNA polymerase activity can be used during sequencing. For example, nucleotide incorporations can be detected through fluorescence resonance energy transfer (FRET), as described for example in Levene et al., Science (2003), 299, 682-686, Lundquist et al., Opt. Lett. (2008), 33, 1026-1028, and Korlach et al., Proc. Natl. Acad. Sci. USA (2008), 105, 1176-1181.

In some aspects, the analysis and/or sequence determination can be carried out at room temperature for best preservation of tissue morphology with low background noise and error reduction. In some embodiments, the analysis and/or sequence determination comprises eliminating error accumulation as sequencing proceeds.

In some embodiments, the analysis and/or sequence determination involves washing to remove unbound polynucleotides, thereafter revealing a fluorescent product for imaging.

### IV. ASSESSING IN SITU ASSAY PERFORMANCE

In some aspects, the methods provided herein comprise detecting probes or probe sets hybridized to target RNA transcripts in a test biological sample (or products of the hybridized probes or probe sets, such as RCA products), thereby detecting RNA transcripts of selected genes in the test biological sample. In some aspects, the methods include identifying discrete cells in the test biological sample and determining the detected RNA transcripts in the discrete cells, and comparing the detected RNA transcripts in the discrete cells to expected expression levels of the selected genes.

In some aspects, the methods provided herein comprise detecting probes or probe sets hybridized to target RNA transcripts in a test biological sample (or products of the hybridized probes, such as RCA products), thereby detecting RNA transcripts of selected genes in the test biological sample. In some aspects, the methods include identifying discrete cells in the test biological sample and determining the detected RNA transcripts in the discrete cells, and comparing the detected RNA transcripts in the discrete cells to expected expression levels of the selected genes.

In some aspects, the methods provided herein comprise detecting probe sets hybridized to target RNA transcripts in a test biological sample (or products of the hybridized probe sets, such as RCA products), thereby detecting RNA transcripts of selected genes in the test biological sample. In some aspects, the methods include identifying discrete cells in the test biological sample and determining the detected RNA transcripts in the discrete cells, and comparing the detected RNA transcripts in the discrete cells to expected expression levels of the selected genes.

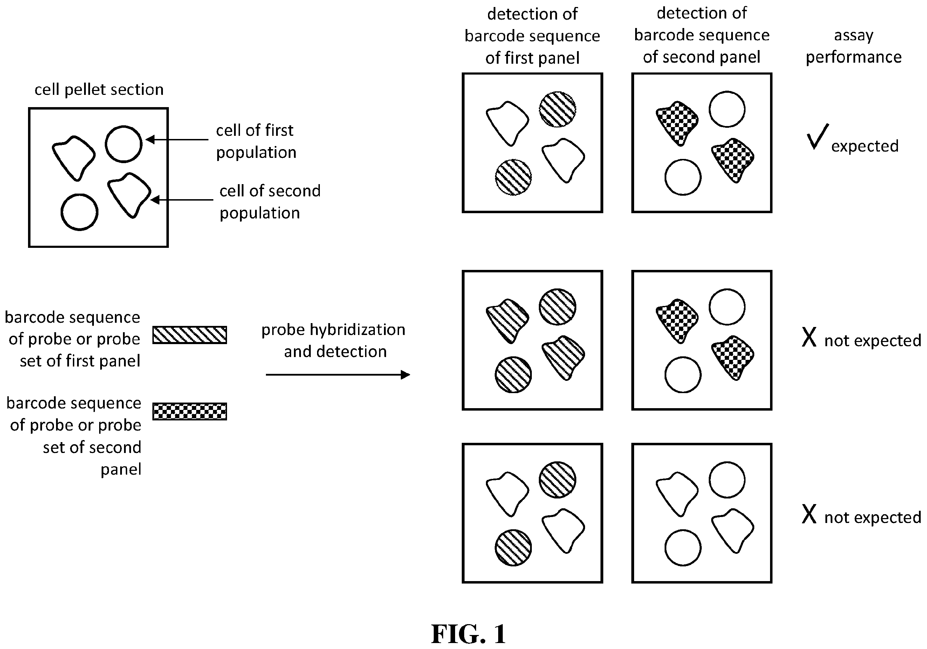

As illustrated in **FIG. 1****,** in some embodiments, a method for analyzing *in situ* assay performance herein comprises contacting a test biological sample (e.g., a cell pellet section) with a probes mixture comprising a first panel of probes or probe sets and a second panel of probes or probe sets. The test biological sample comprises a first cell population and a second cell population in a defined mixture. In the example shown in **FIG. 1****,** the first cell population and the second cell population are mixed in a cell pellet, and the test biological sample is a section of the cell pellet on a substrate. In some embodiments, each probe or probe set of the first panel comprises a recognition sequence complementary to a target sequence of an RNA transcript or product thereof of a first set of genes expressed in the first cell population, and each probe or probe set of the second panel comprises a recognition sequence complementary to a target sequence of an RNA transcript or product thereof of a second set of genes expressed in the second cell population. In some embodiments, the first set of genes is specifically expressed in the first cell population, and the second set of genes is specifically expressed in the second cell population. In some embodiments, the first set of genes is not detectably expressed in the second cell population. In some embodiments, the second set of genes is not detectably expressed in the first cell population. The method comprises detecting barcode sequences or complements thereof of the probes or probe sets or products thereof (e.g., RCA products thereof) hybridized to their target sequences in the test biological sample. In some embodiments, products of the hybridized probes or probe sets are amplification products of the hybridized probes or probe sets. In some embodiments, products of the hybridized probes or probe sets are rolling circle amplification products (RCPs). The barcode sequences can be detected by any suitable means (e.g., sequential probe hybridization) In some embodiments, the barcode sequences are detected by sequential binding of detectably labeled probes to the barcode sequences or complements thereof, directly or indirectly (via an intermediate probe).

In some embodiments, a method for analyzing *in situ* assay performance herein comprises contacting a test biological sample (e.g., a cell pellet section) with a probes mixture comprising a first panel of probes and a second panel of probes. In some embodiments, the test biological sample comprises a first cell population and a second cell population in a defined mixture. In the example shown in **FIG. 1****,** the first cell population and the second cell population are mixed in a cell pellet, and the test biological sample is a section of the cell pellet on a substrate. In some embodiments, each probe of the first panel comprises a recognition sequence complementary to a target sequence of an RNA transcript or product thereof of a first set of genes expressed in the first cell population, and each probe of the second panel comprises a recognition sequence complementary to a target sequence of an RNA transcript or product thereof of a second set of genes expressed in the second cell population. In some embodiments, the first set of genes is specifically expressed in the first cell population, and the second set of genes is specifically expressed in the second cell population. In some embodiments, the first set of genes is not detectably expressed in the second cell population. In some embodiments, the second set of genes is not detectably expressed in the first cell population. The method comprises detecting barcode sequences or complements thereof of the probes or products thereof (e.g., RCA products thereof) hybridized to their target sequences in the test biological sample. The barcode sequences can be detected by any suitable means (e.g., sequential probe hybridization) In some embodiments, the barcode sequences are detected by sequential binding of detectably labeled probes to the barcode sequences or complements thereof, directly or indirectly (via an intermediate probe).

In some embodiments, a method for analyzing *in situ* assay performance herein comprises contacting a test biological sample (e.g., a cell pellet section) with a probes mixture comprising a first panel of probe sets and a second panel of probe sets. In some embodiments, the test biological sample comprises a first cell population and a second cell population in a defined mixture. The first cell population and the second cell population are mixed in a cell pellet, and the test biological sample is a section of the cell pellet (e.g. on a substrate). In some embodiments, each probe set of the first panel comprises a recognition sequence complementary to a target sequence of an RNA transcript or product thereof of a first set of genes expressed in the first cell population, and each probe set of the second panel comprises a recognition sequence complementary to a target sequence of an RNA transcript or product thereof of a second set of genes expressed in the second cell population. In some embodiments, the first set of genes is specifically expressed in the first cell population, and the second set of genes is specifically expressed in the second cell population. In some embodiments, the first set of genes is not detectably expressed in the second cell population. In some embodiments, the second set of genes is not detectably expressed in the first cell population. The method comprises detecting barcode sequences or complements thereof of the probe sets or products thereof (e.g., RCA products thereof) hybridized to their target sequences in the test biological sample. The barcode sequences can be detected by any suitable means (e.g., sequential probe hybridization) In some embodiments, the barcode sequences are detected by sequential binding of detectably labeled probes to the barcode sequences or complements thereof, directly or indirectly (via an intermediate probe).

In some embodiments, a method for analyzing *in situ* assay performance herein comprises contacting a test biological sample (e.g., a cell pellet section) with a probes mixture comprising a first panel of probes or probe sets and a second panel of probes or probe sets. The test biological sample comprises a first cell population and a second cell population in a defined mixture. In some embodiments, the first cell population comprises a population of Jurkat cells and the second cell population comprises a population of Raji cells. In some embodiments, the population of Jurkat cells and the population of Raji cells are mixed in a cell pellet, and the test biological sample is a section of the cell pellet on a substrate. In some embodiments, each probe or probe set of the first panel comprises a recognition sequence complementary to a target sequence of an RNA transcript or product thereof of a first set of genes expressed in Jurkat cells, and each probe or probe set of the second panel comprises a recognition sequence complementary to a target sequence of an RNA transcript or product thereof of a second set of genes expressed in Raji cells. In some embodiments, the first set of genes is specifically expressed in Jurkat cells, and the second set of genes is specifically expressed in Raji cells. In some embodiments, the first set of genes is not detectably expressed in the population of Raji cells. In some embodiments, the second set of genes is not detectably expressed in the population of Jurkat cells. The method comprises detecting barcode sequences or complements thereof of the probes or probe sets or products thereof (e.g., RCA products thereof) hybridized to their target sequences in the test biological sample. The barcode sequences can be detected by any suitable means (e.g., sequential probe hybridization) In some embodiments, the barcode sequences are detected by sequential binding of detectably labeled probes to the barcode sequences or complements thereof, directly or indirectly (via an intermediate probe).

In some embodiments, a method for analyzing *in situ* assay performance herein comprises contacting a test biological sample (e.g., a cell pellet section) with a probes mixture comprising a first panel of probe sets and a second panel of probe sets. In some embodiments, the test biological sample comprises a first cell population comprising a cell population of Jurkat cells and a second cell population comprising a cell population of Raji cells in a defined mixture,. In some embodiments, the population of Jurkat cells and the population of Raji cells are mixed in a cell pellet, and the test biological sample is a section of the cell pellet on a substrate. In some embodiments, each probe set of the first panel comprises a recognition sequence complementary to a target sequence of an RNA transcript or product thereof of a first set of genes expressed in Jurkat cells, and each probe set of the second panel comprises a recognition sequence complementary to a target sequence of an RNA transcript or product thereof of a second set of genes expressed in Raji cells. In some embodiments, the first set of genes is specifically expressed in Jurkat cells, and the second set of genes is specifically expressed in Raji cells. In some embodiments, the first set of genes is not detectably expressed in the population of Raji cells. In some embodiments, the second set of genes is not detectably expressed in the population of Jurkat cells. The method comprises detecting barcode sequences or complements thereof of the probe sets or products thereof (e.g., RCA products thereof) hybridized to their target sequences in the test biological sample. The barcode sequences can be detected by any suitable means (e.g., sequential probe hybridization) In some embodiments, the barcode sequences are detected by sequential binding of detectably labeled probes to the barcode sequences or complements thereof, directly or indirectly (via an intermediate probe).

As shown in **FIG. 1****,** the assay performance can be assessed by comparing the detected barcode sequences or complements thereof of the first and second panels of probes or probe sets corresponding to the first and second sets of genes with expected expression of the first and second sets of genes in the first cell population and second cell population. In some embodiments, detecting the barcode sequences or complements thereof of the first panel of probes or probe sets specifically in the first cell population and not in the second cell population is consistent with successful assay performance (an expected result). In some embodiments, detecting the barcode sequences or complements thereof of the second panel of probes or probe sets specifically in the second cell population and not in the first cell population is consistent with successful assay performance (an expected result). As shown in FIG. 1, in some embodiments, detection of the barcode sequences or complements thereof of the first panel of probes or probe sets in the second cell population (or vice versa for the second panel of probes or probes sets and the first cell population) is not an expected result, indicating a problem with assay performance (e.g., lack of specificity). As shown in the bottom right panel of **FIG. 1****,** in some embodiments, failure to detect a barcode sequence or complement thereof of the second panel of probes in the second cell population is not an expected result, indicating a problem with assay performance (e.g., lack of sensitivity).

In some embodiments, the assay performance can be completed by comparing the detected barcodes or complements thereof of the first and second panels of probes corresponding to the first and second sets of genes with expected expression of the first and second sets of genes in the first cell population and second cell population. In some embodiments, detecting the barcode sequences or complements thereof of the first panel of probes specifically in the first cell population and not in the second cell population is consistent with successful assay performance (an expected result). In some embodiments, detecting the barcode sequences or complements thereof of the second panel of probes specifically in the second cell population and not in the first cell population is consistent with successful assay performance (an expected result). In some embodiments, detection of the barcode sequences or complements thereof of the first panel of probes in the second cell population (or vice versa for the second panel of probes and the first cell population) is not an expected result, indicating a problem with assay performance (e.g., lack of specificity). In some embodiments, failure to detect a barcode sequence or complement thereof of the second panel of probes is not an expected result, indicating a problem with assay performance (e.g., lack of sensitivity).

In some embodiments, the assay performance can be completed by comparing the detected barcodes or complements thereof of the first and second panels of probe sets corresponding to the first and second sets of genes with expected expression of the first and second sets of genes in the first cell population and second cell population. In some embodiments, detecting the barcode sequences or complements thereof of the first panel of probe sets specifically in the first cell population and not in the second cell population is consistent with successful assay performance (an expected result). In some embodiments, detecting the barcode sequences or complements thereof of the second panel of probe sets specifically in the second cell population and not in the first cell population is consistent with successful assay performance (an expected result). In some embodiments, detection of the barcode sequences or complements thereof of the first panel of probe sets in the second cell population (or vice versa for the second panel of probe sets and the first cell population) is not an expected result, indicating a problem with assay performance (e.g., lack of specificity). In some embodiments, failure to detect a barcode sequence or complement thereof of the second panel of probe sets is not an expected result, indicating a problem with assay performance (e.g., lack of sensitivity). In some embodiments, failure to decode a barcode sequence correctly can be indicative of a problem with assay performance.

In some embodiments, the first cell population comprises a population of Jurkat cells and the second cell population comprises a population of Raji cells. In some embodiments, the assay performance can be assessed by comparing the detected barcode sequences or complements thereof of the first and second panels of probes corresponding to the first and second sets of genes with expected expression of the first and second sets of genes in the population of Jurkat cells and the population of Raji cells. In some embodiments, detecting the barcode sequences or complements thereof of the first panel of probes specifically in the population of Jurkat cells and not in the population of Raji cells is consistent with successful assay performance (an expected result). In some embodiments, detecting the barcode sequences or complements thereof of the second panel of probes specifically in the population of Raji cells and not in the population of Jurkat cells is consistent with successful assay performance (an expected result). In some embodiments, detection of the barcode sequences or complements thereof of the first panel of probes in the population of Raji cells (or vice versa for the second panel of probes and the population of Jurkat cells) is not an expected result, indicating a problem with assay performance (e.g., lack of specificity). In some embodiments, failure to detect a barcode sequence or complement thereof of the second panel of probes in the population of Raji cells is not an expected result, indicating a problem with assay performance (e.g., lack of sensitivity).

In some embodiments, the first cell population comprises a population of Jurkat cells and the second cell population comprises a population of Raji cells. In some embodiments, the assay performance can be assessed by comparing the detected barcode sequences or complements thereof of the first and second panels of probe sets corresponding to the first and second sets of genes with expected expression of the first and second sets of genes in the population of Jurkat cells and the population of Raji cells. In some embodiments, detecting the barcode sequences or complements thereof of the first panel of probe sets specifically in the population of Jurkat cells and not in the population of Raji cells is consistent with successful assay performance (an expected result). In some embodiments, detecting the barcode sequences or complements thereof of the second panel of probe sets specifically in the population of Raji cells and not in the population of Jurkat cells is consistent with successful assay performance (an expected result). In some embodiments, detection of the barcode sequences or complements thereof of the first panel of probe sets in the population of Raji cells (or vice versa for the second panel of probes sets and the population of Jurkat cells) is not an expected result, indicating a problem with assay performance (e.g., lack of specificity). In some embodiments, failure to detect a barcode sequence or complement thereof of the second panel of probe sets in the population of Raji cells is not an expected result, indicating a problem with assay performance (e.g., lack of sensitivity).

In some embodiments, the selected genes include genes expected to be specifically expressed in a first cell population in the test biological sample, and genes expected to be specifically expressed in a second cell population in the test biological sample. In some embodiments, the selected genes include a first and second set of genes having mutually exclusive expression in the first and second cell population, respectively. In some aspects, comparing the detected RNA transcripts (e.g., the detected barcode sequences or complements thereof of probes or probe sets designed to hybridize to the corresponding RNA transcripts) comprises evaluating the specificity of the assay. In some embodiments, a barcode sequence or complements thereof corresponding to an RNA transcript of a gene that is not detectably expressed in the first cell population, but is detected in the first cell population, is designated as a false positive. In some embodiments, a barcode sequence or complements thereof corresponding to an RNA transcript of a gene that is not detectably expressed in the second cell population, but is detected in the second cell population, is designated as a false positive. In some embodiments, the method comprises determining the number of false positive barcode sequences detected in test biological sample.

In some embodiments, the selected genes include genes expected to be specifically expressed in a first cell population in the test biological sample, and genes expected to be specifically expressed in a second cell population in the test biological sample. In some embodiments, the first cell population comprises a population of Jurkat cells and the second cell population comprises a population of Raji cells. In some embodiments, the selected genes include a first and second set of genes having mutually exclusive expression in Jurkat cells and Raji cells, respectively. In some aspects, comparing the detected RNA transcripts (e.g., the detected barcode sequences or complements thereof of probes or probe sets designed to hybridize to the corresponding RNA transcripts) comprises evaluating the specificity of the assay. In some embodiments, a barcode sequence or complements thereof corresponding to an RNA transcript of a gene that is not detectably expressed in the population of Jurkat cells, but is detected in the population of Jurkat cells, is designated as a false positive. In some embodiments, a barcode sequence or complements thereof corresponding to an RNA transcript of a gene that is not detectably expressed in the population of Raji cells, but is detected in the population of Raji cells, is designated as a false positive. In some embodiments, the method comprises determining the number of false positive barcode sequences detected in test biological sample.

In any of the embodiments herein, identifying the discrete cells can comprise performing cell segmentation. In any of the embodiments herein, the cell segmentation can comprise identifying nuclei of the discrete cells. In any of the embodiments herein, identifying the nuclei of the discrete cells can comprise detecting a nuclear stain. In any of the embodiments herein, a nuclear stain may be a DAPI stain. In some embodiments, the cell segmentation comprises using a membrane based stain and/or a cytosolic stain. In some cases, the cell segmentation comprises identifying nuclei and performing nuclear expansion. In some aspects, any suitable cell segmentation algorithm such as cellpose (cellpose.org) or omnipose (Culter et al., Nature Methods volume 19, pages1438-1448 (2022)) can be used. In any of the embodiments herein, the cell segmentation can comprise identifying an area of maximum distance 5 µm, 10 µm, or 15 µm from the nucleus. In some embodiments, the cell segmentation comprises identifying an area of maximum distance 5 µm from the nucleus. In any of the embodiments herein, the cell segmentation can comprise identifying the boundaries of the discrete cells. In some embodiments, cell segmentation comprises assigning detected signals and associated decoded transcripts to cells. The detected probes or probe sets or products thereof within a particular cell segment can be assigned to the same cell. In some embodiments, products of the hybridized probes or probe sets are amplification products of the hybridized probes or probe sets. In some embodiments, products of the hybridized probes or probe sets are rolling circle amplification products (RCPs). In some embodiments, the detected probes within a particular cell can be assigned to the same cell. In some embodiments, the detected probe sets within a particular cell segment can be assigned to the same cell. In some embodiments, the products of the detected probes or probe sets, comprising a nucleic acid product, comprising a rolling circle amplification product (RCP) can be assigned to the same cell.

In some embodiments, cell segmentation and decoding results are used to cluster and/or compare cell populations. In some embodiments, the first and second cell populations can be identified by clustering the *in situ* gene expression data to group cells based on their similarities in gene expression profiles (e.g., an unsupervised machine learning step to group cells based on their similarities in gene expression profile). In some embodiments, the grouped cells are identified as belonging to either the first cell population or the second cell population based on their gene expression profiles. In some embodiments, the first and second cell populations are in different areas on a substrate supporting the test biological sample (e.g., within the same optical field of view during the detection steps). Thus, in some cases, the discrete cells and the detected probes or probe sets can be identified as the first or second cell population based on their position on the substrate (e.g., without clustering). In some cases, the discrete cells and the detected probes can be identified as the first or second cell population based on their position on the substrate (e.g., without clustering). In some cases, the discrete cells and the detected probe sets can be identified as the first or second cell population based on their position on the substrate (e.g., without clustering). In some embodiments, the first cell population are Jurkat cells, and the second cell population are Raji cells.

In some embodiments, the first cell population comprises a population of Jurkat cells and the second cell population comprises a population of Raji cells. In some embodiments, the population of Jurkat cells and the population of Raji cells can be identified by clustering the *in situ* gene expression data to group cells based on their similarities in gene expression profiles (e.g., an unsupervised machine learning step to group cells based on their similarities in gene expression profile). In some embodiments, the grouped cells are identified as belonging to either the population of Jurkat cells or the population of Raji cells based on their gene expression profiles. In some embodiments, the population of Jurkat cells and the population of Raji cells are in different areas on a substrate supporting the test biological sample (e.g., within the same optical field of view during the detection steps). Thus, in some cases, the discrete cells and the detected probes or probe sets can be identified as the first or second cell population based on their position on the substrate (e.g., without clustering). In some embodiments, the first cell population are Jurkat cells, and the second cell population are Raji cells.

In some embodiments, the method can comprise analyzing one or more success metrics selected from the group consisting of: a quality score for barcode or barcode complement detection or decoding, median number of different genes detected per cell, mean number of different genes detected per cell, median number of transcripts detected per cell, mean number of transcripts detected per cell, number of transcripts decoded per 100 µm², nuclear transcript density per area (e.g., per 100 µm²), total high quality decoded transcripts, RNA transcript density, and percent of detected transcripts within identified cells. In some aspects, the success metrics can be used to characterize an assay performance as pass (e.g., satisfactory performance) or fail (e.g., unsatisfactory performance). For example, results from the assay can be compared to expected results, such as an expected expression level of a plurality of genes (e.g., mean or median number of transcripts detected per cell, different genes detected per cell, transcript density, or other metrics described herein) to characterize an assay performance as pass (e.g., satisfactory performance) or fail (e.g., unsatisfactory performance). In some aspects, one or more of the metrics are reported as a readout of the assay performed. In some embodiments, results from the assay can be used to determine the median number of transcripts detected per cell, number of cells detected, and number of transcripts decoded per area (e.g., per 100 µm²) to characterize an assay performance as pass (e.g., satisfactory performance) compared to expected results (e.g., minimum levels for passing). In some embodiments, results from the assay can be used to determine the median number of transcripts detected per cell, number of cells detected, total high quality decoded transcripts, and nuclear transcript density per area (e.g., per 100 µm²) to characterize an assay performance as pass (e.g., satisfactory performance) compared to expected results (e.g., minimum levels for passing).

In some aspects, a success metric for a test biological sample comprising a first cell population of Jurkat cells and a second cell population of Raji cells is at least 100 mean transcript counts detected per Jurkat cell and at least 40 mean transcript counts detected per Raji cell. In some aspects, a success metric for a test biological sample comprising a first cell population of Jurkat cells and a second cell population of Raji cells is at least 100 median transcript counts detected per Jurkat cell and at least 40 median transcript counts detected per Raji cell.

In some aspects, the method comprises determining if a calculated score for a test biological sample meets a success metric. In some cases, the score can be a quality score. In any of the embodiments herein, the method can comprise determining a quality score for the sequenced or decoded barcode sequence or complement thereof. In any of the embodiments herein, the quality score can be at least q20. In any of the embodiments herein, the density of detected RNA transcripts having a quality score of at least 20 can be at least 0.15 per µM² (15 transcripts per 100 µM² of tissue area). In any of the embodiments herein, the density of detected RNA transcripts having a quality score of at least 20 can be at least 0.1 per µM² (10 transcripts per 100 µM² of tissue area). In any of the embodiments herein, the percent of detected transcripts within identified cells can be at least any one of 95%, 96%, 97%, 98%, 99%, and 99.5%.

In some aspects, the quality score can be a Phred quality score (Q-score). For example, the quality score can be an indication of the quality of the barcode sequence(s) decoded during the assay. In some aspects, the quality score can be an indication of the likelihood that the barcode sequence decoded was correctly identified and was not an error. In some cases, the quality score is a Phred-scaled quality value estimating the probability of incorrect call. In some embodiments, a probabilistic approach to decoding is used where each observed string of intensity in a neighborhood (e.g., a defined distance around a detected object) is matched to its most likely codeword with some probability and the probability is used to calculate a raw Q-score. In some cases, the raw Q-score is then refined by the negative controls for an adjusted Q-score. In some embodiments, a system is used to determine Phred scaled quality scores. The system may comprise a script, file, program, application, set of instructions, or computer-executable code that is configured to enable a computing device to calculate any of the metrics described herein, e.g., statistics such as mean and median values, quality scores, etc.

In some examples, secondary analysis is performed to assess the quality and specificity of the assay with regards to the test biological sample. In some embodiments, unsupervised clustering analysis and visualization of clusters using UMAP projection is used to assess the quality and specificity of the assay. In some embodiments, k-means clustering is used to assign all cells into two distinct clusters. In some embodiments, the presence of clearly defined clusters defined by expression of population-specific genes indicates specificity of the assay.

In some examples, secondary analysis is performed to assess the quality and specificity of the assay with regards to the test biological sample wherein the biological sample comprises a cell pellet comprising a population of Jurkat cells and a population of Raji cells. In some embodiments, unsupervised clustering analysis and visualization of clusters using UMAP projection is used to assess the quality and specificity of the assay. In some embodiments, k-means clustering is used to assign all cells into two distinct clusters. In some embodiments, the presence of a first cluster defined by relative high expression of Jurkat-specific genes and relative low expression of Raji-specific genes, and a second cluster defined by relative low expression of Jurkat-specific genes and relative high expression of Raji-specific genes indicates specificity of the assay.

In some cases, analysis is performed on one or more images captured to assess the performance of the *in situ* assay, and may comprise processing the image(s) and/or quantifying signals observed. In some embodiments, images of signals from different fluorescent and/or non-fluorescent channels and/or probe hybridization cycles can be compared and analyzed. In some embodiments, images of signals (or absence thereof) at a particular location in a sample from different fluorescent channels and/or sequential detectable probe hybridization cycles can be aligned to analyze an analyte at the location. For instance, a particular location in a sample can be tracked and signal spots from sequential hybridization cycles can be analyzed to detect a target polynucleotide sequence (e.g., an associated barcode sequence or complement thereof) at the location. The analysis may comprise processing information of one or more cell populations (e.g., compared to expected expression of cell populations described in Section II) and various success metrics described herein. In some cases, the analysis includes determining whether particular cells of a cell population and/or signals are present that correlate with one or more expected analytes detected using a panel of probes or probe sets (e.g., as described in Section III). In some embodiments, the analysis includes determining whether particular cells of a cell population and/or signals are present that correlate with one or more expected analytes detected using a panel of probes. In some embodiments, the analysis includes determining whether particular cells of a cell population and/or signals are present that correlate with one or more expected analytes detected using a panel of probe sets. In some instances, the analysis includes using cell segmentation. In some embodiments, the obtained information may be compared to an expected value to determine the success and performance of the *in situ* assay. In some cases, the analysis further includes displaying the information from the assessment. In some embodiments, software may be used to automate the processing, analysis, and/or display of data.

In some cases, analysis is performed on one or more images captured to assess the performance of the *in situ* assay, and may comprise processing the image(s) and/or quantifying signals observed. In some embodiments, images of signals from different fluorescent and/or non-fluorescent channels and/or probe hybridization cycles can be compared and analyzed. In some embodiments, images of signals (or absence thereof) at a particular location in a sample from different fluorescent channels and/or sequential detectable probe hybridization cycles can be aligned to analyze an analyte at the location, wherein a sample comprises a test biological sample comprising a population of Jurkat cells and a population of Raji cells. For instance, a particular location in a sample can be tracked and signal spots from sequential hybridization cycles can be analyzed to detect a target polynucleotide sequence (e.g., an associated barcode sequence or complement thereof) at the location. The analysis may comprise processing information of Jurkat and/or Raji cells (e.g., compared to expected expression of Jurkat and Raji cells described in Section II) and various success metrics described herein. In some cases, the analysis includes determining whether particular cells of a population of Jurkat cells and/or a population of Raji cells and/or signals are present that correlate with one or more expected analytes detected using a panel of probes or probe sets (e.g., as described in Section III). In some embodiments, the analysis includes determining whether particular cells of a population of Jurkat cells and/or a population of Raji cells and/or signals are present that correlate with one or more expected analytes detected using a panel of probes. In some embodiments, the analysis includes determining whether particular cells of a population of Jurkat cells and/or a population of Raji cells and/or signals are present that correlate with one or more expected analytes detected using a panel of probe sets. In some instances, the analysis includes using cell segmentation. In some embodiments, the obtained information may be compared to an expected value to determine the success and performance of the *in situ* assay. In some cases, the analysis further includes displaying the information from the assessment. In some embodiments, software may be used to automate the processing, analysis, and/or display of data.

In any of the embodiments herein, the median number of different genes detected per cell can be at least 5 or 10. In some embodiments, the median number of transcripts detected per cell can be at least 15, at least 20, at least 30, at least 40, at least 60, at least 80, or at least 100. In some embodiments, the median number of transcripts detected per cell is at least 40. In some embodiments, the mean number of transcripts detected per cell can be at least 15, at least 20, at least 30, at least 40, at least 60, at least 80, or at least 100. In some embodiments, the mean expected number of transcripts per cell is at least 15, at least 20, at least 30, at least 40, at least 60, at least 80, or at least 100. In some embodiments, the mean number of transcripts per cell in a given cell population is compared to the expected mean number of transcripts per cell of the cell population to analyze the sensitivity and/or specificity of the assay performance. In some embodiments, the expected mean number of transcripts per cell is the mean expected total number of transcripts based on the selected genes targeted by the probes or probe sets of then probe mixture, and on the cell type of the first and second cell populations. In some embodiments, the median and/or mean number of transcripts detected per cell is/are determined by detecting an anchor sequence common among the probes or probe sets in the probe mixture.

In any of the embodiments herein, a success metric for assaying a test biological sample is that the number of transcripts decoded per 100 µm² is at least 10 or 15. In any of the embodiments herein, a success metric can be that the number of transcripts decoded per 100 µm² is at least 5, at least 10, at least 15, or at least 40. In some embodiments, at least 10 transcripts (e.g., RCPs are decoded to a gene in the set of reference genes) are decoded per 100 µm². In some embodiments, at least 15 transcripts (e.g., RCPs are decoded to a gene in the set of reference genes) are decoded per 100 µm². In some aspects, determining the number of transcripts decoded per 100 µm² comprises dividing the number of high-quality decoded transcripts by the total estimated tissue area to determine a decoded transcript density. In some cases, number of transcripts decoded per 100 µm² provides an estimate of transcript density that is independent of cell segmentation, which is performed algorithmically. In some cases, fewer than 10 transcripts per 100 µm² decoded may indicate a problem with assay performance. In some embodiments, the transcripts decoded are nuclear transcripts.

In any of the embodiments herein, a success metric for assaying a test biological sample is that the number of cells detected is at least 500. In some embodiments, the number of cells detected can be at least 25, at least 50, at least 75, at least 100, at least 200, at least 300, at least 400, or at least 500. In some embodiments, the number of cells detected can include analyzing the total number of detected cells and/or detected number of cells in at least two cell populations to evaluate the assay performance. In some cases, the number of cells detected depends on test biological sample and the size of the region of interest selected in the analysis.

In some embodiments a success metric for assaying a test biological sample is that at least or about 20%, at least or about 25%, at least or about 30%, at least or about 35%, at least or about 40%, at least or about 45%, at least or about 50%, at least or about 55%, at least or about 60%, at least or about 65%, or at least or about 70% of detected RCPs are correctly decoded. In some embodiments at least or about 20%, at least or about 25%, at least or about 30%, at least or about 35%, at least or about 40%, at least or about 45%, at least or about 50%, at least or about 55%, at least or about 60%, at least or about 65%, or at least or about 70% of detected RCPs are decoded with a quality score of at least 20.

In some embodiments, a success metric for assaying a test biological sample is that at least 40% of detected RCPs are decoded with a quality score of at least 20. In some embodiments, at least 29% of detected RCPs are decoded with a quality score of at least 20. In some embodiments, the fraction of objects decoded to gene with Q-score >= 20 is at least 40%. In some embodiments, the fraction of objects decoded to gene with Q-score >= 20 is at least 29%. In some embodiments, at least 10 transcripts (e.g., RCPs correctly decoded to a gene) are decoded per 100 µm² region. In some embodiments, at least 15 transcripts are decoded per 100 µm² region. In some embodiments, a success metric evaluates the total high quality decoded transcripts in the test biological sample.

In some embodiments, a success metric for assaying a test biological sample is that the number of detected RCPs per µm² with a quality score of at least 20 is greater than 0, at least or about 0.0001, at least or about 0.00015, at least or about 0.0002, at least or about 0.00025, at least or about 0.0003, at least or about 0.00035, at least or about 0.0004, at least or about 0.00045, at least or about 0.0005, at least or about 0.00055, at least or about 0.0006, at least or about 0.00065, at least or about 0.0007, at least or about 0.00075, at least or about 0.0008, at least or about 0.00085, at least or about 0.0009, at least or about 0.00095, or at least or about 0.001. In some embodiments, the detected gene density with a quality score of at least 20 is greater than 0, at least 0.0001, at least 0.0002, or at least 0.0003, at least 0.0004, at least 0.0005, at least 0.0006, at least 0.0007, at least 0.0008, at least 0.0009, or at least 0.001.

In some embodiments, a success metric for assaying a test biological sample is that the thickness of detected RCPs with a quality score of at least 20 is less than or about 1.2 µm, less than or about 1.1 µm, less than or about 1.0 µm, less than or about 0.9 µm, less than or about 0.8 µm, less than or about 0.7 µm, less than or about 0.6 µm, less than or about 0.5 µm, or less than or about 0.4 µm.

In some embodiments, a success metric for assaying a test biological sample is that the maximum decoding false positive rate is less than or about 25%, less than or about 20%, less than or about 15%, less than or about 10%, less than or about 5%, or less than about 2%. In some embodiments, the one or more pre-defined criteria can comprise that the maximum decoding false positive rate is less than 15%.

In some embodiments, a success metric for assaying a test biological sample is that the maximum decoding false negative rate is less than or about 95%, less than or about 90%, less than or about 85%, less than or about 80%, less than or about 75%, less than or about 70%, less than or about 65%, less than or about 60%, less than or about 55%, less than or about 50%, less than or about 45%, or less than or about 40%. In some embodiments, the maximum decoding false negative rate is less than 90%.

In some embodiments, a success metric for assaying a test biological sample is that the minimal number of detected RCPs that are decoded with a quality score of at least 20 per field of view (FOV) is greater than 0, at least or about 1, at least or about 100, at least or about 1,000, at least or about 2,000, or at least or about 5,000. In some embodiments, the minimal number of detected RCPs that are decoded (e.g., to a gene in a set of references genes) with a quality score of at least 20 per field of view (FOV) is between about 1 and about 100, between about 100 and bout 1,000, between about 1,000 and about 2,000, or between about 2,000 and about 5,000.

In some embodiments, a success metric for assaying a test biological sample is that the quartile coefficient of dispersion of decoded different identifier sequences (e.g., corresponding to different genes in a set of references genes) with a quality score of at least 20 per field of view (FOV) is less than or about 75%, less than or about 70%, less than or about 65%, less than or about 60%, less than or about 55%, less than or about 50%, less than or about 45%, less than or about 40%, less than or about 35%, less than or about 30%, less than or about 25%, less than or about 20%, or less than or about 15%. In some embodiments, the quartile coefficient of dispersion of decoded genes with a quality score of at least 20 per field of view (FOV) is less than 60%.

In some embodiments, a success metric for assaying a test biological sample is that the percent of transcripts (e.g., RCPs are decoded to a gene in the set of reference genes) that are detected within cells is at least or about 40%, at least or about 45%, at least or about 50%, at least or about 55%, at least or about 60%, at least or about 65%, at least or about 70%, at least or about 75%, at least or about 80%, at least or about 85%, at least or about 90%, or at least or about 95%. In some embodiments, the percent of transcripts that are detected within cells is at least or about 80%. In some embodiments, the percent of transcripts that are detected within cells is at least or about 95%.

In any of the embodiments herein, the method can comprise contacting the test biological sample with a negative control probe or probe set comprising a negative control barcode sequence, and detecting the presence or absence of the negative control probe or probe set or a product thereof in the test biological sample. In any of the embodiments herein, the detecting the presence or absence of the negative control probe or probe set can comprise detecting the presence or absence of the negative control barcode sequence or a complement thereof in the negative control probe or probe set or a product thereof. In any of the embodiments herein, the method can comprise analyzing the detection rate of the negative control probe or probe set or product thereof.

### V. SYSTEMS, COMPOSITIONS AND KITS

In some aspects, provided herein are compositions and kits comprising any of the test biological samples and/or panels of probes or probe sets disclosed herein. The test biological sample can be any described in Section II above and herein. In some embodiments, the kit includes any of the panels of probes or probe sets described in Section III above. In some embodiments, the kit includes instructions for performing any of the methods disclosed herein for analyzing performance of the *in situ* analyte detection assay.

In some embodiments, the kits can contain reagents and/or consumables required for performing one or more steps of the provided methods. In some embodiments, the kits contain reagents for fixing, embedding, and/or permeabilizing the biological sample. In some embodiments, the kits contain reagents, such as enzymes and buffers for ligation and/or amplification, such as ligases and/or polymerases. In some aspects, the kit can also comprise any of the reagents described herein, e.g., wash buffer and ligation buffer. In some embodiments, the kits contain reagents for detection, such as barcode detection probes or detectable labels. In some embodiments, the kits optionally contain other components, for example nucleic acid primers, enzymes and reagents, buffers, nucleotides, photoreactive nucleotides, and reagents for additional assays.

Also provided herein are systems for performing the assessment of the test biological samples using the panels of probes or probe sets disclosed herein, including the contacting of the sample with a probe mixture and the detection of the hybridized probes or probe sets. In some embodiments, the *in situ* assay can be performed using an instrument having integrated optics and fluidics modules (an "opto-fluidic instrument" or "opto-fluidic system"). In an opto-fluidic instrument, the fluidics module is configured to deliver one or more reagents (e.g., fluorescent probes) to the biological sample and/or remove spent reagents therefrom. Additionally, the optics module is configured to illuminate the biological sample with light having one or more spectral emission curves (over a range of wavelengths) and subsequently capture one or more images of emitted light signals from the biological sample during one or more cycles. In various embodiments, the captured images may be processed in real time and/or at a later time to determine the presence of the one or more target molecules in the biological sample, as well as three-dimensional position information associated with each detected target molecule. Additionally, the opto-fluidics instrument includes a sample module configured to receive (and, optionally, secure) one or more biological samples. In some instances, the sample module includes an X-Y stage configured to move the biological sample along an X-Y plane (*e.g.*, perpendicular to an objective lens of the optics module). In some embodiments, the opto-fluidic instrument is configured according to any of the embodiments described in Section VI.

In various embodiments, the opto-fluidic instrument is configured to analyze one or more target molecules in their naturally occurring place (*i.e., in situ*) within the biological sample. For example, an opto-fluidic instrument may be an *in-situ* analysis system used to analyze a biological sample and detect target molecules including but not limited to DNA, RNA, proteins, antibodies, and/or the like.

It is to be noted that, although the above discussion relates to an opto-fluidic instrument that can be used for *in situ* target molecule detection via probe hybridization, the discussion herein equally applies to any opto-fluidic instrument that employs any imaging or target molecule detection technique. That is, for example, an opto-fluidic instrument may include a fluidics module that includes fluids needed for establishing the experimental conditions required for the probing of target molecules in the sample. Further, such an opto-fluidic instrument may also include a sample module configured to receive the sample, and an optics module including an imaging system for illuminating (*e.g.*, exciting one or more fluorescent probes within the sample) and/or imaging light signals received from the probed sample. The *in-situ* analysis system may also include other ancillary modules configured to facilitate the operation of the opto-fluidic instrument, such as, but not limited to, cooling systems, motion calibration systems, etc. In some aspects, the results from an assay performed using an opto-fluidic instrument can be analyzed as described in Section IV for assessment of the assay.

### VI. OPTOFLUIDIC INSTRUMENTS FOR ANALYSIS OF BIOLOGICAL SAMPLES

Provided herein is an instrument having integrated optics and fluidics modules (an "opto-fluidic instrument" or "opto-fluidic system") for detecting target molecules (*e.g.*, nucleic acids, proteins, antibodies, *etc*.) in biological samples (*e.g.,* one or more cells or a tissue sample) as described herein. In an opto-fluidic instrument, the fluidics module is configured to deliver one or more reagents (*e.g.*, panels of probes or probe sets) to the biological sample and/or remove spent reagents therefrom. In some cases, the fluidics module is configured to remove the In some embodiments, the fluidics module is configured to remove non-specifically hybridized probe(s). In some embodiments, the fluidics module is configured to deliver one or more detectably labeled probes and optionally intermediate probes to detect the RCP(s) in the biological sample. In some embodiments, an opto-fluidic instrument is used to evaluate performance of an *in situ* analyte detection assay to ensure that results are generated as expected and producing reliable data. In some embodiments, an opto-fluidic instrument is used to perform an assay on a test biological sample comprising a defined mixture of a first cell population and a second cell population (e.g., as described in Section II) using the probes for detection (as described in Section III).

Additionally, the optics module is configured to illuminate the biological sample with light having one or more spectral emission curves (over a range of wavelengths) and subsequently capture one or more images of emitted light signals from the biological sample during one or more probing cycles (e.g., as described in Section III). In various embodiments, the captured images may be processed in real time and/or at a later time to determine the presence of the one or more target molecules in the biological sample, as well as three-dimensional position information associated with each detected target molecule. Additionally, the opto-fluidics instrument includes a sample module configured to receive (and, optionally, secure) one or more biological samples. In some instances, the sample module includes an X-Y stage configured to move the biological sample along an X-Y plane (*e.g.*, perpendicular to an objective lens of the optics module).

In various embodiments, the opto-fluidic instrument is configured to analyze one or more target RNA transcripts (e.g., as described in Section III) in their naturally occurring place (*i.e., in situ*) within the biological sample. In some embodiments, the opto-fluidic instrument is configured to analyze one or more target RNA transcripts (e.g., as described in Section III) in relative spatial locations within the biological sample. For example, an opto-fluidic instrument may be an *in-situ* analysis system used to analyze a biological sample and detect target molecules including but not limited to DNA, RNA, proteins, antibodies, and/or the like. In some embodiments, the *in situ* analysis system is used to detect one or more target RNAs using target-primed RCA according to the methods disclosed herein.

It is to be noted that, although the above discussion relates to an opto-fluidic instrument that can be used for *in situ* target molecule detection using target-primed RCA according to the methods disclosed herein, the discussion herein equally applies to any opto-fluidic instrument that employs any imaging or target molecule detection technique. That is, for example, an opto-fluidic instrument may include a fluidics module that includes fluids needed for establishing the experimental conditions required for the probing of target molecules in the sample. Further, such an opto-fluidic instrument may also include a sample module configured to receive the sample, and an optics module including an imaging system for illuminating (e.g., exciting one or more fluorescent probes within the sample) and/or imaging light signals received from the probed sample. The *in-situ* analysis system may also include other ancillary modules configured to facilitate the operation of the opto-fluidic instrument, such as, but not limited to, cooling systems, motion calibration systems, etc.

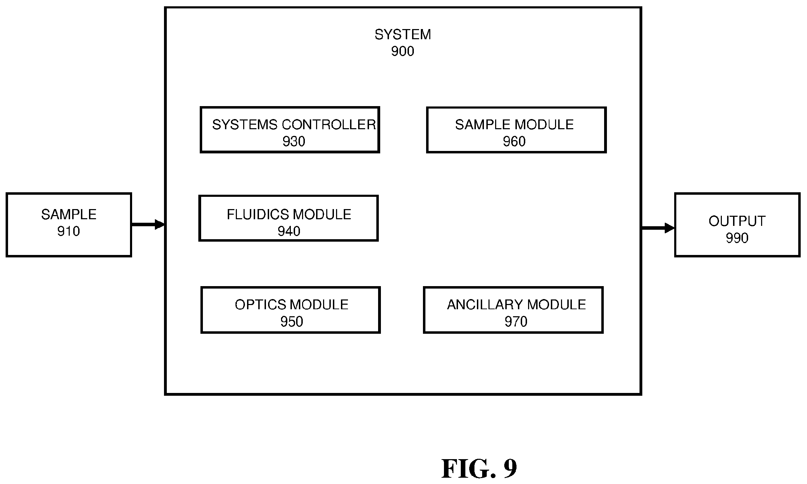

In various embodiments, the sample can be a biological sample (e.g., a tissue) that includes molecules such as DNA, RNA, proteins, antibodies, etc. For example, the sample can be a sectioned **FIG. 9** shows an example workflow of analysis of a biological sample 910 (*e.g.,* cell or tissue sample) using an opto-fluidic instrument 920, according to various embodiments. In various embodiments, the sample 910 can be a biological sample (e.g., a tissue) that includes molecules such as DNA, RNA, proteins, antibodies, etc. For example, the sample 910 can be a sectioned tissue that is treated to access the RNA thereof for labeling with probes described herein (e.g., in Section III). Ligation of a circularizable probe or probe set may generate a circular probe which can be enzymatically amplified and bound with detectably labeled probes, which can create bright signal that is convenient to image and has a high signal-to-noise ratio.

In various embodiments, the sample 910 may be placed in the opto-fluidic instrument 920 for analysis and detection of the molecules in the sample 910. In various embodiments, the opto-fluidic instrument 920 can be a system configured to facilitate the experimental conditions conducive for the detection of the target molecules. For example, the opto-fluidic instrument 920 can include a fluidics module 940, an optics module 950, a sample module 960, and an ancillary module 970, and these modules may be operated by a system controller 930 to create the experimental conditions for the probing of the molecules in the sample 910 by selected probes (e.g., circularizable DNA probes), as well as to facilitate the imaging of the probed sample (e.g., by an imaging system of the optics module 950). In various embodiments, the various modules of the opto-fluidic instrument 920 may be separate components in communication with each other, or at least some of them may be integrated together.

In various embodiments, the sample module 960 may be configured to receive the sample 910 into the opto-fluidic instrument 920. For instance, the sample module 960 may include a sample interface module (SIM) that is configured to receive a sample device (e.g., cassette) onto which the sample 910 can be deposited. That is, the sample 910 may be placed in the opto-fluidic instrument 920 by depositing the sample 910 (e.g., the sectioned tissue) on a sample device that is then inserted into the SIM of the sample module 960. In some instances, the sample module 960 may also include an X-Y stage onto which the SIM is mounted. The X-Y stage may be configured to move the SIM mounted thereon (e.g., and as such the sample device containing the sample 910 inserted therein) in perpendicular directions along the two-dimensional (2D) plane of the opto-fluidic instrument 920.

The experimental conditions that are conducive for the detection of the molecules in the sample 910 may depend on the target molecule detection technique that is employed by the opto-fluidic instrument 920. For example, in various embodiments, the opto-fluidic instrument 920 can be a system that is configured to detect molecules in the sample 910 via hybridization of probes. In such cases, the experimental conditions can include molecule hybridization conditions that result in the intensity of hybridization of the target molecule (e.g., nucleic acid) to a probe (e.g., oligonucleotide) being significantly higher when the probe sequence is complementary to the target molecule than when there is a single-base mismatch. The hybridization conditions include the preparation of the sample 910 using reagents such as washing/stripping reagents, hybridizing reagents, etc., and such reagents may be provided by the fluidics module 940.

In various embodiments, the fluidics module 940 may include one or more components that may be used for storing the reagents, as well as for transporting said reagents to and from the sample device containing the sample 910. For example, the fluidics module 940 may include reservoirs configured to store the reagents, as well as a waste container configured for collecting the reagents (e.g., and other waste) after use by the opto-fluidic instrument 920 to analyze and detect the molecules of the sample 910. Further, the fluidics module 940 may also include pumps, tubes, pipettes, etc., that are configured to facilitate the transport of the reagent to the sample device (e.g., and as such the sample 910). For instance, the fluidics module 940 may include pumps ("reagent pumps") that are configured to pump washing/stripping reagents to the sample device for use in washing/stripping the sample 910 (e.g., as well as other washing functions such as washing an objective lens of the imaging system of the optics module 950).

In various embodiments, the ancillary module 970 can be a cooling system of the opto-fluidic instrument 920, and the cooling system may include a network of coolant-carrying tubes that are configured to transport coolants to various modules of the opto-fluidic instrument 920 for regulating the temperatures thereof. In such cases, the fluidics module 940 may include coolant reservoirs for storing the coolants and pumps (e.g., "coolant pumps") for generating a pressure differential, thereby forcing the coolants to flow from the reservoirs to the various modules of the opto-fluidic instrument 920 via the coolant-carrying tubes. In some instances, the fluidics module 940 may include returning coolant reservoirs that may be configured to receive and store returning coolants, e.g., heated coolants flowing back into the returning coolant reservoirs after absorbing heat discharged by the various modules of the opto-fluidic instrument 920. In such cases, the fluidics module 940 may also include cooling fans that are configured to force air (e.g., cool and/or ambient air) into the returning coolant reservoirs to cool the heated coolants stored therein. In some instance, the fluidics module 940 may also include cooling fans that are configured to force air directly into a component of the opto-fluidic instrument 920 so as to cool said component. For example, the fluidics module 940 may include cooling fans that are configured to direct cool or ambient air into the system controller 930 to cool the same.

As discussed above, the opto-fluidic instrument 920 may include an optics module 950 which include the various optical components of the opto-fluidic instrument 920, such as but not limited to a camera, an illumination module (e.g., LEDs), an objective lens, and/or the like. The optics module 950 may include a fluorescence imaging system that is configured to image the fluorescence emitted by the probes (e.g., oligonucleotides) in the sample 910 after the probes are excited by light from the illumination module of the optics module 950.

In some instances, the optics module 950 may also include an optical frame onto which the camera, the illumination module, and/or the X-Y stage of the sample module 960 may be mounted.

In various embodiments, the system controller 930 may be configured to control the operations of the opto-fluidic instrument 920 (e.g., and the operations of one or more modules thereof). In some instances, the system controller 930 may take various forms, including a processor, a single computer (or computer system), or multiple computers in communication with each other. In various embodiments, the system controller 930 may be communicatively coupled with data storage, set of input devices, display system, or a combination thereof. In some cases, some or all of these components may be considered to be part of or otherwise integrated with the system controller 930, may be separate components in communication with each other, or may be integrated together. In other examples, the system controller 930 can be, or may be in communication with, a cloud computing platform.

In various embodiments, the opto-fluidic instrument 920 may analyze the sample 910 and may generate the output 990 that includes indications of the presence of the target molecules in the sample 910. For instance, with respect to the example embodiment discussed above where the opto-fluidic instrument 920 employs a hybridization technique for detecting molecules, the opto-fluidic instrument 920 may cause the sample 910 to undergo successive rounds of detectably labeled probe hybridization (e.g., using two or more sets of fluorescent probes, where each set of fluorescent probes is excited by a different color channel) and be imaged to detect target molecules in the probed sample 910. In such cases, the output 990 may include optical signatures (e.g., a codeword) specific to each gene, which allow the identification of the target molecules.

### VII. TERMINOLOGY

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

The terms "polynucleotide," and "nucleic acid molecule", used interchangeably herein, refer to polymeric forms of nucleotides of any length, either ribonucleotides or deoxyribonucleotides. Thus, this term comprises, but is not limited to, single-, double-, or multi- stranded DNA or RNA, genomic DNA, cDNA, DNA-RNA hybrids, or a polymer comprising purine and pyrimidine bases or other natural, chemically or biochemically modified, non-natural, or derivatized nucleotide bases. The backbone of the polynucleotide can comprise sugars and phosphate groups (as may typically be found in RNA or DNA), or modified or substituted sugar or phosphate groups.

"Hybridization" as used herein may refer to the process in which two single-stranded polynucleotides bind non-covalently to form a stable double-stranded polynucleotide. In one aspect, the resulting double-stranded polynucleotide can be a "hybrid" or "duplex." "Hybridization conditions" typically include salt concentrations of approximately less than 1 M, often less than about 500 mM and may be less than about 200 mM. A "hybridization buffer" includes a buffered salt solution such as 5% SSPE, or other such buffers known in the art. Hybridization temperatures can be as low as 5°C, but are typically greater than 22°C, and more typically greater than about 30°C, and typically in excess of 37°C. Hybridizations are often performed under stringent conditions, e.g., conditions under which a sequence will hybridize to its target sequence but will not hybridize to other, non-complementary sequences. Stringent conditions are sequence-dependent and are different in different circumstances. For example, longer fragments may require higher hybridization temperatures for specific hybridization than short fragments. As other factors may affect the stringency of hybridization, including base composition and length of the complementary strands, presence of organic solvents, and the extent of base mismatching, the combination of parameters is more important than the absolute measure of any one parameter alone. Generally stringent conditions are selected to be about 5°C lower than the T*ₘ* for the specific sequence at a defined ionic strength and pH. The melting temperature T*ₘ* can be the temperature at which a population of double-stranded nucleic acid molecules becomes half dissociated into single strands. Several equations for calculating the T*ₘ* of nucleic acids are well known in the art. As indicated by standard references, a simple estimate of the T*ₘ* value may be calculated by the equation, T*ₘ* =81.5 + 0.41 (% G + C), when a nucleic acid is in aqueous solution at 1 M NaCl (see e.g., Anderson and Young, Quantitative Filter Hybridization, in Nucleic Acid Hybridization (1985)). Other references (*e.g.,* Allawi and SantaLucia, Jr., Biochemistry, 36:10581-94 (1997)) include alternative methods of computation which take structural and environmental, as well as sequence characteristics into account for the calculation of T*ₘ*.

In general, the stability of a hybrid is a function of the ion concentration and temperature. Typically, a hybridization reaction is performed under conditions of lower stringency, followed by washes of varying, but higher, stringency. Exemplary stringent conditions include a salt concentration of at least 0.01 M to no more than 1 M sodium ion concentration (or other salt) at a pH of about 7.0 to about 8.3 and a temperature of at least 25°C. For example, conditions of 5 × SSPE (750 mM NaCl, 50 mM sodium phosphate, 5 mM EDTA at pH 7.4) and a temperature of approximately 30°C are suitable for allele-specific hybridizations, though a suitable temperature depends on the length and/or GC content of the region hybridized. In one aspect, "stringency of hybridization" in determining percentage mismatch can be as follows: 1) high stringency: 0.1 × SSPE, 0.1% SDS, 65°C; 2) medium stringency: 0.2 × SSPE, 0.1% SDS, 50°C (also referred to as moderate stringency); and 3) low stringency: 1.0 × SSPE, 0.1% SDS, 50°C. It is understood that equivalent stringencies may be achieved using alternative buffers, salts and temperatures. For example, moderately stringent hybridization can refer to conditions that permit a nucleic acid molecule such as a probe to bind a complementary nucleic acid molecule. The hybridized nucleic acid molecules generally have at least 60% identity, including for example at least any of 70%, 75%, 80%, 85%, 90%, or 95% identity. Moderately stringent conditions can be conditions equivalent to hybridization in 50% formamide, 5 × Denhardt's solution, 5x SSPE, 0.2% SDS at 42°C, followed by washing in 0.2 × SSPE, 0.2% SDS, at 42°C. High stringency conditions can be provided, for example, by hybridization in 50% formamide, 5 × Denhardt's solution, 5 × SSPE, 0.2% SDS at 42°C, followed by washing in 0.1 × SSPE, and 0.1% SDS at 65°C. Low stringency hybridization can refer to conditions equivalent to hybridization in 10% formamide, 5 × Denhardt's solution, 6 × SSPE, 0.2% SDS at 22°C, followed by washing in 1x SSPE, 0.2% SDS, at 37°C. Denhardt's solution contains 1% Ficoll, 1% polyvinylpyrolidone, and 1% bovine serum albumin (BSA). 20 × SSPE (sodium chloride, sodium phosphate, ethylene diamide tetraacetic acid (EDTA)) contains 3M sodium chloride, 0.2M sodium phosphate, and 0.025 M EDTA. Other suitable moderate stringency and high stringency hybridization buffers and conditions are well known to those of skill in the art and are described, for example, in Sambrook et al., Molecular Cloning: A Laboratory Manual, 2nd ed., Cold Spring Harbor Press, Plainview, N.Y. (1989); and Ausubel et al., Short Protocols in Molecular Biology, 4th ed., John Wiley & Sons (1999).

Alternatively, substantial complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. *See* M. Kanehisa, Nucleic Acids Res. 12:203 (1984).

A "primer" used herein can be an oligonucleotide, either natural or synthetic, that is capable, upon forming a duplex with a polynucleotide template, of acting as a point of initiation of nucleic acid synthesis and being extended from its 3' end along the template so that an extended duplex is formed. The sequence of nucleotides added during the extension process is determined by the sequence of the template polynucleotide. Primers usually are extended by a DNA polymerase. "Ligation" may refer to the formation of a covalent bond or linkage between the termini of two or more nucleic acids, e.g., oligonucleotides and/or polynucleotides, in a template-driven reaction. The nature of the bond or linkage may vary widely and the ligation may be carried out enzymatically or chemically. As used herein, ligations are usually carried out enzymatically to form a phosphodiester linkage between a 5' carbon terminal nucleotide of one oligonucleotide with a 3' carbon of another nucleotide.

"Sequencing," "sequence determination" and the like means determination of information relating to the nucleotide base sequence of a nucleic acid. Such information may include the identification or determination of partial as well as full sequence information of the nucleic acid. Sequence information may be determined with varying degrees of statistical reliability or confidence. In one aspect, the term includes the determination of the identity and ordering of a plurality of contiguous nucleotides in a nucleic acid. "High throughput digital sequencing" or "next generation sequencing" means sequence determination using methods that determine many (typically thousands to billions) of nucleic acid sequences in an intrinsically parallel manner, i.e., where DNA templates are prepared for sequencing not one at a time, but in a bulk process, and where many sequences are read out preferably in parallel, or alternatively using an ultra-high throughput serial process that itself may be parallelized. Such methods include but are not limited to pyrosequencing (for example, as commercialized by 454 Life Sciences, Inc., Branford, Conn.); sequencing by ligation (for example, as commercialized in the SOLiD^{™} technology, Life Technologies, Inc., Carlsbad, Calif.); sequencing by synthesis using modified nucleotides (such as commercialized in TruSeq^{™} and HiSeq^{™} technology by Illumina, Inc., San Diego, Calif.; HeliScope^{™} by Helicos Biosciences Corporation, Cambridge, Ma.; and PacBio RS by Pacific Biosciences of California, Inc., Menlo Park, Calif.), sequencing by ion detection technologies (such as Ion Torrent^{™} technology, Life Technologies, Carlsbad, Calif.); sequencing of DNA nanoballs (Complete Genomics, Inc., Mountain View, Calif.); nanopore-based sequencing technologies (for example, as developed by Oxford Nanopore Technologies, LTD, Oxford, UK), and like highly parallelized sequencing methods."Multiplexing" or "multiplex assay" herein may refer to an assay or other analytical method in which the presence and/or amount of multiple targets, e.g., multiple nucleic acid target sequences, can be assayed simultaneously by using more than one capture probe conjugate, each of which has at least one different detection characteristic, e.g., fluorescence characteristic (for example excitation wavelength, emission wavelength, emission intensity, FWHM (full width at half maximum peak height), or fluorescence lifetime) or a unique nucleic acid or protein sequence characteristic.

The term "about" as used herein refers to the usual error range for the respective value readily known to the skilled person in this technical field. Reference to "about" a value or parameter herein comprises (and describes) embodiments that are directed to that value or parameter per se.

As used herein, the singular forms "a," "an," and "the" comprise plural referents unless the context clearly dictates otherwise. For example, "a" or "an" means "at least one" or "one or more."

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be comprised in the smaller ranges, and are also encompassed within the claimed subject matter, subject to any specifically excluded limit in the stated range. Where the stated range comprises one or both of the limits, ranges excluding either or both of those comprised limits are also comprised in the claimed subject matter. This applies regardless of the breadth of the range.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, use of a), b), etc., or i), ii), etc. does not by itself connote any priority, precedence, or order of steps in the claims. Similarly, the use of these terms in the specification does not by itself connote any required priority, precedence, or order.

### EXAMPLES

The following examples are included for illustrative purposes only and are not intended to limit the scope of the present disclosure.

In some aspects, *in situ* analyte detection assays can involve complex equipment, reagents, and procedures, and may be prone to human and/or technical error, for example when the user does not have extensive experience with the assay. In some aspects, performing an *in situ* analyte detection assay on a test biological sample with known cell types can allow a user to confidently assess whether the *in situ* analyte detection assay performs as expected. These examples disclose exemplary methods for preparing a test biological sample with known cell types, and for identifying the cell types in the test biological sample in an *in situ* analyte detection assay using probes for cell type-specific genes.

### Example 1: Preparation of a test biological sample comprising a mixed population of known cell types

This example discloses exemplary methods for preparing a test biological sample for use in an assay performance test for an *in situ* analyte detection assay. In some aspects, the test biological sample is a fixed cell block comprising a mixture of two known cell types that express different sets of genes at high levels. In some aspects, performing an *in situ* analyte detection assay on a test biological sample with known cell types can allow a user to confidently assess whether the *in situ* analyte detection assay is performing as expected (e.g., assay performance is satisfactory).

In particular, this example describes a method of preparing a fixed cell block comprising a mixture of cells from Jurkat and Raji cell lines (e.g. as shown in FIG. 2, right panel). The test biological sample can be used to assess *in situ* analyte detection assay performance, for example as described herein and as exemplified in Example 2.

Frozen samples of the non-adherent immune cell lines Jurkat (ATCC Cat#: TIB-152) and Raji (ATCC Cat#: CCL-86) were separately thawed and resuspended in fresh media (RPMI-1640 (ATCC Cat#: 30-2001) supplemented with 10% fetal bovine serum (ATCC Cat#: 30-2021)) at 2x10^5 cells per mL for culture and expansion under standard conditions. Cells were incubated at 37° C in 5% CO2, and counted 2-3 times per week to monitor proliferation. Cells were regularly split and resuspended at 2x10^5 cells per mL until a sufficient number of cells was reached. A density of 2x10^6 cells per mL was not exceeded during the process, and cells exceeding 25 passages or 6 weeks in culture were not used.

To harvest the cells, cell suspensions were centrifuged at 300x g for 5 minutes at 23° C, resuspended in 10 mL PBS, and filtered by passing through a 40 µm nylon cell filter (Corning Cat#: 431750). The cells were counted, and cells from the two cell lines were mixed at a 1:1 ratio. The cell mixture was fixed by resuspending in 10% neutral buffered formalin and incubating on a rotator at room temperature for 1 hour.

The fixed cells were again centrifuged, resuspended in PBS, and filtered. Next, the cells were centrifuged and resuspended in 95% ethanol. The cells were then aliquoted, centrifuged, and resuspended in liquefied Histogel (Epredia Cat#: HG-400-012; incubated in 65° C water bath for approximately 1 hour to liquify) at a density of 7x10^7 total cells per 500µL, then vortexed for 10 seconds, and visible remaining aggregates were removed. The Histogel suspensions were each transferred to a 2 mL round-bottom Eppendorf tube (Eppendorf Cat#: 022431048) with a pre-aliquoted and solidified Histogel "bed" to produce a homogenous pellet with a consistent diameter and that was flat on both sides. The tubes were placed on ice for 5 minutes to solidify. Pellets were removed from the tubes and incubated in 10% neutral buffered formalin (ThermoFisher Cat#: 5735) for 2 hours. Pellets were rinsed twice in PBS and stored in 75% ethanol for 13 hours before further processing.

Pellets were then serially dehydrated by sequentially incubating in the solutions shown in **Table E1.**

**Table E1: solutions for serial dehydration**

| **Solution #** | **Contents** | **Time** | **Temperature** |
|---|---|---|---|
| 1 | 95% ethanol | 60 min | Ambient |
| 2 | 95% ethanol | 60 min | Ambient |
| 3 | 100% ethanol | 60 min | Ambient |
| 4 | 100% ethanol | 60 min | Ambient |
| 5 | 100% ethanol | 60 min | Ambient |
| 6 | Xylene | 45 min | Ambient |
| 7 | Xylene | 45 min | Ambient |
| 8 | Xylene | 45 min | Ambient |
| 9 | Paraffin | 60 min | 62C |
| 10 | Paraffin | 60 min | 62C |
| 11 | Paraffin | 60 min | 62C |

Each pellet was embedded in paraffin in a square mold and then transferred to a cold plate for 15 minutes to solidify, producing a solidified cell block. Cell blocks were stored at 4° C until further sectioning and analysis, as described below.

### Example 2: Identification of cell types in a test biological sample comprising a mixed population of known cell types

This Example discloses exemplary methods for testing performance of an *in situ* analyte detection assay. In some aspects, *in situ* analyte detection assays can involve complex equipment, reagents, and procedures, and may be prone to human and/or technical error, for example when the user is not experienced in the assay. In some aspects, performing an *in situ* analyte detection assay on a test biological sample with known cell types can allow a user to confidently assess whether the *in situ* analyte detection assay performs as expected.

In particular, this Example discloses exemplary methods for identifying cell types in a test biological sample comprising a mixed population of two known cell types using an *in situ* analyte detection assay with probes specific for cell-type-specific genes (e.g., genes expressed at high levels in one cell type and not the other).

Cell blocks comprising a mixed population of Jurkat and Raji cells (as described in Example 1) were prepared and sectioned onto slides for analysis.

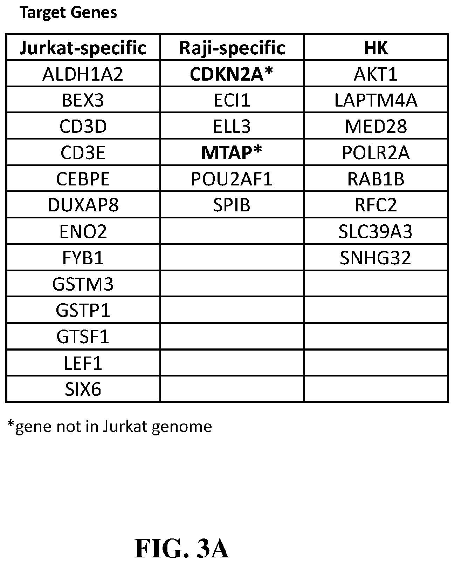

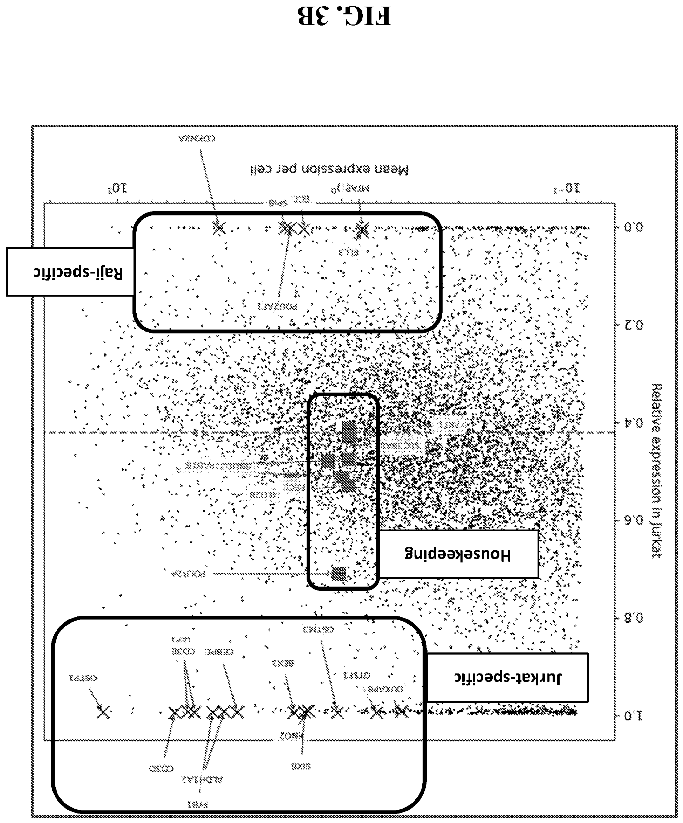

Genes with Jurkat cell- and Raji cell-specific expression patterns were identified based on relative expression data from Jurkat and Raji cells, as shown in **FIGS. 3A-****3B.** Genes were selected that exhibited a greater than 100-fold difference in expression between the two cell types. 13 Jurkat-specific and 6 Raji-specific genes were selected for use in the assay. 2 of the Raji-specific genes (CDKN2A and MTAP) are not present in the Jurkat genome. 8 "housekeeping" (HK) genes (i.e., commonly expressed genes) were selected as controls. In total, 27 target genes were selected for use in the assay.

A set of circularizable padlock probes targeting transcripts for each of the target genes and comprising corresponding analyte-specific barcodes were added to the sample and allowed to hybridize overnight at 50 °C. Following probe hybridization, samples were washed for 30 minutes at 37 °C to remove unbound probes. Hybridized padlock probes were ligated to form circularized templates for rolling circle amplification (RCA). Primers for RCA were added to the sample and allowed to hybridize to the circularized padlock probes. RCA was performed by adding RCA reaction mixture containing polymerase, reaction buffer, and dNTPs, and incubating the sample for polymerization. The RCA reaction was then terminated.

Next, sequential hybridization and detection cycles were performed to identify RCA products (RCPs) corresponding to each transcript. Briefly, in each cycle, intermediate probes were added to the sample, each comprising (i) a region hybridizing to a target sequence in an RCP barcode, and (ii) an overhang region hybridizing to a fluorescently labeled detection probe. Fluorescent signals corresponding to specific sequences within barcodes were detected and recorded at specific locations in the sample. The intermediate probes and detection probes were stripped from the sample before starting each additional cycle. The detection of a sequence of fluorescent signals at a given location through multiple cycles was indicative of the presence of a specific barcode, thereby revealing the presence of a specific transcript corresponding to the barcode sequence of the probe that hybridizes to that transcript.

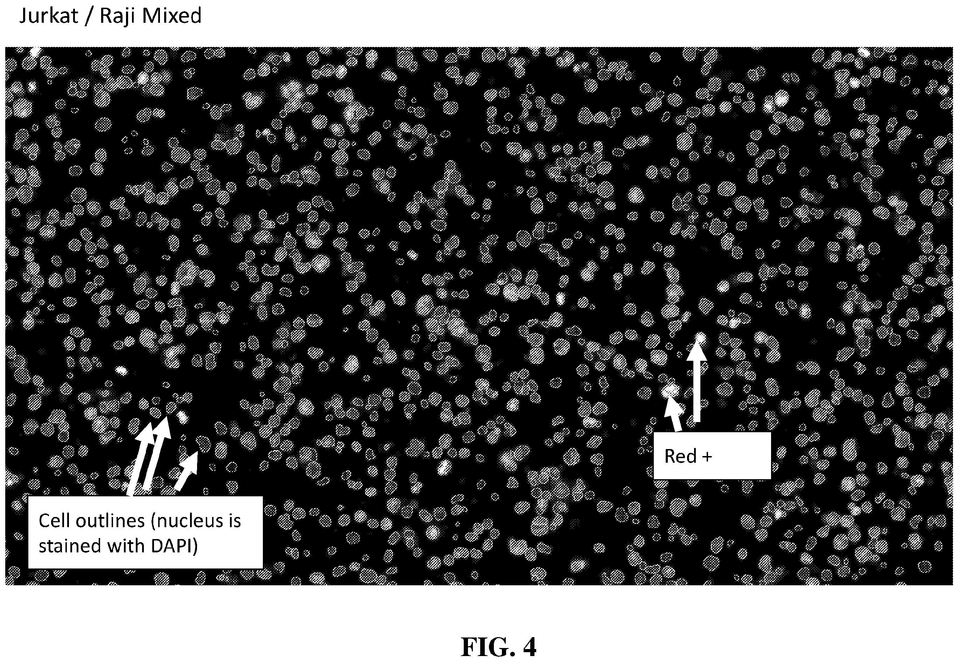

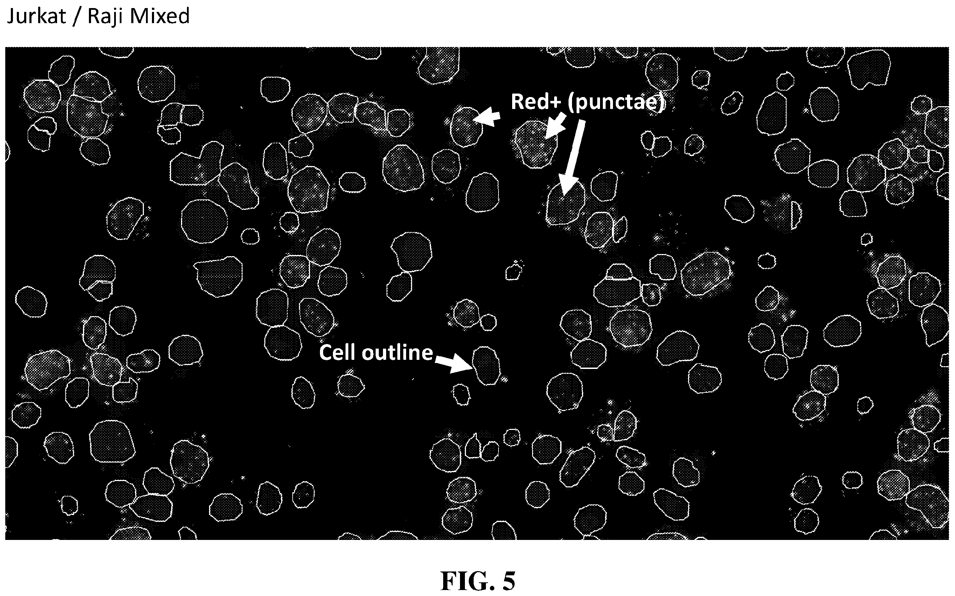

Individually detected transcripts were each assigned to a nucleus in close proximity to the transcript, using DAPI staining for detection of nuclei. Gene expression for each cell corresponding to a nucleus was characterized based on all the transcripts assigned to that nucleus. **FIG. 4** shows a section from a cell block containing a mixed population of Jurkat and Raji cells stained with DAPI to reveal cell nuclei. The nuclei were segmented (outlines around nuclei) to identify individual nuclei. **FIG. 5** shows an exemplary imaged section from a single detection cycle using a detection oligonucleotide with fluorescence in the red channel. As expected for signals corresponding to a cell-type specific gene, two cell populations were easily discerned: a first population with a high frequency of signals per cell, and a second population with a low frequency of signals per cell.

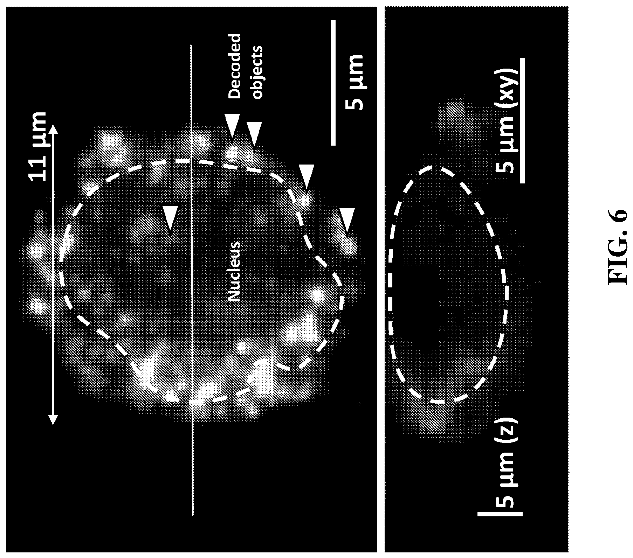

**FIG. 6** shows the spatial relationship of decoded objects (i.e. RCPs corresponding to specific transcripts) to a proximal nucleus visualized by DAPI staining. A single nucleus is shown from two different perspectives, a top and side view (top panel and bottom panel). The majority of decoded objects in the image are located in or within approximately 2 µm of the nucleus.

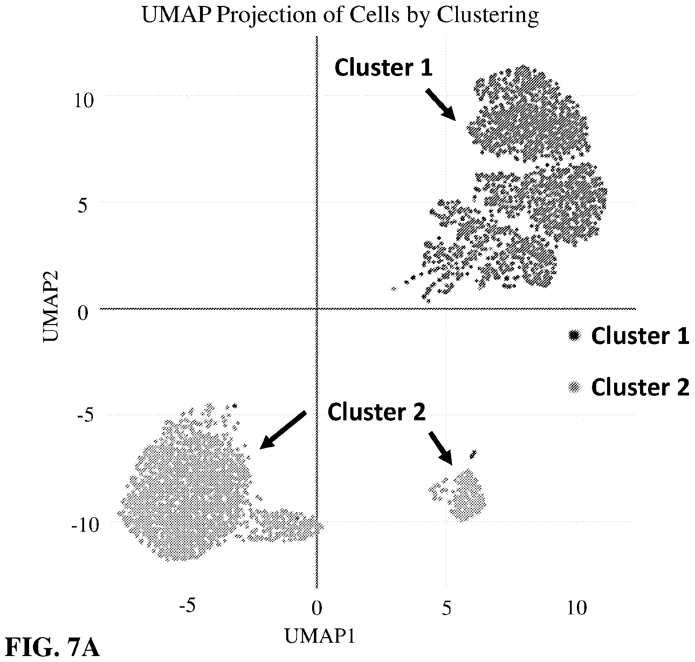

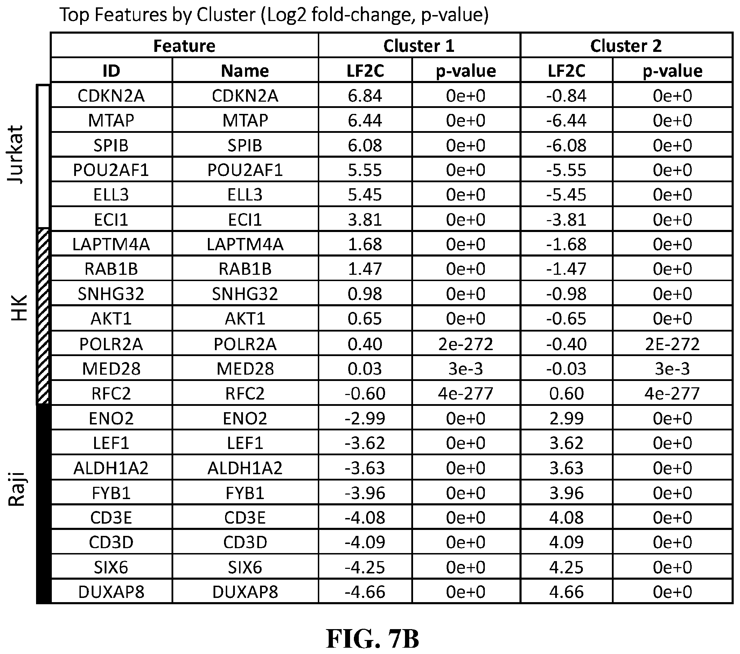

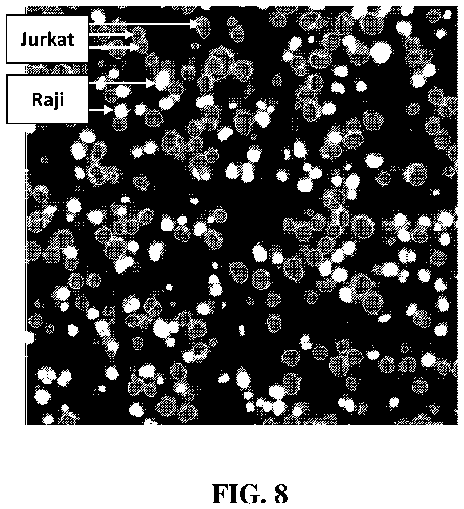

After gene expression profiles were determined for individual cells, secondary analysis was performed to assess the quality and specificity of the assay. Unsupervised clustering analysis and visualization of clusters using UMAP projection revealed distinct clusters of cells based on gene expression, as shown in **FIGS. 7A-7B****.** k-means clustering was used to assign all cells into two distinct clusters. The first cluster (shown as cluster 1) was defined by relative high expression of Jurkat-specific genes and relative low expression of Raji-specific genes, and the second cluster (shown as cluster 2) was defined by relative high expression of Raji-specific specific genes and relative low expression of Jurkat-specific genes. Mapping cluster identity to individual nuclei in the imaged biological sample revealed an intermixed population of clearly defined Jurkat and Raji cells, as shown in **FIG. 8****.**

Together, these data demonstrate an exemplary robust workflow for probe-based detection of cell types based on mutually exclusive gene expression patterns in a mixed population of defined cell types. In addition, various metrics such as a quality score for barcode decoding, median number of different genes detected per cell, mean number of different genes detected per cell, median number of transcripts detected per cell, mean number of transcripts detected per cell, RNA transcript density, and/or percent of detected transcripts within identified cells can be used to determine an assay performance as pass (e.g., satisfactory performance) or fail (e.g., unsatisfactory performance). The workflow can be employed by a user to confidently assess performance of *in situ* analyte detection assays.

## Claims

1. A method, comprising:
(a) providing a test biological sample, wherein the test biological sample is a section of a cell pellet comprising a defined mixture of a first cell population and a second cell population;
(b) contacting the test sample with a probe mixture comprising:
(i) a first panel of probes or probe sets, wherein each probe or probe set of the first panel comprises a recognition sequence complementary to a target sequence of an RNA transcript or product thereof of a first set of genes expressed in the first cell population, and
(ii) a second panel of probes or probe sets, wherein each probe or probe set of the second panel comprises a recognition sequence complementary to a target sequence of an RNA transcript or product thereof of a second set of genes expressed in the second cell population, and
allowing the probes or probe sets to hybridize to their target sequences in the test biological sample, wherein the first cell population does not detectably express the second set of genes, and the second cell population does not detectably express the first set of genes;
(c) detecting the hybridized probes or probe sets or products of the hybridized probes or probe sets at locations in the biological sample, thereby detecting RNA transcripts of the first and second sets of genes in the test biological sample; and
(d) identifying discrete cells in the test biological sample and comparing the detected RNA transcripts of the first and second sets of genes in the cells to expected expression levels of the first and second sets of genes in the first and second cell populations.

2. The method of claim 1, wherein the probe mixture comprises: (iii) a third panel of probes or probe sets, wherein each probe or probe set of the third panel comprises a recognition sequence complementary to a target sequence of an RNA transcript or product thereof of a third set of genes expressed in both the first cell population and the second cell population, and
wherein the method comprises allowing the probes or probe sets of the third panel to hybridize to their target sequences in the test biological sample,
detecting the hybridized probes or probe sets or products of the hybridized probes or probe sets of the third panel at locations in the biological sample, thereby detecting RNA transcripts of the third set of genes in the test biological sample; and
comparing the detected RNA transcripts of the third set of genes in the cells to expected expression levels of the third set of genes in the first and second cell populations.

3. The method of claim 1 or 2, wherein the first, and/or second sets of genes comprise genes having at least two different expression levels.

4. The method of any of claims 1-3, wherein the first panel of probes or probe sets and the second panel of probes or probe sets each is a panel of circularizable probes or probe sets, and the method comprises ligating the circularizable probes or probe sets hybridized to their target sequences, thereby generating circularized probes at locations in the test biological sample.

5. The method of any of claims 1-4, wherein the first panel of probes or probe sets and the second panel of probes or probe sets each comprise barcode sequences corresponding to the RNA transcripts comprising their corresponding target sequences.

6. The method of claim 4 or claim 5, wherein the method comprises performing rolling circle amplification of the circularized probes to generate rolling circle amplification products at locations in the test biological sample.

7. The method of claim 5, wherein detecting the hybridized probes or probe sets or products of the hybridized probes or probe sets comprises detecting the barcode sequences in the hybridized probes or probe sets.

8. The method of claim 6, wherein detecting the hybridized probes or probe sets or products of the hybridized probes or probe sets comprises detecting the rolling circle amplification products, optionally wherein detecting the rolling circle amplification products comprises detecting the complements of the barcode sequences.

9. The method of claim 7 or 8, wherein detecting the barcode sequences or complements thereof comprises (i) sequencing all or a portion of the barcode sequences or complements thereof, or (ii) sequential binding of detectably labeled probes directly or indirectly to the barcode sequences or complements thereof.

10. The method of claim 9, wherein the method comprises determining a quality score for the sequenced or decoded barcode sequence or complement thereof.

11. The method of any of claims 1-10, wherein identifying the discrete cells comprises performing cell segmentation, optionally wherein the cell segmentation comprises (i) detecting a nuclear stain; and/or (ii) detecting a membrane stain.

12. The method of any of claims 1-11, wherein:
(a) the first cell population is a first cultured cell line, and the second cell population is a second cultured cell line; and/or
(b) the first cell population is a T cell line, and the second population is a B cell line.

13. The method of any one of claims 1-12, wherein the first cell population are Jurkat cells, and the second cell population are Raji cells.

14. The method of any of claims 1-13, wherein the method comprises embedding the cell pellet in an embedding medium and sectioning the embedded cell pellet to provide the test biological sample.

15. The method of any of claims 11-14, wherein the detected RNA transcripts are assigned to the identified discrete cells, optionally wherein the detected RNA transcripts are assigned to the identified discrete cells for clustering the identified discrete cells.

## Patentansprüche

1. Verfahren, umfassend:
(a) Bereitstellen einer biologischen Testprobe, wobei die biologische Testprobe ein Abschnitt eines Zellpellets ist, das eine definierte Mischung aus einer ersten Zellpopulation und einer zweiten Zellpopulation umfasst;
(b) Kontaktieren der Testprobe mit einer Sondenmischung, umfassend:
(i) ein erstes Panel von Sonden oder Sondensätzen, wobei jede Sonde oder jeder Sondensatz des ersten Panels eine Erkennungssequenz umfasst, die zu einer Zielsequenz eines RNA-Transkripts oder einem Produkt davon eines ersten Satzes von Genen, die in der ersten Zellpopulation exprimiert werden, komplementär ist, und
(ii) ein zweites Panel von Sonden oder Sondensätzen, wobei jede Sonde oder jeder Sondensatz des zweiten Panels eine Erkennungssequenz umfasst, die zu einer Zielsequenz eines RNA-Transkripts oder einem Produkt davon eines zweiten Satzes von Genen, die in der zweiten Zellpopulation exprimiert werden, komplementär ist, und
Ermöglichen, dass die Sonden oder Sondensätze mit ihren Zielsequenzen in der biologischen Testprobe hybridisieren, wobei die erste Zellpopulation den zweiten Satz von Genen nicht nachweisbar exprimiert und die zweite Zellpopulation den ersten Satz von Genen nicht nachweisbar exprimiert;
(c) Nachweisen der hybridisierten Sonden oder Sondensätze oder Produkte der hybridisierten Sonden oder Sondensätze an Stellen in der biologischen Probe, wodurch RNA-Transkripte des ersten und zweiten Satzes von Genen in der biologischen Testprobe nachgewiesen werden; und
(d) Identifizieren diskreter Zellen in der biologischen Testprobe und Vergleichen der nachgewiesenen RNA-Transkripte des ersten und zweiten Satzes von Genen in den Zellen mit erwarteten Expressionsniveaus des ersten und zweiten Satzes von Genen in der ersten und zweiten Zellpopulation.

2. Verfahren nach Anspruch 1, wobei die Sondenmischung umfasst: (iii) ein drittes Panel von Sonden oder Sondensätzen, wobei jede Sonde oder jeder Sondensatz des dritten Panels eine Erkennungssequenz umfasst, die zu einer Zielsequenz eines RNA-Transkripts oder einem Produkt davon eines dritten Satzes von Genen, die sowohl in der ersten Zellpopulation als auch in der zweiten Zellpopulation exprimiert werden, komplementär ist, und
wobei das Verfahren umfasst: Ermöglichen, dass die Sonden oder Sondensätze des dritten Panels mit ihren Zielsequenzen in der biologischen Testprobe hybridisieren,
Nachweisen der hybridisierten Sonden oder Sondensätze oder Produkte der hybridisierten Sonden oder Sondensätze des dritten Panels an Stellen in der biologischen Probe, wodurch RNA-Transkripte des dritten Satzes von Genen in der biologischen Testprobe nachgewiesen werden; und
Vergleichen der nachgewiesenen RNA-Transkripte des dritten Satzes von Genen in den Zellen mit erwarteten Expressionsniveaus des dritten Satzes von Genen in der ersten und zweiten Zellpopulation.

3. Verfahren nach Anspruch 1 oder 2, wobei die ersten und/oder zweiten Sätze von Genen Gene mit mindestens zwei unterschiedlichen Expressionsniveaus umfassen.

4. Verfahren nach einem der Ansprüche 1 bis 3, wobei das erste Panel von Sonden oder Sondensätzen und das zweite Panel von Sonden oder Sondensätzen jeweils ein Panel von zirkularisierbaren Sonden oder Sondensätzen ist und das Verfahren das Ligieren der zirkularisierbaren Sonden oder Sondensätze, die an ihre Zielsequenzen hybridisiert sind, umfasst, wodurch zirkularisierte Sonden an Stellen in der biologischen Testprobe erzeugt werden.

5. Verfahren nach einem der Ansprüche 1 bis 4, wobei das erste Panel von Sonden oder Sondensätzen und das zweite Panel von Sonden oder Sondensätzen jeweils Barcodesequenzen umfassen, die den RNA-Transkripten entsprechen, die ihre entsprechenden Zielsequenzen umfassen.

6. Verfahren nach Anspruch 4 oder Anspruch 5, wobei das Verfahren das Durchführen einer Rolling-Circle-Amplifikation der zirkularisierten Sonden umfasst, um Rolling-Circle-Amplifikationsprodukte an Stellen in der biologischen Testprobe zu erzeugen.

7. Verfahren nach Anspruch 5, wobei das Nachweisen der hybridisierten Sonden oder Sondensätze oder Produkte der hybridisierten Sonden oder Sondensätze das Nachweisen der Barcodesequenzen in den hybridisierten Sonden oder Sondensätzen umfasst.

8. Verfahren nach Anspruch 6, wobei das Nachweisen der hybridisierten Sonden oder Sondensätze oder Produkte der hybridisierten Sonden oder Sondensätze das Nachweisen der Rolling-Circle-Amplifikationsprodukte umfasst, wobei das Nachweisen der Rolling-Circle-Amplifikationsprodukte optional das Nachweisen der Komplemente der Barcodesequenzen umfasst.

9. Verfahren nach Anspruch 7 oder 8, wobei das Nachweisen der Barcodesequenzen oder der Komplemente davon (i) das Sequenzieren aller oder eines Teils der Barcodesequenzen oder der Komplemente davon oder (ii) das sequentielle Binden nachweisbar markierter Sonden direkt oder indirekt an die Barcodesequenzen oder die Komplemente davon umfasst.

10. Verfahren nach Anspruch 9, wobei das Verfahren das Bestimmen eines Qualitätswertes für die sequenzierte oder decodierte Barcodesequenz oder das Komplement davon umfasst.

11. Verfahren nach einem der Ansprüche 1 bis 10, wobei das Identifizieren der diskreten Zellen das Durchführen einer Zellsegmentierung umfasst, wobei die Zellsegmentierung optional umfasst (i) das Nachweisen eines nukleären Farbstoffs; und/oder (ii) das Nachweisen einer Membranfärbung.

12. Verfahren nach einem der Ansprüche 1 bis 11, wobei:
(a) die erste Zellpopulation eine erste kultivierte Zelllinie ist und die zweite Zellpopulation eine zweite kultivierte Zelllinie ist; und/oder
(b) die erste Zellpopulation eine T-Zelllinie ist und die zweite Population eine B-Zelllinie ist.

13. Verfahren nach einem der Ansprüche 1 bis 12, wobei die erste Zellpopulation Jurkat-Zellen und die zweite Zellpopulation Raji-Zellen sind.

14. Verfahren nach einem der Ansprüche 1 bis 13, wobei das Verfahren das Einbetten des Zellpellets in ein Einbettungsmedium und das Schneiden des eingebetteten Zellpellets umfasst, um die biologische Testprobe bereitzustellen.

15. Verfahren nach einem der Ansprüche 11 bis 14, wobei die nachgewiesenen RNA-Transkripte den identifizierten diskreten Zellen zugeordnet werden, optional wobei die nachgewiesenen RNA-Transkripte den identifizierten diskreten Zellen zugeordnet werden, um die identifizierten diskreten Zellen zu clustern.

## Revendications

1. Procédé, comprenant :
(a) la fourniture d'un échantillon biologique d'essai, dans lequel l'échantillon biologique d'essai est une section d'un culot cellulaire comprenant un mélange défini d'une première population cellulaire et d'une deuxième population cellulaire ;
(b) la mise en contact de l'échantillon à tester avec un mélange de sondes comprenant :
(i) un premier panel de sondes ou d'ensembles de sondes, dans lequel chaque sonde ou ensemble de sondes du premier panel comprend une séquence de reconnaissance complémentaire d'une séquence cible d'une transcription d'ARN ou d'un produit de celui-ci d'un premier ensemble de gènes exprimés dans la première population cellulaire, et
(ii) un deuxième panel de sondes ou d'ensembles de sondes, dans lequel chaque sonde ou ensemble de sondes du deuxième panel comprend une séquence de reconnaissance complémentaire d'une séquence cible d'une transcription d'ARN ou d'un produit de celui-ci d'un deuxième ensemble de gènes exprimés dans la deuxième population cellulaire, et
le fait de permettre aux sondes ou aux ensembles de sondes de s'hybrider à leurs séquences cibles dans l'échantillon biologique d'essai, dans lequel la première population cellulaire n'exprime pas de manière détectable le deuxième ensemble de gènes, et la deuxième population cellulaire n'exprime pas de manière détectable le premier ensemble de gènes ;
(c) la détection des sondes hybridées ou des ensembles de sondes, ou des produits des sondes hybridées ou des ensembles de sondes, à des emplacements dans l'échantillon biologique, détectant ainsi des transcriptions d'ARN des premier et deuxième ensembles de gènes dans l'échantillon biologique d'essai ; et
(d) l'identification de cellules discrètes dans l'échantillon biologique d'essai et la comparaison des transcriptions d'ARN détectés des premier et deuxième ensembles de gènes dans les cellules aux niveaux d'expression attendus des premier et deuxième ensembles de gènes dans les première et deuxième populations cellulaires.

2. Procédé selon la revendication 1, dans lequel le mélange de sondes comprend : (iii) un troisième panel de sondes ou d'ensembles de sondes, dans lequel chaque sonde ou ensemble de sondes du troisième panel comprend une séquence de reconnaissance complémentaire d'une séquence cible d'une transcription d'ARN ou d'un produit de celui-ci d'un troisième ensemble de gènes exprimés à la fois dans la première population cellulaire et dans la deuxième population cellulaire, et
dans lequel le procédé comprend l'étape consistant à permettre aux sondes ou aux ensembles de sondes du troisième panel de s'hybrider à leurs séquences cibles dans l'échantillon biologique d'essai,
la détection des sondes hybridées ou des ensembles de sondes, ou des produits des sondes hybridées ou des ensembles de sondes, du troisième panel à des emplacements dans l'échantillon biologique, détectant ainsi des transcriptions d'ARN du troisième ensemble de gènes dans l'échantillon biologique d'essai ; et
la comparaison des transcriptions d'ARN détectées du troisième ensemble de gènes dans les cellules avec les niveaux d'expression attendus du troisième ensemble de gènes dans les première et deuxième populations cellulaires.

3. Procédé selon la revendication 1 ou 2, dans lequel les premier et/ou deuxième ensembles de gènes comprennent des gènes ayant au moins deux niveaux d'expression différents.

4. Procédé selon l'une quelconque des revendications 1 à 3, dans lequel le premier ensemble de sondes ou d'ensembles de sondes et le deuxième ensemble de sondes ou d'ensembles de sondes sont chacun un ensemble de sondes ou d'ensembles de sondes circularisables, et le procédé comprend la ligature des sondes ou des ensembles de sondes circularisables hybridées à leurs séquences cibles, générant ainsi des sondes circularisées à des emplacements dans l'échantillon biologique d'essai.

5. Procédé selon l'une quelconque des revendications 1 à 4, dans lequel le premier ensemble de sondes ou d'ensembles de sondes et le deuxième ensemble de sondes ou d'ensembles de sondes comprennent chacun des séquences de code-barres correspondant aux transcriptions d'ARN comprenant leurs séquences cibles correspondantes.

6. Procédé selon la revendication 4 ou la revendication 5, dans lequel le procédé comprend la réalisation d'une amplification en cercle roulant des sondes circularisées pour générer des produits d'amplification en cercle roulant à des emplacements dans l'échantillon biologique d'essai.

7. Procédé selon la revendication 5, dans lequel la détection des sondes hybridées ou des ensembles de sondes, ou des produits des sondes hybridées ou des ensembles de sondes, comprend la détection des séquences de code-barres dans les sondes hybridées ou les ensembles de sondes.

8. Procédé selon la revendication 6, dans lequel la détection des sondes hybridées ou des ensembles de sondes, ou des produits des sondes hybridées ou des ensembles de sondes, comprend la détection des produits d'amplification en cercle roulant, facultativement dans lequel la détection des produits d'amplification en cercle roulant comprend la détection des compléments des séquences de code-barres.

9. Procédé selon la revendication 7 ou 8, dans lequel la détection des séquences de code-barres ou de leurs compléments comprend (i) le séquençage de tout ou partie des séquences de code-barres ou de leurs compléments, ou (ii) la liaison séquentielle de sondes marquées de manière détectable directement ou indirectement aux séquences de code-barres ou à leurs compléments.

10. Procédé selon la revendication 9, dans lequel le procédé comprend la détermination d'un score de qualité pour la séquence de code-barres séquencée ou décodée, ou son complément.

11. Procédé selon l'une quelconque des revendications 1 à 10, dans lequel l'identification des cellules discrètes comprend la réalisation d'une segmentation cellulaire, facultativement dans lequel la segmentation cellulaire comprend (i) la détection d'un colorant nucléaire ; et/ou (ii) la détection d'une coloration membranaire.

12. Procédé selon l'une quelconque des revendications 1 à 11, dans lequel :
(a) la première population cellulaire est une première lignée cellulaire cultivée, et la deuxième population cellulaire est une deuxième lignée cellulaire cultivée ; et/ou
(b) la première population cellulaire est une lignée de cellules T, et la deuxième population cellulaire est une lignée de cellules B.

13. Procédé selon l'une quelconque des revendications 1 à 12, dans lequel la première population cellulaire est constituée de cellules Jurkat, et la deuxième population cellulaire est constituée de cellules Raji.

14. Procédé selon l'une quelconque des revendications 1 à 13, dans lequel le procédé comprend l'incorporation du culot cellulaire dans un milieu d'incorporation et le sectionnement du culot cellulaire incorporé pour fournir l'échantillon biologique d'essai.

15. Procédé selon l'une quelconque des revendications 11 à 14, dans lequel les transcriptions d'ARN détectées sont affectées aux cellules discrètes identifiées, éventuellement dans lequel les transcriptions d'ARN détectées sont affectées aux cellules discrètes identifiées pour regrouper les cellules discrètes identifiées.
